# EUROPEAN PATENT APPLICATION

(11) **EP 4 431 023 A1**
(43) Date of publication of application: **18.09.2024**
(21) Application number: 22891193.9
(22) Date of filing: 08.11.2022
(51) Int. Cl.: A61B 6/12, A61B 6/00

(54) **IMAGE-PROCESSING DEVICE, IMAGE-PROCESSING METHOD, PROGRAM, AND IMAGE-PROCESSING SYSTEM**

(30) Priority: 09.11.2021 JP 2021182423
(71) Applicant: Imed Technologies, Inc., Tokyo 113-0034 (JP)
(72) Inventor: KONO Kenichi, Tokyo 113-0034 (JP); FUKUDA Shogo, Tokyo 113-0034 (JP); SAWAYAMA Tomoko, Tokyo 113-0034 (JP); KAWAI Yuji, Tokyo 113-0034 (JP); MATSUMOTO Minoru, Tokyo 113-0034 (JP); JEONG Doowon, Tokyo 113-0034 (JP)
(74) Representative: Plasseraud IP
(86) International application number: PCT/JP2022/041503
(87) International publication number: WO 2023/085253

(57) **Abstract**

[Problem] To provide a feature for supporting a healthcare worker during a catheter examination or medical treatment of a blood vessel so as to facilitate concentration on work in an area of attention and provide support in making a determination regarding the area of attention. [Solution] In an image processing apparatus 1, an image acquisition unit 110 acquires an X-ray image, created on the basis of an absorption rate of X-rays, where at least a blood vessel and a device for examining or medically treating the inside of the blood vessel is included in a subject. The region of interest acquisition unit 111 acquires one or more regions including at least a portion of the device included in the X-ray image as the region of interest. The tracking unit 112 tracks each of the regions of interest R in the X-ray image. The notification unit 113 notifies a user of the image processing apparatus 1 of the fact that if at least one of the regions of interest satisfies the condition determined for each of the regions of interest. The present disclosure also includes recording and creating a summary of actions taken during surgeries.

## Description

### Technical Field

The present invention relates to an image processing apparatus, an image processing method, a program, and an image processing system, and in particular to an image processing technology for use in the examination or treatment of a blood vessel.

### Background Art

In recent years, for the examination and treatment of blood vessels in the whole body such as the brain and heart, surgery is being performed where a medical worker such as a physician passes a catheter through the blood vessel of a subject and performs various treatments while displaying the location of the catheter on an X-ray image. In this catheterization surgery, a healthcare professional generally passes a medical device such as a catheter from the inguinal region or upper arm of a subject, and advances the device to a region of interest.

For example, when examining or treating a cerebral blood vessel, a medical worker advances a catheter from the subject' s inguinal region or brachial artery through the aortic arch and carotid artery to the cerebral artery. Since a great deal of experience and training is required for healthcare professionals to perform these procedures properly, for example, Patent Document 1 discloses a simulation system for improving the efficiency of surgical procedures performed using a catheter system.

### Citation List

### Patent Documents

Patent Document 1: International Publication Pamphlet WO 2017/139894

### Summary of the Invention

### Problems to be Solved by the Invention

In the examination and treatment of a blood vessel, medical professionals pass a guiding catheter to the target site and then pass multiple devices for examination or treatment such as guide wires, stents, balloons, microcatheters, liquid embolic materials, filters, etc. in the guiding catheter, delivery wires of embolic coils for embolization of aneurysm, etc., and such. That is, in actual examinations and treatments, healthcare professionals need to pay attention to the movements and locations of multiple devices not only when the guiding catheter is passed to the target site but also during the procedure. On the other hand, in order to achieve the original purpose of the examination or treatment, the healthcare professional must concentrate on the work in the area of interest.

The present invention has been made in view of these points, and the purpose is to provide a technology for making healthcare professionals concentrate on the work in the area of interest and supporting the judgment of the attention area in the catheter examination or treatment of a blood vessel.

### Means for Solving the Problems

The first embodiment of the present invention is an image processing apparatus. This device has an image acquisition unit for acquiring an image that includes at least a device for examination or treatment in a blood vessel as a subject, a region-of-interest acquisition unit for acquiring one or more regions that include at least a portion of the device in the image as a region of interest, and a notification unit for notifying a user of the image processing apparatus when at least one of the regions of interest satisfies a condition defined for each of the regions of interest. This image processing apparatus may further include a tracking unit that tracks each of the said regions of interest in the said image.

As a variation of the first embodiment, the image processing apparatus may, instead of or in addition to a notification unit that notifies the user of the image processing apparatus when at least one of the regions of interest satisfies the conditions defined for each region of interest, be provided with an output unit that outputs the information to a device connected to the image processing apparatus when at least one of the regions of interest satisfies the conditions defined for each region of interest. The devices connected to the image processing apparatus can be connected wirelessly or via a wire. The devices may be, for example, surgical robots, VR/AR/MR devices, smart glasses, devices used as the metaverse, etc. When an output is received, for example, the surgical robot may take action such as sounding a notification, issuing a warning, automatically stopping operations, or automatically pulling the device. In the case of AR or smart glasses, one possible function would be to overlay a region of interest where a certain condition is met on the image being viewed so as to notify the viewer. This is just one example, and the operation of the device that receives the output is not limited to this.

In addition, this image processing apparatus may further be equipped with a blood vessel recognition unit that recognizes at least some of the blood vessels in the image. A notification or output may be provided if the region of interest falls within a vessel having a diameter below a threshold, or within a manually or automatically designated vessel.

When an area including the tip portion of a catheter such as a guiding catheter or the tip portion of a guide wire is set as the region of interest, the notification unit or the output unit may notify the user or output the information to a device on the condition that the region of interest disappears from the image.

When a region including the tip portion of a catheter such as a guiding catheter or the tip portion of a guide wire is set as the region of interest, the notification unit or the output unit may notify the user or output information to a device on the condition that the distance between the region of interest and the edge of the image is less than a predetermined threshold distance.

The notification unit or the output unit may notify the user or output information to a device on the condition that at least one of the moving distance, movement speed, and acceleration of the region of interest in the image exceeds the predetermined threshold value.

The notification unit may display the distance between the region of interest and the edge of the image on a display device that displays the image.

The notification unit may change the manner in which the distance is displayed on the display device according to the length of the distance between the region of interest and the edge of the image.

The notification unit or the output unit may notify the user or output information to a device on the condition that the value obtained by dividing the distance between the region of interest and the edge of the image by the movement speed of the region of interest in the image is less than a predetermined threshold value.

The image processing apparatus may be further equipped with a marker detection unit that detects a marker established on the delivery wire of the embolic coil that approaches a region of interest configured in a portion of the guiding catheter that guides the delivery wire. The tracking unit may further track the detected marker, and the notification unit or the output unit may notify the user or output information to a device when the embolic coil may be detached from the delivery wire, as triggered by the superimposition of the marker and the region of interest.

The notification unit or the output unit may notify the user or output information to a device when the marker has passed the region of interest.

The notification unit may display on the display device the distance that the marker should move before detaching the embolic coil from the delivery wire.

When a feature value indicating the shape of the device included in the region of interest satisfies a predetermined condition, the notification unit may notify the user of the image processing apparatus of this fact.

The feature value may be curvature, and the notification unit or the output unit may notify the user or output information to a device on the condition that the curvature of the device in the region of interest exceeds a predetermined threshold curvature, or that the curvature changes but the tip does not move.

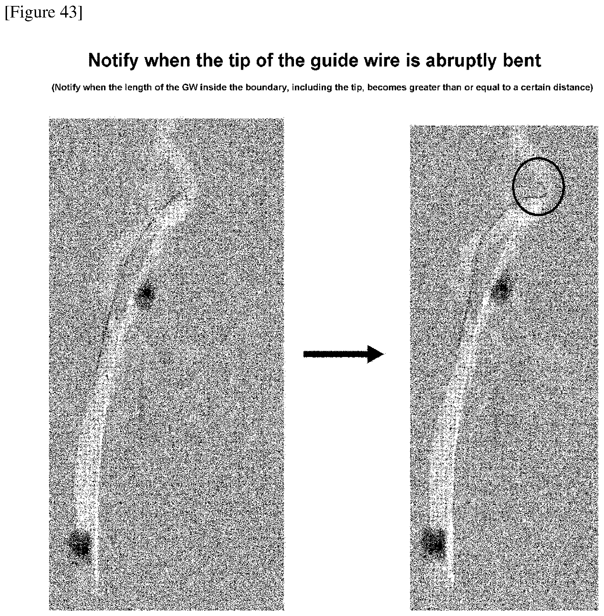

The feature value may be the distance or segmentation area of the guidewire within a region including the guidewire tip. If this exceeds a certain threshold, a notification is sent (Figure 43). A condition that the movement of the tip is within a certain range may be added to this condition. The range of the region may be changed depending on the magnification of the screen, the type of surgery, or the location of the blood vessels. The threshold may be predetermined, may be based on the surgeon's preference, or may be calculated by accumulating values over time during the surgery and notifying the surgeon, for example, when the distance exceeds twice of standard deviations. However, this is merely an example, and the method for determining the threshold is not limited to this.

The notification unit or the output unit may notify the user or output information to a device on the condition that the length of the device in the image or region of interest minus the length of the centerline of the blood vessel in the image or region of interest exceeds a predetermined threshold length.

The notification unit may be equipped with a function of notifying the user by coloring the region of interest with a different color from the image, changing the font, size, or color of the characters displayed, changing the color of the entire screen or part of the screen of the display device, displaying a graphic on the entire screen, outside the frame, or in some other location of the display device, enlarging the region of interest, or changing the color or size of a mark attached to the region of interest.

The notification unit may use sound or vibration for notification.

The second embodiment of the present invention is an image processing method. In this method, a processor of an image processing apparatus performs: a step of acquiring an image that includes at least a device for examination or treatment in a blood vessel as a subject; a step of acquiring one or more regions that include at least a portion of the device in the image as a region of interest; a step of tracking each of the regions of interest in the image; and a step of notifying a user of the image processing apparatus when at least one of the regions of interest satisfies a condition defined for each of the regions of interest. The methods of the present disclosure may be computer-implemented methods.

The third embodiment of the present invention is a program. This program realizes on a computer, a function of acquiring an image that includes at least a device for examination or treatment in a blood vessel as a subject, a function of acquiring one or more regions that include at least a portion of the device in the X-ray image as a region of interest, and a function of tracking each of the regions of interest in the X-ray image, and a function of notifying a user of the computer when at least one of the regions of interest satisfies a condition defined for each of the regions of interest. The program may be a program containing instructions that cause a computer to perform certain steps when the program is executed by the computer.

In order to provide this program or to update a part of the program, a computer-readable recording medium on which the program is recorded may be provided, or the program may be transmitted over a communication line. A third embodiment of the invention includes a computer-implemented method, which may be executed by a computer that includes a memory and processor capable of storing instructions for executing it. The instructions or program for carrying out the method on a computer may be stored on a non-transitory computer readable medium.

The fourth embodiment of the present invention is an image processing system. This system is equipped with the image processing apparatus described above and an imaging apparatus that captures an image of a person in a state in which a device for examination or treatment in a blood vessel is inserted (an image of the surgical field) and transmits the image to the image processing apparatus.

Any combination of the components disclosed in the present specification, and any conversion of the expression of the present invention between methods, devices, systems, computer programs, data configurations, recording media, or such is also valid as an embodiment of the present invention.

A further embodiment of the invention relates to an image processing apparatus including a memory or other storage device and a processor connected to the storage device, wherein the processor performs processing such as acquiring an image that includes at least a device for examination or treatment in a vessel as a subject.

### Effect of the invention

The present invention provides a technology for having medical professionals focus work in the area of interest during catheter examination or treatment of blood vessels, and supporting oversight and delayed judgment in areas other than the area of interest.

### Brief Description of the Drawings

[Figure 1] It is a figure that schematically demonstrates the appearance of an image processing system according to the embodiment.
[Figure 2] It is a figure that schematically demonstrates the functional configuration of an image processing system according to the embodiment.
[Figure 3] It is a figure for demonstrating a region of interest.
[Figure 4] It is a figure for demonstrating an example of the conditions set in a region of interest.
[Figure 5] It is a figure that shows an example of the message which the notification unit notifies.
[Figure 6] It is a figure for demonstrating another example of the conditions set in a region of interest.
[Figure 7] It is a figure for demonstrating another example of the conditions set in a region of interest.
[Figure 8] It is a figure for demonstrating the timing of detachment of an embolic coil.
[Figure 9] It is a figure for demonstrating the condition about the shape of the device set in a region of interest.
[Figure 10] It is a flowchart for demonstrating the flow of the image analysis processing executed by the image processing apparatus according to the embodiment.
[Figure 11] It is a schematic diagram of a typical neural network.
[Figure 12] It is a figure that schematically demonstrates the functional configuration of the image processing apparatus provided with the replay function according to the embodiment.
[Figure 13] It is a figure for demonstrating an example of a replay display when a notification occurs.
[Figure 14] It is a figure for demonstrating an example of displaying a replay playback window on a real-time display screen.
[Figure 15] It is a figure that schematically demonstrates the functional configuration of the image processing apparatus equipped with a status estimation unit according to the embodiment.
[Figure 16] It is a figure for demonstrating an example of the display of the estimation result of the status estimation unit according to the embodiment.
[Figure 17] It is a figure for demonstrating an example of estimation of the catheter tip position using the 2nd marker according to the embodiment.
[Figure 18] It is a figure for demonstrating an embodiment which displays the result of image analysis on two screens of different sizes.
[Figure 19] It is a figure for demonstrating an embodiment which recognizes a screen to be notified by highlighting the frame of the screen.
[Figure 20] It is a figure for demonstrating a display of the product list of the devices (for example, various types of catheters and coils) for examination or treatment in blood vessels according to the embodiment.
[Figure 21] It is a figure for demonstrating the memory processing of the position and shape of the device according to the embodiment.
[Figure 22] It is a flowchart for explaining the flow of estimation processing of the catheter tip position using the 2nd marker, which is performed by the image processing apparatus according to the embodiment.
[Figure 23] It is a flowchart for demonstrating the flow of the replay function processing executed by the image processing apparatus according to the embodiment.
[Figure 24] It is a flowchart for demonstrating the flow of the processing of estimating the position of the region of interest outside the frame executed by the image processing apparatus according to the embodiment.
[Figure 25] It is a figure for illustrating the detection of devices at the image edges.
[Figure 26] It is a flowchart for explaining the detection of devices at the image edges.
[Figure 27] It is a figure for illustrating the detection of devices that have passed the designated boundary line.
[Figure 28] It is a figure for illustrating the detection of devices that have passed the designated boundary line.
[Figure 29] It is a figure for illustrating the display of device positions on the sub-screen.
[Figure 30] It is a figure for illustrating an example of recognition of a surgical situation.
[Figure 31] It is a figure for illustrating an example of a computer configuration for the present disclosure.
[Figure 32] It is a figure showing a specific example of blood vessel extraction. The figure on the left shows the recognized result.
[Figure 33] It is a figure that schematically demonstrates the functional configuration of an image processing system according to the embodiment.
[Figure 34] It is a figure for illustrating the voice (verbal) notification according to the embodiment.
[Figure 35] It is a figure for illustrating the automatic setting of specific boundaries according to the embodiment.

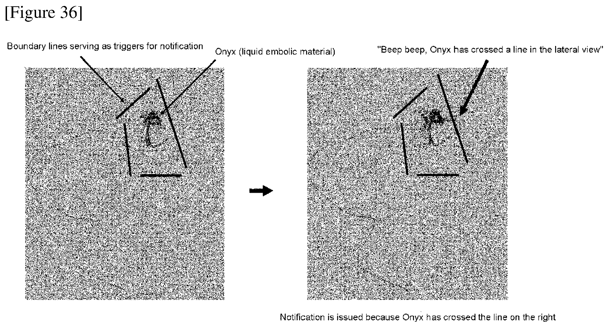

[Figure 36] It is a figure for illustrating the notification when a liquid embolic substance (such as Onyx or NBCA) crosses a specified boundary.
[Figure 37] It is a figure for illustrating variations in the method of setting specific regions according to the embodiment. There are various ways to draw specific areas (boundaries) (four examples, A to C, are shown). A: Designation by rectangle. B: Designation by ellipse. C: Designation by a horizontal straight line. D: Designation by a perpendicular straight line.

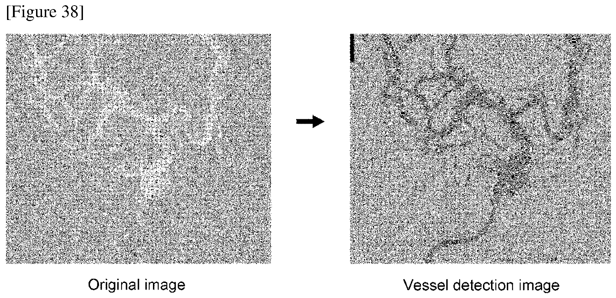

[Figure 38] It is a figure showing a specific example of blood vessel recognition. The figure on the right shows the recognized result.
[Figure 39] It is a figure for explaining the notification when the outside of a blood vessel (outside a recognized blood vessel) is specified as a specific area.
[Figure 40] It is a figure for illustrating the notification when the filter moves.
[Figure 41] It is a diagram showing how the edge of an X-ray portion is automatically detected and a boundary is set accordingly.
[Figure 42] It is a figure for explaining an embodiment in which an area moves or disappears in response to zooming in or out of the screen or a scene change. It may be set automatically or may be suggested and the user may press OK or NO accordingly.
[Figure 43] It is a figure for explaining the notification when the tip of the guidewire is bent abruptly. Specifically, for example, a notification can be made when the length of a GW within a boundary including its tip exceeds a certain distance.

### Detailed Description of the Invention

### <Mode for Carrying Out the Invention>

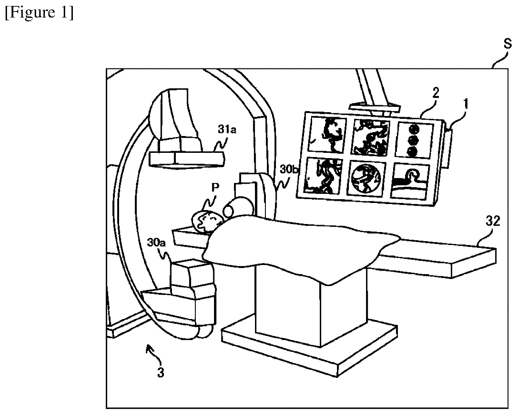

Figure 1 is a diagram that schematically shows the appearance of the image processing system S according to the embodiment. The image processing system S includes an image processing apparatus 1, a display device 2, and an X-ray imaging apparatus 3. An outline of the system will be described below with reference to Figure 1.

The X-ray imaging apparatus 3 is a device for capturing an X-ray image of a test subject P, who is a person in a state in which a device for examination or treatment of blood vessels (hereinafter simply described as "device") is inserted, and transmitting the X-ray image to the image processing apparatus 1. Therefore, the X-ray imaging apparatus 3 is equipped with an X-ray irradiator 30 (a first X-ray irradiator 30a and a second X-ray irradiator 30b) for irradiating test subject P with X-rays, and an X-ray detector 31 for detecting the X-rays emitted by the X-ray irradiator 30 and a bed 32 for supporting the test subject P.

As shown in Figure 1, the first X-ray irradiator 30a and the second X-ray irradiator 30b can irradiate the head of the test subject P with X-rays at different angles of incidence. Here, the X-rays emitted by the first X-ray irradiator 30a are detected by the first X-ray detector 31a and converted into an X-ray image based on the X-ray absorption rate. The X-rays emitted by the second X-ray irradiator 30b are detected by a second X-ray detector not shown and converted into an X-ray image. These X-ray images are displayed on the display device 2. During the medical treatment of the cerebrovascular area, the positions of the first and second X-ray irradiators are generally fixed, and the area of the images displayed on the display device 2 is fixed.

The images generated from X-rays detected by the X-ray detector 31 include the blood vessels (which can be seen by flowing a contrast medium) and tissues such as bones of the test subject P, as well as various types of devices used for blood vessel examination and treatment (for example, catheters such as guiding catheters, guide wires, embolic coils, delivery wires for transporting embolic coils to sites of interest, or such).

The image processing apparatus 1 according to the embodiment is a device for assisting a user who performs catheter examination or treatment of blood vessels (hereinafter, simply referred to as "catheterization surgery," except for the case where there is a distinction between catheter examination of blood vessels and catheter treatment of blood vessels). The image processing apparatus 1 recognizes and/or tracks one or more predetermined regions of interest in the X-ray image generated based on the X-rays detected by the X-ray imaging apparatus 3. By analyzing the X-ray images, the image processing apparatus 1 is triggered when the status of any of the regions of interest satisfies the condition defined for each region of interest, and notifies the user of the image processing apparatus 1, who is a medical professional (hereinafter simply referred to as the "user") of this fact. By setting the region of interest as an area that requires attention other than the area of interest to achieve the purpose of the examination or treatment, the user can concentrate on the work in the area of interest (for example, guiding a microcatheter into an aneurysm, placing a coil into an aneurysm, expanding a balloon, placing a stent, or such).

### <Functional configuration of the image processing apparatus 1 according to the embodiment>

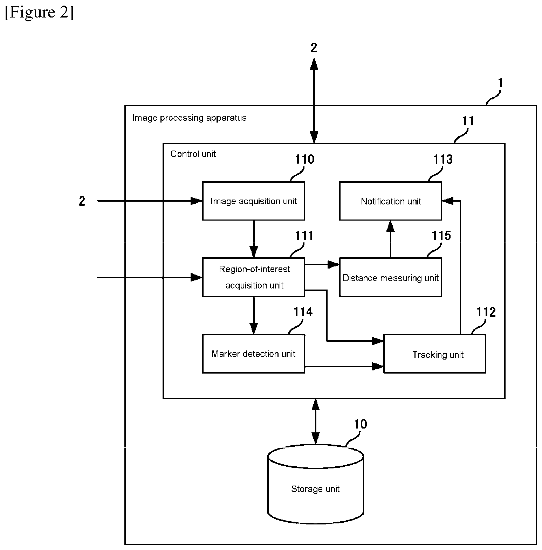

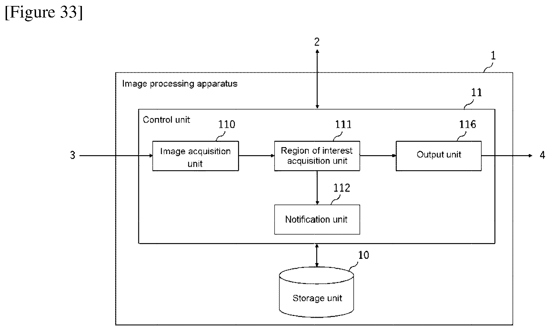

Figure 2 is a diagram that schematically shows a functional configuration of the image processing apparatus 1 according to the embodiment. The image processing apparatus 1 is equipped with a storage unit 10 and a control unit 11. In Figure 2, the arrows indicate the main data flows, and there may be data flows that are not shown in Figure 2. Further, in Figure 2, each functional block shows a configuration of the functional units, not a configuration of the hardware (device) units. Therefore, the functional blocks shown in Figure 2 may be mounted in a single device, or may be mounted separately in a plurality of devices. Data can be exchanged between functional blocks via any arbitrary means such as a data bus, a network, and a portable storage medium or such. Figure 33 is a diagram that schematically shows a functional configuration of the image processing apparatus 1 according to another embodiment. Figure 33 shows that output is performed from output unit 116 to external device 4.

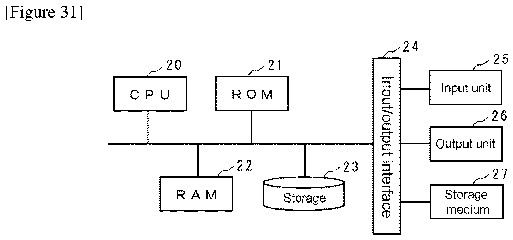

Figure 31 is a figure for illustrating an example of a computer configuration for the present disclosure. As shown in FIG. 31, the computer configuration may include CPU 20, which is a processor capable of performing arithmetic processing, etc., ROM 21, which is memory connected to the processor and capable of storing a BIOS, etc., RAM 22, which is memory that may be a working area, and storage 23, which can store programs, etc., and the computer configuration may further include input unit 25, output unit 26, and storage medium 27 via input/output interface 24. Input unit 25 may include a keyboard or other input device. Output unit 26 may include an output device such as a display. Data may be sent and received via input unit 25 and output unit 26.

In Figure 2, the storage unit 10 is a memory such as a ROM (Read Only Memory) that stores the BIOS (Basic Input Output System) of the computer that realizes the image processing apparatus 1, a RAM (Random Access Memory) which is a work area of the image processing apparatus 1, or a large-capacity storage device such as an HDD (Hard Disk Drive) or SSD (Solid State Drive) that stores an OS (Operating System), an application program, and the various information referred to when the application program is executed.

The control unit 11 is a processor or such as a CPU (Central Processing Unit) or GPU (Graphics Processing Unit) of the image processing apparatus 1, and functions as an image acquisition unit 110 by executing the program stored in a storage unit 10, a region of interest acquisition unit 111, a tracking unit 112, a notification unit 113, a marker detection unit 114, and a distance measuring unit 115.

Furthermore, Figure 2 shows an example in which the image processing apparatus 1 is composed of a single device. However, the image processing apparatus 1 may be materialized by computing resources such as a plurality of processors and memories, for example, a cloud computing system. In this case, each unit constituting the control unit 11 is materialized by executing a program by at least any of the multiple different processors.

The image acquisition unit 110 acquires an X-ray image created based on the absorption rate of X-rays, which includes at least the blood vessel of the test subject P and the device for examination or treatment in the blood vessel as a subject. For example, the X-ray image may include an aneurysm formed in a blood vessel of the test subject P, which is of interest to a healthcare professional, or a narrowed part or infarcted part of the blood vessel. The region of interest acquisition unit 111 acquires one or more regions including at least a portion of the device included in the X-ray image as the region of interest. The region of interest may be established as, for example, the tip of a guide wire (GW), the tip of a guiding catheter (GC), a catheter marker, a coil, etc., but multiple areas, for example, the tip of GC and GW, the tip of two GW and the tip of the GW and the coil may be simultaneously established as the multiple regions of interest. In some embodiments, the region of interest may include blood vessels (vascular lesion areas such as cerebral aneurysms and stenosis) and bones.

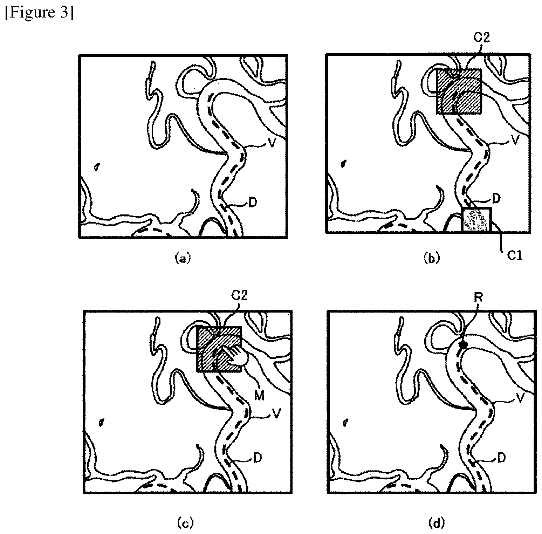

Figures 3 (a)-(d) are diagrams for explaining the region of interest. Specifically, Figure 3 (a) is a diagram that schematically demonstrates an example of an X-ray image obtained by imaging a blood vessel V. In the example shown in Figure 3 (a), a guide wire, which is one type of device D, is present in the blood vessel V.

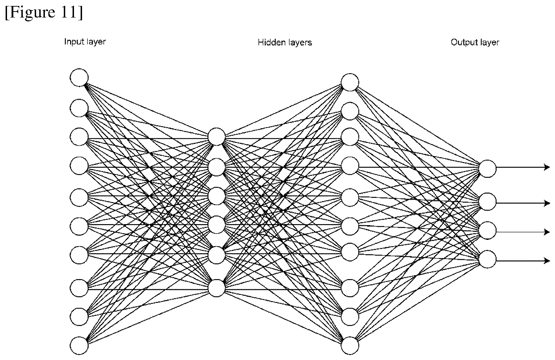

Figure 3 (b) is a diagram showing an example of a candidate region C which is a candidate region of interest. The region of interest acquisition unit 111 may include a candidate region detector generated by using a known machine learning method such as a neural network (Figure 11). In Figure 3 (b), the first candidate region C1 (tip of the guiding catheter) and the second candidate region C2 (tip of the guide wire) are shown as candidate regions of interest. This candidate region C is a result obtained by the region of interest acquisition unit 111 of inputting a frame image of an X-ray image to the candidate region detector. Without limitation, as an example, the candidate region detector is learned to detect the tip of a guide wire, the tip of a guiding catheter, a marker of a catheter, and the like.

Detection generally means identifying the position or shape of an object in a single-frame still image in a video. For example, in the detection of the tip of a guide wire, since the guide wire is thin, it may be detected as point coordinates (x, y) on the screen. The detection of the entire guide wire can be done by detecting a bent thin thread-like shape as a one-dimensional curve or as a two-dimensional segmentation.

As the object detection algorithm that can be used in the candidate region detector, for example, algorithms such as Faster R-CNN, YOLO, SSD, U-Net, and ResNet can be used, but without being limited thereto. Further, in implementing the object recognition algorithm, for example, an image processing library such as OpenCV (Open Source Computer Vision Library) may be used.

### (Inputs and outputs for learning detection)

Detection methods are not limited to machine learning, but in the case of machine learning, for example, input data for learning includes actual surgery and examination videos, videos from angiography devices using phantoms, and virtually created surgery videos. It can be a still image, not a video. Virtual surgical videos may be created manually, or surgical-like images may be generated by algorithms, such as "Generative Adversarial Networks" (GANs).

Videos of endovascular surgery and examinations of any site may be used for learning (e.g., brain, heart, peripheral limbs, abdomen, and pelvis). Any medical image such as X-ray images, CT images, MRI images, etc. may also be used.

A video may be appropriately divided into still images, and one or more still images may be used as input images for learning. The input image may be subjected to any transformation (e.g., normalization, denoising, etc.). Multiple images may be used as input as a time-series image. Frontal and lateral images may be used as input simultaneously, or mask and live images may be used. A combination of these images may be used as an input image, for example, a time series of frontal live, lateral live, frontal mask, and lateral mask images may be used. Including multiple related images is expected to improve accuracy.

Annotation may be performed based on any endovascular treatment device, blood vessel/bone/lesion site (aneurysm, stenosis, vascular malformation, etc.), vascular anatomy (internal carotid artery, Sylvian vein, etc.), magnification, location (head, chest, etc.), image quality (degree of noise), image type (masked image, live image, during contrast, during 3D imaging, etc.), surgical procedure, device used in surgery, patient information (age, gender, disease, etc.), and more. For endovascular treatment devices, blood vessels, bones, lesion sites, and vascular anatomy, annotations such as points, lines, circles, ellipses, curves, closed curves, fills (segmentation), and bounding boxes are made on the images. An ID may be assigned as an instance.

This is learned through machine learning and other means. Using the model learned here, the location and shape of the device (e.g., guidewire curve/segmentation, guidewire tip/perimeter, balloon inflation, stent section, stent shape, etc.), position, shape, and nature of the vessel (e.g., stenosis), and image quality (e.g., noisy), and the type of image (e.g., during contrasting, masked image, etc.) are inferred. In this case, the inference results of the live image may be combined with the inference results of the mask image. Alternatively, the inference results of the frontal image may be combined with the inference results of the lateral image. This is expected to improve accuracy.

This model may be used for object detection in images, as well as for inference on any data input as described above, such as scene classification for still and video images.

### (Vascular Recognition and Display)

The image processing apparatus 1 according to the embodiment can recognize not only a region of interest including a device, but also blood vessels. The method is the same as for device recognition described above. Machine learning/deep learning (AI) or rule-based methods may be used, but methods are not limited to. For example, blood vessels are annotated (e.g., filled in by segmentation) in live or masked video or still images and learned by deep learning. Alternatively, since the images are grayscale (monochrome), only white and black areas may be extracted by setting a threshold value. Blood vessels may be extracted by contour extraction. For video, an additive average may be taken. These combinations are used to extract blood vessels. Vessels may be recognized from a single image or multiple mask images over time, or from one or more images at the time of contrast. Furthermore, the frontal and lateral images may be recognized together. Recognition accuracy is expected to be improved by using images from two directions.

The recognition may be performed in real time, but is not limited to real time. The images to be used may include intraoperative DSA images, mask images, and 3D images (3DRA), preoperative CTA, MRA, and 3DRA, and other images. The blood vessels are recognized by, but not limited to, filling (segmentation), segmentation boundaries, a set of curves, a tree structure of curves, and the like.

### (Vascular Anatomy Recognition and Display)

When recognizing blood vessels, information on anatomy may also be inferred at the same time. For example, recognition may be performed based on anatomical classification such as discrimination between left and right, internal carotid artery, external carotid artery, middle cerebral artery, posterior cerebral artery, posterior communicating artery, and anterior communicating artery. The internal carotid arteries may be divided into C1 to C5, and the middle cerebral arteries may be divided into M1, M2 (superior trunk, inferior trunk, anterior temporal artery, etc.), and M3, etc. when recognizing. Furthermore, blood vessels may not only be segmented (filled in, 2D), but may also be converted into a tree-like structure (linear, 1D) by drawing a center line from the blood vessels. The methods of recognition and expression are the same as those described above.

### (Recognition and Display of Vascular Lesions)

Lesions may be recognized when recognizing blood vessels. For example, cerebral aneurysms, stenosis, cerebral arteriovenous malformations, occlusions, etc. may be recognized. The methods of recognition and expression are the same as those described above.

### (Recognition and Display of Important Blood Vessels in Surgery)

Blood vessels important in surgery may be extracted manually or automatically and highlighted, or only these blood vessels may be displayed. Examples of automatic extraction include, but are not limited to, selecting blood vessels whose diameter is greater than a threshold value, extracting main blood vessels such as the internal carotid artery from the above-mentioned recognition of vascular anatomy, and identifying aneurysms and stenosis from the above-mentioned recognition of lesions and extracting only the blood vessels along them.

### (Specific examples of blood vessel extraction)

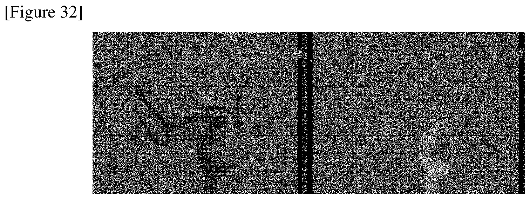

A specific example of vascular extraction is shown in Figure 32. Such vessel extraction may be performed automatically.

### (Learning specific to (cerebral) endovascular treatment)

A distinctive feature of endovascular treatment is that it is performed by viewing up to four X-ray images (mask and live images from two directions including frontal and lateral views). Therefore, instead of one of the four screens as an input image, two to four screens may be used as input for learning. Alternatively, an image generated by combining the four screens as appropriate (e.g., by taking differences) can be used as the input image. Alternatively, since the source of the mask image is the difference between the previously taken DSA image (digital subtraction angiography, i.e., the difference between the live images before and after the contrast agent is added) and the real-time DA image (digital angiography), these original source images may be used as input images.

It is also possible to use the model to perform inference on one to four screens, and then combine the inference results to improve accuracy. For example, if the catheter marker is in the same place on both screens of the live image and the masked image, it is more likely to be the correct recognition, but if only one of them is involved, it is less likely to be correct (lower confidence level). This may be used to increase recognition accuracy.

In some embodiments, the present disclosure relates to a method of producing a machine learning model, characterized in that the training is performed using cerebrovascular images acquired from multiple directions as input.

### (Exemplary detection algorithms)

Faster R-CNN is a CNN (Convolutional Neural Network) that simultaneously cuts out and recognizes regions. A convolutional neural network (CNN) is a neural network with many deep layers composed by piling up layers with some characteristic functions such as "convolutional layers" and "pooling layers," and it demonstrates excellent performance specifically in the field of image recognition. Faster R-CNN can cut out and recognize a region of interest from the input image in almost real time (about 10 to 20 frames per second). Faster R-CNN enables end-to-end learning from image input to object detection.

YOLO (You Only Look Once) is also a CNN that simultaneously cuts out and recognizes regions. In YOLO, the entire image is divided into grids, and a Bounding Box is sought for each region. YOLO's CNN architecture enables detection of highspeed objects.

SSD (Single Shot MultiBox Detector) is also a CNN that cuts out and recognizes regions at the same time. SSD can output multi-scale detection frames from the output layers of various levels. SSD puts about 9000 rectangular frames called default boxes with different sizes and shapes on the image, and calculates the predicted value for each of the frames. SSD offers higher speeds by reducing the filter size.

U-Net is a CNN that recognizes an object called segmentation pixel by pixel. It is composed of convolutional layers and has an almost symmetrical Encoder-Decoder configuration. The feature map downsampled through the pooling of the Encoder is upsampled by the Decoder.

Figure 3 (c) is a diagram showing an example of a method for setting the region of interest R. In the example shown in Figure 3 (c), the user selects a second candidate region C2 as the region of interest R. Specifically, the user selects the region of interest R by moving the mouse cursor M to the second candidate region C2 using a pointing device not shown, such as a mouse. The presentation of the candidate region C by the region of interest acquisition unit 111 is arbitrary, and the user may set the region of interest R directly in the X-ray image in a state where the candidate region C is not presented. For example, the region of interest R can be set by drawing a rectangle in the image and designating the region using a pointing device such as a mouse. Alternatively, the region of interest acquisition unit 111 may acquire the output of the candidate region detector generated by using a known machine learning method or the like as the region of interest R.

Figure 3(d) is a diagram showing a region of interest R set by the user. In the example shown in Figure 3 (d), the region of interest R is shown as a black pentagon and is a region that includes the tip portion of the device D. In Figure 3(d), only one region of interest R is set, but the user can set two or more regions of interest R.

The description in Figure 2 is returned to. The tracking unit 112 tracks each one or more regions of interest R in the X-ray image. The tracking unit 112 can materialize tracking of the region of interest R by using a known image tracking technique. Known image tracking techniques that are available include, but are not limited to, algorithms such as Boosting, MIL, TLD, MedianFlow, KCF, GOTURN, MOSSE, CSRT and the like. The tracking algorithm may be used in combination with the object detection algorithm described above. In implementing the algorithm, for example, a library such as OpenCV (Open Source Computer Vision Library) may be used. Further, it should be noted that, in the context of the present invention, tracking the region of interest R also includes intermittently detecting the region of interest R and identifying its state. Therefore, in some embodiments of the present invention, it is not required that the region of interest R be recognized in all frames of the X-ray image acquired over time. In some embodiments of the invention, tracking of the region of interest R may be performed using a tracking algorithm or an object detection algorithm, or a combination thereof.

The BOOSTING tracker is a tracker based on the online version of AdaBoost (an algorithm used internally by HAAR cascade-based face detector). This classifier is trained at run time using correct and incorrect examples of objects. The first bounding box identified by the user (or another object detection algorithm) is treated as a correct example of the object, and the image outside the bounding box is treated as the background. When a new frame is given, the classifier is applied to all pixels near the previous position and the score is recorded. The new position of the object is the position where the score is maximized. In this way, another correct example of the classifier is obtained. More frames are entered and the classifier is updated by this additional data.

The MIL tracker is based on the same concept as the BOOSTING tracker above. The big difference is that the current position of the object is not just considered as a correct example, but at a small neighborhood around the current position is investigated to generate some potential correct examples. In MIL, instead of specifying correct examples and incorrect examples, a "bag" of correct and incorrect answers is specified. The group of images in the correct bag is not all correct examples. Only one image of the correct bag needs to be an example of the correct answer. The correct bag contains an image centered on the current position of the object and an image of a small neighborhood around it. Even if the current position of the tracked object is not accurate, if a sample near the current position is in the correct bag, there is a high possibility that this bag will contain at least one image of the object properly positioned in the center. The MIL tracker has high performance, does not drift as much as the BOOSTING tracker, and provides reasonable performance even in the event of partial occlusion (shielding).

KFC stands for kernelized correlation filter. This tracker is based on the concepts advocated by the above two trackers. This tracker takes advantage of the fact that a plurality of correct samples used in the MIL tracker has a large overlap area. Such duplicated data provides some excellent mathematical properties, while at the same time making tracking faster and more accurate. It is superior to MIL in both accuracy and speed, and is also excellent in reporting tracking failures.

TLD means tracking/learning/detection. As the name implies, this tracker breaks down the long-term tracking task into three components: (short-term) tracking, learning, and detection. This tracker tracks objects frame by frame. The detector identifies all previously observed appearances and calibrates the tracker as necessary. The detector error is estimated by learning and updated to avoid occurrence of future errors. The output of this tracker tends to be somewhat unstable; for example, if there are other pedestrians in the scene when tracking a pedestrian, this tracker may temporarily track another walker different from the pedestrian to be tracked. The advantage is that it works best even under occlusion (shielding) across multiple frames. The disadvantage is that there are many false positives.

The MEDIANFLOW tracker tracks an object both in the forward direction and backward direction in time and measures the discrepancy between these two trajectories. By minimizing this Forward/Backward error, it is possible to detect tracking failures with certainty and select a reliable trajectory in the video. This tracker performs best when the movement is predictable and small, and when there is no obstruction. Unlike other trackers that continue even when the tracking clearly fails, this tracker can recognize that the tracking has failed.

The GOTURN tracker is an algorithm based on a convolutional neural network (CNN). This tracker is robust to changes of viewpoint, changes of lighting, and deformations, but may not handle shielding (occlusion) well.

The MOSSE (Minimum Output Sum of Squared Error) tracker uses adaptive correlation for object tracking. The MOSSE tracker is robust to lighting, scale, pose changes, and non-rigid deformation. The tracker can also detect occlusions based on the peak-to-sidelobe ratio and resume tracking from where it left off when the object reappears. The MOSSE tracker also works at high frame rates (450 fps or above). The advantage is that it is very easy to implement, as accurately as other complex trackers, and much faster.

The CSRT tracker uses spatial reliability maps for tracking. The CSRT tracker operates at a relatively low frame rate (25 fps), but provides higher accuracy for object tracking.

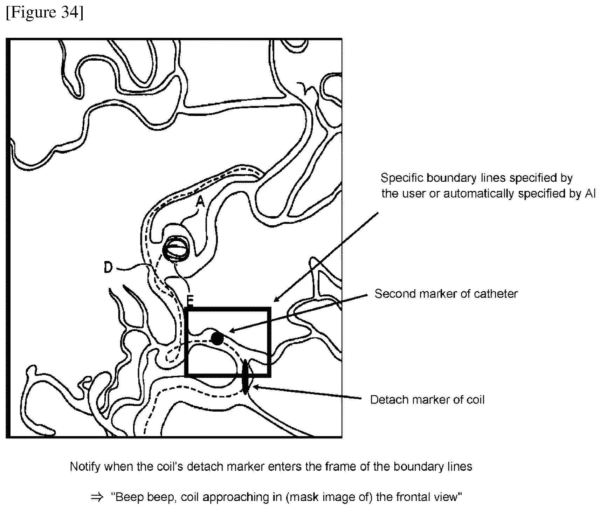

The notification unit 113 notifies the user of the fact that if at least one of the regions of interest R satisfies the condition determined for each of the regions of interest. Specifically, the notification unit 113 notifies the user, for example, when at least one of the regions of interest R satisfies the condition defined for each of the regions of interest, by displaying a message indicating that fact on the display device 2 or by sounding a notification tone on the speaker of the image processing apparatus 1 not shown. Alternatively, when the user wears a device equipped with a vibrating component such as a smartphone, the user may be notified by vibrating the vibration component. The type, amplitude, and frequency of the notification sound may be changed according to the conditions. In addition, the notification sound may be combined with voice (Figure 34). One issue is that surgeons and assistants may be concentrating on the surgery and not be able to see the notification screen, or they may view it late. This problem can be solved if the notified status can be determined using audio alone. For example, audio notifications such as "Beep,beep, on the frontal mask image, the coil is approaching" (Figure 34), "Beep, on the lateral live image, the guide wire has gone off the screen," "Beep, on the frontal live image, the guiding catheter has fallen off the screen," "Ring, on the lateral (on plane B), a balloon has appeared on the screen," "Ring, ring, on the front (on plane A), the filter has moved," etc. allow the surgeon and assistants to understand what is happening without taking their eyes off the main surgical screen. It is sufficient to communicate by audio what happened on which screen, on which device, and in what manner, and the manner in which the audio is communicated is not limited to the above example. The vibration may change the period and amplitude of the vibration according to the conditions.

In this way, by setting the region of interest R in the X-ray image in advance, the user of the image processing apparatus 1 can have the image processing apparatus 1 track the behavior of the set region of interest R, allowing the user to concentrate on working in the area of interest. Further, when displaying the region of interest, the notification unit 113 may color the region of interest with a color different from the color of the acquired image (X-ray image) and display the region of interest. In general, angiographic images are monochrome, and therefore it is very difficult to recognize the device contained in the image. However, by coloring the region of interest, the device can be easily recognized and it will be possible to provide the user with more accurate information. Further, when a plurality of regions of interest is established, the regions of interest may be colored using different colors.

### [Specific example of the conditions set in the region of interest R]

Next, a specific example of the conditions set in the region of interest R will be described.

In blood vessel catheterization surgery, the user first passes a guiding catheter into the test subject P's blood vessel to the vicinity of the area of interest. Subsequently, the user performs observation (diagnosis) and treatment of the site of interest, for example, coil embolization of a cerebral aneurysm, through another device such as a guide wire, a delivery wire, or a balloon catheter in the guiding catheter.

A catheter is a device that is an elongated tube with a lumen. This catheter is passed through a blood vessel and guided into an aneurysm, for example, a coil is inserted through the lumen of the catheter, and the coil is inserted into the aneurysm to prevent rupture.

There are several types of catheters, but first, the guiding catheter is slightly thick with a diameter of about 2-4 mm, and plays a role of connecting from the puncture site to the front of the target site. A smaller device is inserted into this guiding catheter and guided to the target site. The advantage of guiding catheters is that various devices can be carried via a guiding catheter from the puncture site of a blood vessel in the groin or arm to the vicinity of the target site, and thereby eliminates the need to check the passage one by one. As a result, for example, the treatment can be efficiently performed without moving the fixed screen.

Some guiding catheters have a balloon at the tip (a guiding catheter attached with a balloon), which can stop the flow in the blood vessel and stabilize the guiding catheter.

An intermediate catheter is placed in a guiding catheter and positioned at an area proximally to the target site where the blood vessel is thin, in order to deliver the thinner device to the target site. A plurality of intermediate catheters may be used (gradually become thinner).

A microcatheter is the finest catheter, which is soft and can be inserted into thin blood vessels. A coil or stent is placed in this microcatheter and carried to the target site. An embolic material may also be flushed from the microcatheter.

A balloon catheter has a balloon attached near the tip of a microcatheter, and the balloon is inflated near the target site. As a result, for example, the coil is prevented from coming out of the aneurysm, the stenotic site is dilated, and the blood flow is stopped in the event of vascular perforation to stop bleeding. The balloon usually refers only to the balloon portion of the balloon catheter.

Further, when guiding these catheters, a guide wire of an appropriate size is often used inside. In rare cases, they may be guided by placing them in the flow of blood.

A guide wire is an elongated wire. Typically, guide wires are used to guide a soft catheter to a site of interest, by selecting a vascular bifurcation. In addition, there are delivery wires to carry stents and devices that have a balloon attached to the guide wire to stop blood flow. In the present specification, the term guide wire is defined in a broad sense to include them. The reason for this is that the tips of those guide wires can perforate blood vessels and cause serious complications. Since one of the purposes of the present invention is to support the prevention of such perforation of blood vessels by the tip of such a guide wire, any wire including a thin wire that has a possibility to cause vascular perforation at the tip is referred to as a guide wire.

During the procedure, the blood vessel of the test subject P is present in the body of the test subject P, so that the user cannot directly observe the various devices inserted in the blood vessel. Therefore, as described above, the user can manipulate the device D while viewing the blood vessel V and device D (e.g., guiding catheter, guide wire, coil, catheter, balloon, stent, etc.) that are imaged in the X-ray image generated from X-rays transmitted through the test subject P's body. As shown in Figure 1, the X-ray imaging apparatus 3 used in catheterization is a device for generating X-ray images of a specific region including the area of interest (e.g., aneurysm, stenosis site, or infarction site in need of treatment) inside the test subject P's body. During the procedure, in many cases, especially in the cerebrovascular region, the area to be acquired as an X-ray image is fixed. Therefore, the entirety of device D is not always captured in the X-ray image that can be observed by the user.

Therefore, when the user is concentrating on a task in the area of interest, such as inserting a coil into an aneurysm or placing a stent, for example, the tip of a guide wire or the tip of a guiding catheter may disappear from the X-ray image because it is out of the angle of view of the X-ray image, and the user may be unable to observe it. In such cases, a vascular perforation at the tip of the device may go unnoticed. Vascular perforation is a serious life-threatening complication. Vascular perforation is most likely to occur at the tip of a device, and it is most likely to occur especially at the tip of a guide wire or catheter. For this reason, the present invention focuses specifically on the tip. There may be multiple tips within a single screen, or the tip of the device may cause vascular perforation at a location other than the operator's area of interest. In addition, in cerebral endovascular treatment, it is necessary to treat while viewing a maximum of four multiple screens at the same time, and it is impossible for the operator to always pay attention to multiple tips in the multiple screens. This support system compensates for that issue with AI and other technologies. Therefore, an example of the conditions established for the region of interest R used in the present invention is a condition related to the distance between the tip of the guiding catheter or the tip of the guide wire and the edge of the X-ray image. Alternatively, the condition may be set to be that the region of interest R exceeds a specific range specified on the X-ray image (for example, a boundary specified by the user with a pointing device such as a mouse) or simply that it moves. That is, in some embodiments of the present invention, not only the edge portion of the image but also the range of any arbitrary region in the image may be treated the same as the edge portion. Further, the boundary line may be a straight line, a curve, a circle, a rectangle, or any other polygon. The boundary line may be able to be moved, deformed, enlarged, or reduced by user manipulation. In some embodiments of the present invention, the distance between the region of interest and the edge of a particular range may be displayed. The manner in which the distance is displayed may vary depending on the length of the distance between the region of interest and the edge of the specific range. In some embodiments of the present invention, the notification unit may notify the user on the condition that the value obtained by dividing the distance between the region of interest and the edge of the specific range by the movement speed of the region of interest in the image is less than a predetermined threshold value. Some embodiments of the present invention include a function to make it easier for the operator to make a decision by making a specific range easier to see by superimposed display or other means. The method of displaying the range may use arrows or such as well as superimposed display, but it is not limited thereto. Further, in some embodiments of the present invention, the distance may be determined by either a straight line distance or a distance along a blood vessel. In some embodiments of the present invention, instead of or in addition to the notification unit, there may be an output unit that outputs information to a device connected to the image processing apparatus.

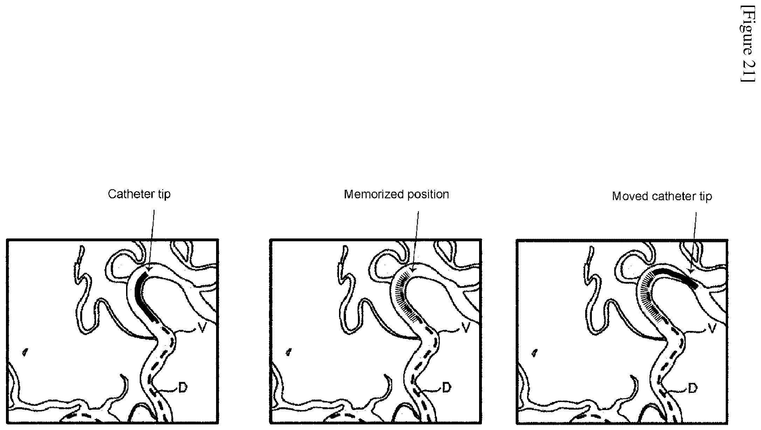

Furthermore, by having the location of a device for examination or treatment (also called device of interest) in the vessel, rather than the region of interest, specified automatically or by the user (surgeon/assistant) at a certain point in time, all or part of the device detected at that point in time may be superimposed on the subsequent real-time image. This allows the user (surgeon/assistant) to recognize the movement (deviation) of the device from a specific point in time by superimposing the real-time image and the superimposed display (Figure 21). The shape of the device may be detected automatically by image analysis or by the surgeon marking it with a mouse or a touch panel. The devices to be superimposed and displayed include, but are not limited to, guide wire tips, markers, stents, guiding catheters, and the like.

For example, suppose that the position and shape of the guide wire tip (black portion) at the time of the left figure in Figure 21 are memorized. In this case, for example, when the user specifies the guide wire tip with a pointing device such as a touch panel or a mouse, the guide wire tip is recognized and the stored guide wire tip is superimposed on the subsequent X-ray image obtained in real time, as shown in the central figure in Figure 21. As shown in the right figure in Figure 21, this makes it easy to visualize and grasp how far the guide wire tip has moved since then.

Thus, in some embodiments of the present invention, the image processing apparatus includes a storage unit that acquires and stores the position and/or shape of a device for examination or treatment in a blood vessel at any point in time, and has the function of superimposing the stored device position and/or shape on images since the acquisition.

For example, in carotid artery stenting, it is important that filters or balloons placed in the distal region of the internal carotid artery remain stable and not move too much to prevent distal embolization (stroke). A notification can be issued if the tip of a guide wire attached to them or their tip moves out of a certain range specified by the surgeon or automatically. In cerebral aneurysm embolization, if the tip of the guide wire in the balloon catheter moves out of the specified range, there is a risk of the balloon slipping, not being returned and perforating the blood vessel at the distal region, and therefore a notification can be issued. Likewise, when inserting a coil, if it moves out of a certain area, a notification can be sent out because it may occlude an important blood vessel. If the coil deviates from the mask image (region) of the aneurysm, a notification can also be issued because of the possibility that the aneurysm may be perforated by the coil, guide wire, or catheter. While it is important that the guiding catheter remains stable for all endovascular procedures, there are times when it is desirable to correct the situation before it disappears outside of the frame, and a notification can be issued if it appears to be moving out of a certain area. In tumor embolization, embolization of cerebral arteriovenous malformation, dural arteriovenous fistula embolization or such, embolization is performed using an embolization material such as liquid or particles, and if the embolization material exceeds the designated area, there is a possibility that it may cause a stroke in an important area, and a notification can be issued. Thus, in some embodiments of the present invention, the device may also include liquid embolic materials, particle embolic materials and such. In addition to the above examples, care is taken in endovascular surgery to keep any arbitrary device, embolic material and such within a designated area and this can be supported.

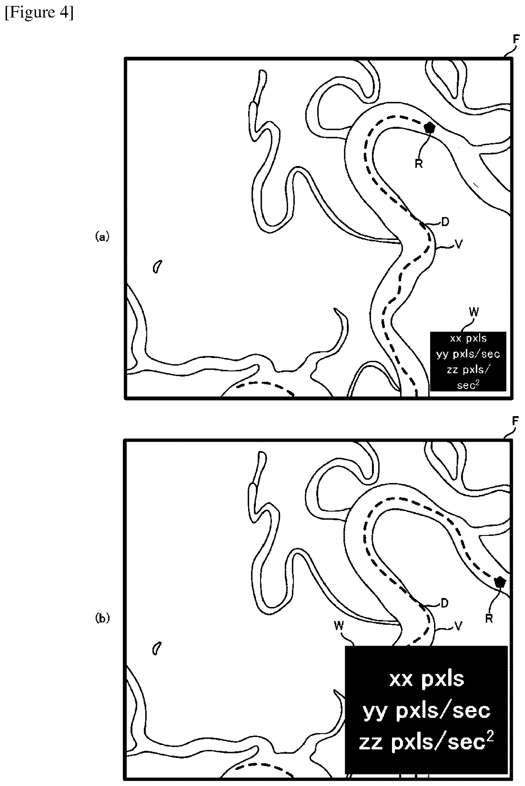

Figures 4(a)-(b) illustrate an example of the conditions set for the region of interest R. In the example shown in Figure 4(a), the region of interest R is set at the tip portion of the device D. In the example shown in Figure 4(a), information W indicating the speed and acceleration of the region of interest R and the distance (in pixels) between the region of interest R and the edge area F of the X-ray image is superimposed on the X-ray image. Further, Figure 4(a) shows an example when the device D is a guide wire. In some embodiments of the present invention, a specific region specified in the X-ray image may be considered the same as the edge area F of the X-ray image. In the example shown in Figure 4 (a), a guide wire, which is one type of device D, is present in the blood vessel V.

The notification unit 113 notifies the user on the condition that the region of interest R disappears from the X-ray image, when a region including the tip portion of the guide wire is set as the region of interest R. In some embodiments, the X-ray image is an image showing a fixed area that includes the region of interest. Usually, during the procedure, the area displayed as the X-ray image is fixed. Further, when a region including the tip portion of the guide wire is set as the region of interest R, the notification unit 113 notifies the user on the condition that the distance between the region of interest R and the edge area F of the X-ray image is less than a predetermined threshold distance.

The "predetermined threshold distance" is a "reference distance for judgment of ejection" established for the notification unit 113 to judge whether or not there is a high probability that the tip of the device will be outside the angle of view of the X-ray image. The specific value of the predetermined threshold distance may be determined by experimentation, taking into consideration the frequency of notification by the notification unit 113 and usability, or such. For example, either the number of pixels in the vertical direction or the number of pixels in the horizontal direction of the X-ray image is 5% of the number of pixels in the other. This allows the user to be notified when the region of interest R, which is an area including the tip portion of a guiding catheter or the tip portion of a guide wire, approaches the edge area F of the X-ray image, before and after the region of interest R is moved out of the edge area F.

The size of movement of the device D in the blood vessel V can be a useful indicator for the user to predict the time it will take for the tip portion of the device D to reach the edge area F of the X-ray image. Specifically, the greater the speed or acceleration of the tip portion of D, the shorter the time until the tip portion of D reaches the edge area F of the X-ray image. Therefore, when a region including the tip portion of a guiding catheter or the tip portion of a guide wire is set as the region of interest R, the notification unit 113 may notify the user on the condition that at least either of the movement speed and the acceleration of the region of interest R in the X-ray image exceeds a predetermined threshold value. As a result, when the movement speed or acceleration of the region of interest R is greater than the threshold value, the user's attention can be drawn before the region of interest R approaches the edge area F of the X-ray image.

Furthermore, the distance between the tip portion of the device D and the edge area F of the X-ray image in the blood vessel V is a useful indicator for the user to determine the probability of the tip portion of device D reaching the edge area F of the X-ray image. Therefore, as shown in the information W in Figure 4, the notification unit 113 may display the distance between the region of interest R and the edge area F of the X-ray image on the display device 2 which displays the X-ray image.

Here, the "distance between the region of interest R and the edge area F of the X-ray image" may be the distance when the region of interest R moves to the edge area F of the X-ray image along the blood vessel V inserted with the device D in which the region of interest R is set. This can be achieved by the distance measuring unit 115, which extracts the blood vessel V using a blood vessel recognition engine generated in advance using a known machine learning method or the like, and measures the distance from the region of interest R to the edge area F along the blood vessel V. Alternatively, the distance measuring unit 115 may measure the distance described above based on the trajectory of the device D as it travels through the blood vessel V. Specifically, when the user advances a guiding catheter in the blood vessel V, the tracking unit 112 tracks the tip portion of the guiding catheter and stores the trajectory in the storage unit 10. The distance measuring unit 115 may set the length of the trajectory which is included in the region of interest R among the trajectories stored in the storage unit 10 as the length described above.

By having the notification unit 113 display the distance between the tip portion of the device D and the edge area F of the X-ray image on the display device 2, the user can objectively grasp at a glance how far the device D must move to reach the edge area F of the X-ray image. Furthermore, the notification unit 113 may change the display mode of the distance according to the length of the distance between the region of interest R and the edge area F. For example, the font size may be increased as the distance becomes shorter, the color may be changed according to the distance (from blue to yellow to red), the region of interest may be enlarged according to the length of the distance, and the color or size of the mark attached to the region of interest may be changed according to the length of the distance. By working on the above-mentioned mode of display, it can be made easier for the user to notice the change in distance.

In the example shown in Figure 4(b), the region of interest R set at the tip portion of the device D is closer to the edge area F than in the example shown in Figure 4(a). Therefore, the font of the information W, which indicates the speed and acceleration of the region of interest R and the distance (in pixels) between the region of interest R and the edge area F of the X-ray image, is greater than in the example shown in Figure 4(a).

The "distance between the region of interest R and the edge area F of the X-ray image" may be the shortest distance between the region of interest R and the edge area F of the X-ray image, or it may be the length measured along the direction of movement of the region of interest R to the edge area F of the X-ray image. In this case, the blood vessel extraction processing by the distance measuring unit 115 can be omitted, which is advantageous in that it can speed up the processing.

The notification unit 113 may notify the user on the condition that the value obtained by dividing the distance between the region of interest R and the edge area F of the X-ray image by the movement speed of the region of interest R in the X-ray image is less than a predetermined threshold value. The value obtained by dividing the distance between the region of interest R and the edge area F of the X-ray image by the movement speed of the region of interest R in the X-ray image is, so to speak, the expected time until the region of interest R reaches the edge area F of the X-ray image. Therefore, the value of the "predetermined threshold value" is the "grace period for judgment of ejection" established for the notification unit 113 to judge whether or not there is a high probability that the region of interest R will be outside the angle of view of the X-ray image. The specific value of the grace period may be determined by experimentation, taking into consideration the frequency of notification by the notification unit 113 and usability or such, and it is, for example, 3 seconds.

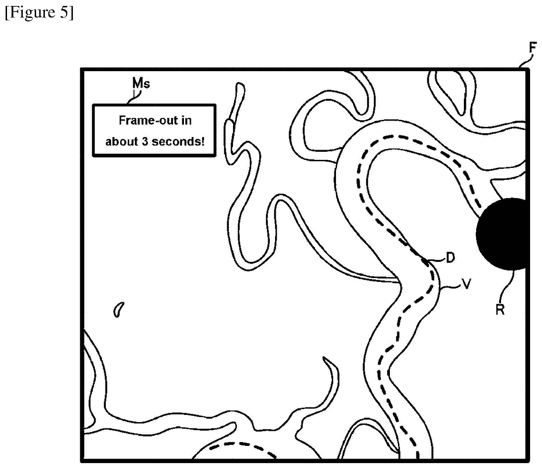

Figure 5 shows an example of a message Ms notified by the notification unit 113. In the example shown in Figure 5, the notification unit 113 displays a message superimposed on the X-ray image indicating that the region of interest R will disappear out of the X-ray image in 3 seconds (so-called frame out). Furthermore, as in the example shown in Figure 5, the notification unit 113 may change the shape of the region of interest R (changed to a circle in Figure 5), increase the size of the region of interest R, or change the color of the region of interest R when the region of interest R approaches the edge area F of the X-ray image. This allows the notification unit 113 to make it easier for the surgeon to recognize that the region of interest R is about to frame out.

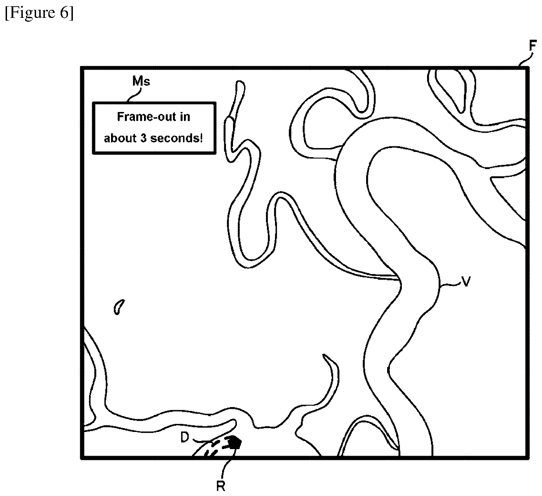

Figure 6 is a figure that illustrates another example of the conditions set for the region of interest R. Specifically, Figure 6 shows an example where the device D is a guiding catheter. Unlike the case of the guide wire shown in Figures 4(a)-(b), the guiding catheter differs in that not only its tip portion but also the entire device D can disappear from the X-ray image. However, whether the device D is a guiding catheter or a guide wire, it is the same in that the tip portion disappears from the X-ray image. Therefore, in the case of a guiding catheter, the region of interest R is set at the tip portion of the catheter as in the case of a guide wire.

Further, in the above example, the threshold is determined based on the distance between the region of interest R and the edge area F of the X-ray image as the "predetermined threshold distance" mainly from the viewpoint of preventing frame-out of the surgical instrument. However, attention is paid to the distance moved in the region of interest R itself, and the threshold value can also be configured to issue a notification to the user when the distance moved in the region of interest R becomes greater than a predetermined threshold. For example, in the state at which the tip of a catheter is inside an aneurysm, when a stent is about to be deployed, it is necessary to take care so that the tip of the catheter does not come out of the aneurysm, but at this time, the surgeon is looking at the stent and cannot follow the movement of the catheter tip with his eyes. In such a case, by configuring issuance of a notification to the user when the movement distance of the region of interest set at the tip of the catheter exceeds a predetermined movement distance, the user can immediately notice the abnormality when the tip of the catheter is about to come out of the aneurysm. The threshold for the movement distance may be set after the region of interest (for example, the tip of the catheter) reaches a predetermined position (for example, within the aneurysm), based on the position reached.

### (Guiding of the embolic coil)

Next, as another example of the conditions set for the region of interest R, the conditions related to the guiding catheter that guides the delivery wire for the embolic coil are described.

As a catheter treatment for a cerebral aneurysm, embolization in which an embolic coil is filled into the cerebral aneurysm is known. This treatment involves passing a guiding catheter to the vicinity of the cerebral aneurysm, which is the site of interest, passing the delivery wire of the embolic coil through the guiding catheter, and detaching and filling the embolic coil into the cerebral aneurysm. It is an operation aimed to block the inflow of blood into a cerebral aneurysm.

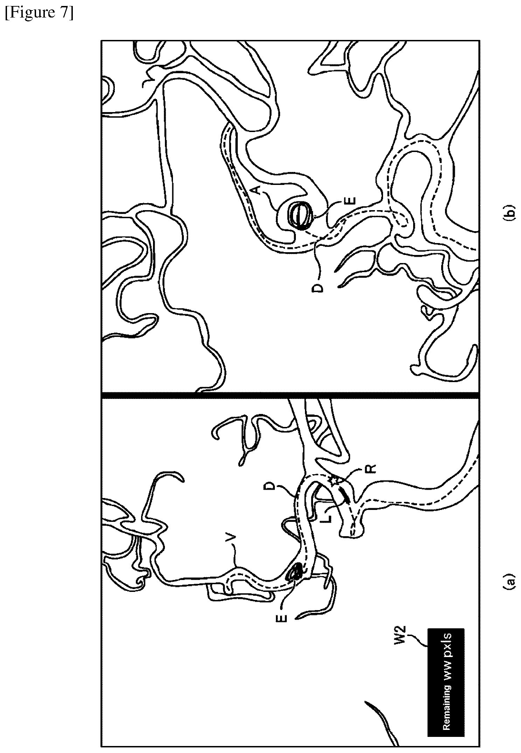

Figures 7(a)-(b) schematically show an example of an X-ray image in which a guiding catheter that guides the delivery wire of the coil for embolization is imaged, and is a figure for illustrating another example of the conditions set for the region of interest R.

As described above, the X-ray imaging apparatus 3 can irradiate the head of the test subject P with X-rays at different angles of incidence. Figures 7(a) and 7(b) illustrate examples of X-ray images taken by irradiating the head of test subject P with X-rays at different angles of incidence from each other. The user performs the embolization procedure while viewing the two images shown in Figures 7(a) and 7(b).).

In Figure 7 (b), the aneurysm is indicated by the symbol A. The user embolizes the aneurysm A by placing a plurality of embolic coils E inside the aneurysm A. Here, since the aneurysm A can be observed in the X-ray image shown in Figure 7(b), the user focuses on one X-ray image among the two X-ray images.

The delivery wire used to transport the embolic coil E to the aneurysm A is coupled to the embolic coil E at its tip, and the user performs a task of detaching the embolic coil E from the delivery wire after transporting the embolic coil E to the aneurysm A. As shown in Figures 7(a)-(b), when the embolic coil E reaches the aneurysm A, the position of the embolic coil E being transported becomes unclear in the X-ray image due to the other embolic coils E that are already in place in the aneurysm A. For this reason, the delivery wire of the embolic coil E is pre-equipped with a marker L to determine the timing for detaching the embolic coil E. The user uses the marker L on the delivery wire, rather than the embolic coil E itself in the X-ray image, as a landmark to determine the timing for detaching the embolic coil E.

However, as shown in Figures 7 (a)-(b), it is possible that the X-ray image in which the aneurysm A can be observed and the X-ray image in which the marker L can be observed are different. Further, even within the same screen, the positions of the aneurysm A and the marker L are separated, and it may be difficult to observe two points at the same time. Therefore, the user of the image processing apparatus 1 first sets the region of interest R in a portion of the guiding catheter that guides the delivery wire of the embolic coil E. Specifically, the user sets the region of interest R at a position where the marker L and the region of interest R overlap at the timing of detaching the embolic coil E from the delivery wire.

The region of interest acquisition unit 111 receives the region of interest R set in a part of the guiding catheter that guides the delivery wire. The region of interest R can be designated using, for example, a pointing device such as a mouse, a touch panel, or the like. In Figure 7 (a), the region of interest R is indicated by a white star. The marker detection unit 114 detects the marker L that approaches the region of interest R among the markers L provided on the delivery wire of the embolic coil E for embolizing the aneurysm. Further, the tracking unit 112 tracks the marker L detected by the marker detection unit 114.

The notification unit 113 notifies the user of the timing for cutting and detaching the embolic coil E from the delivery wire based on the positional relationship between the marker L and the region of interest R, triggered by the superimposition of the marker L and the region of interest R.

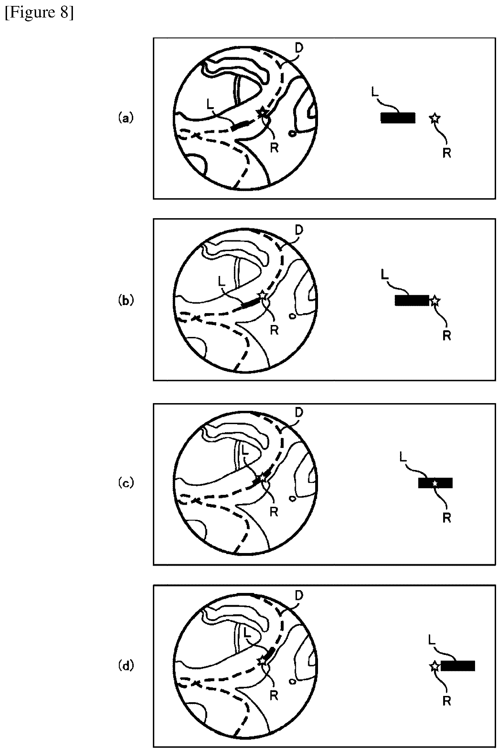

Figures 8(a)-(d) are figures for illustrating the timing for detaching the embolic coil E. The device D, shown in dashed lines in Figures 8(a)-(d), is a delivery wire. As shown in Figures 8(a)-(d), the delivery wire is marked with a marker L at a certain section on the wire. Figures 8(a)-(d) show a general example of the marker L. In addition, it may be a single dotted line "- . -" or a long straight line.

When the user moves the delivery wire, the marker L also moves in the X-ray image in conjunction with the movement of the delivery wire. On the other hand, the guiding catheter used to guide the delivery wire may move slightly due to friction or other reasons as the delivery wire moves, but the amount of movement is small compared to the movement of the delivery wire. Therefore, the user sets the region of interest R in advance at the position of the guiding catheter corresponding to the position where the marker L should be when the embolic coil E reaches the aneurysm A.

As shown in Figure 8(a)-(d), the marker L is present over a certain section on the wire. When the user moves the delivery wire, the tip portion of the marker L comes into contact with the region of interest R, as shown in Figure 8(b). This indicates that the embolic coil E has come close to the aneurysm A.

When the user further moves the delivery wire, the marker L and the region of interest R are superimposed, as shown in Figure 8(c). The notification unit 113 takes the superimposition of the marker L and the region of interest R as a trigger to begin the action of notifying the user of the timing for cutting and detaching the embolic coil E from the delivery wire.

Specifically, as shown by the symbol W2 in Figure 7(a), the notification unit 113 causes the display device 2 to show information W2 indicating the distance that the marker L should travel before the embolic coil E is cut from the delivery wire, as triggered by the superimposition of the marker L and the region of interest R.

More specifically, the user sets the region of interest R so that the end portion of the marker L passes through the region of interest R just when the embolic coil E reaches the aneurysm A, as shown in Figure 8(d). In this case, the information W2 displayed by the notification unit 113 on the display device 2 will show the distance until the end portion of the marker L passes through the region of interest R.

When the marker L passes through the region of interest R, the notification unit 113 further notifies the user of this fact. This allows the user to notice the timing for detaching the embolic coil E from the delivery wire, even if the user is concentrating on the image of the aneurysm A taken as shown in Figure 7(b).

### (Shape of the device D)

Next, as yet another example of the conditions set for the region of interest R, conditions related to the shape of the device D (for example, guide wire) will be described.

When a feature value indicating the shape of the device D included in the region of interest R satisfies a predetermined condition, the notification unit 113 notifies the user of the image processing apparatus 1 of this fact. Specifically, the notification unit 113 notifies based on the curvature of the device D included in the region of interest R or a feature value indicating the "deflection" of the device D included in the region of interest.

When the user tries to advance the device D while the tip portion of the device D is caught in a vessel wall or the like, the tip part of the device D is bent. Generally, when the tip portion of the device D is bent, elastic energy is accumulated in that portion. The more the tip portion of device D bends, i.e., the greater the curvature (smaller the radius of curvature) of the device D, the greater the amount of this elastic energy is accumulated. As the accumulated amount of elastic energy increases, the elastic force of the device D can cause release of connection of the tip portion and the tip part may move at a higher speed. As a result, the tip portion of the device D may suddenly disappear from the X-ray image.

Therefore, the notification unit 113 notifies the user on the condition that the curvature of the device D included in the region of interest exceeds a predetermined threshold curvature or that the curvature is changing but the tip is not moving. It is also possible to take into account that the tip of device D is immobile or that the distance moved is lower than a certain threshold value. The "predetermined threshold curvature" is the "reference curvature for judgment of notification" established for the notification unit 113 to judge whether or not there is a high probability that the tip portion of the device D moves at a high speed. The specific value of the predetermined threshold curvature can be determined by experimentation, taking into consideration the frequency of notification by the notification unit 113, usability, material and size of the device D, elastic modulus, or such.

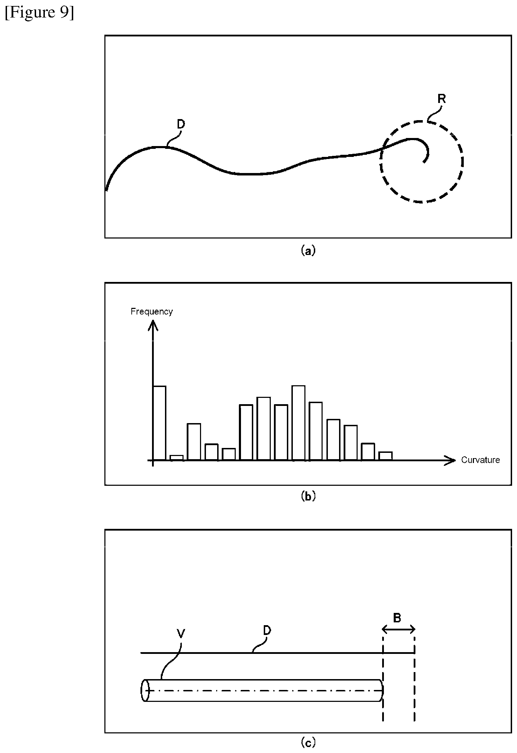

Figures 9(a)-(c) are figures for illustrating conditions related to the shape of device D set in the region of interest R. Specifically, Figures 9(a)-(b) are figures for illustrating notifications based on the curvature of the device D. In Figure 9(a), the region of interest R is indicated by a dashed circle. Without limitations, as an example, the region of interest R in Figure 9(a) is a circle with a radius of 1 cm centered at the tip portion of the device D. Figure 9(b) is a histogram showing the distribution of the curvature of the device D included in the region of interest R. Specifically, Figure 9(b) shows the distribution of the radius of curvature of the device D in each small region by dividing the device D included in the region of interest R into multiple small regions and determining the radius of curvature of the device D in each small region.

In the histogram showing the distribution of the curvature of the device D, the notification unit 113 notifies the user on the condition that a feature value calculated from the distribution of the curvature (for example, a statistic such as an average value, a mode value, or a median value of the curvature) exceeds a predetermined threshold curvature or that the tip does not move despite a change in the curvature. In this way, the image processing apparatus 1 can provide an opportunity for the user to notice that elastic force is accumulating in the device D.

Further, the notification unit 113 may also notify the user on the condition that the value obtained by subtracting the length of the center line of the blood vessel V included in the region of interest R from the length of the device D included in the region of interest R exceeds a predetermined threshold length (threshold value). Figure 9(c) is a schematic diagram showing the relationship between the length of the device D included in the region of interest R and the length of the center line of the blood vessel V included in the region of interest R. Although the device D and blood vessel are bent in the body of test subject P, for the convenience of explanation, the device D and blood vessel V are shown as straight lines in Figure 9(c). Further, in Figure 9(c), the center line of the blood vessel V is shown as a single dotted line.

The distance measuring unit 115 uses a blood vessel recognition engine to extract the blood vessel V in the region of interest R and track its center line to obtain its length D1. Similarly, the distance measuring unit 115 uses a device recognition engine generated using a known machine learning method or the like to extract the device D and obtain its length D2.

Generally, when a user advances a device D in a blood vessel V, the device D will meander along the wall of the blood vessel V. Therefore, the length D2 of the device D in the blood vessel V is longer than the length of the blood vessel V (length of the center line of the blood vessel V) D1, which means that the device D is deflected in the blood vessel V and elastic energy is accumulated in the device D. As the amount of this deflection increases, it is possible that some trigger will release the elastic energy and cause the device D to move significantly. As a result, the tip portion of the device D may disappear from the X-ray image.

The differential length B, which is the length D1 subtracted from the length D2 calculated by the distance measuring unit 115, can serve as an indicator of the amount of deflection of the device D in the blood vessel V. Therefore, the notification unit 113 displays the length B, which is the length D2 calculated by the distance measuring unit 115 minus the length D1, and if it is longer than the predetermined threshold length, it notifies the user of this fact. In this way, the image processing apparatus 1 can provide an opportunity for the user to notice that elastic force is accumulating in the device D.

### (Estimation of the catheter tip position using a 2nd marker)

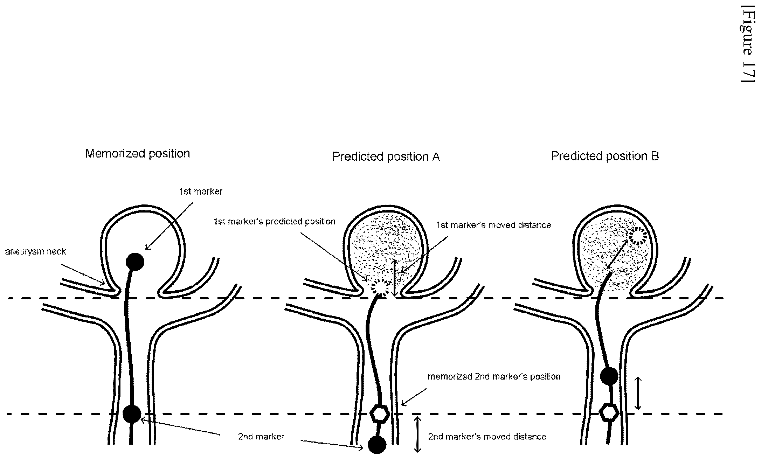

Aneurysm embolization catheters are marked with a tip marker (1st marker) and a 2nd marker (usually at a position 3 cm from the tip). It is important for the surgeon to know where the tip of the catheter is located within the aneurysm in order to perform the procedure safely. However, when the coil enters the aneurysm, the location of the tip marker becomes difficult to find and safety is reduced (see Figure 17). For example, if the tip marker moves deeper into the aneurysm, the tip, or the coil coming out of the tip, can perforate the aneurysm, leading to a serious complication of subarachnoid hemorrhage. Conversely, if the tip marker is about to exit the aneurysm, the catheter or coil must be dislodged from the aneurysm and reinserted into the aneurysm, and this operation involves the risk of perforation of the aneurysm wall.

For example, in Figure 17, the figure on the left shows the state in which the microcatheter is inserted into an aneurysm. The size of the aneurysm could be, for example, 10 mm. The distance between the 1st marker and 2nd marker attached to the microcatheter is constant (usually 30 mm). For example, suppose that the positions of the 1st marker and 2nd marker are recorded in this state, respectively (memorized position). The middle figure in Figure 17 shows the state in which the position of the microcatheter has moved. Once the coil enters the aneurysm, the position of the 1st marker becomes invisible or hard to see. Therefore, the position of the 1st marker is estimated from the difference between the position of the 2nd marker at this point and the previously stored position of the 2nd marker. In this case, the 1st marker is predicted to be at a place approximately 0 mm from the aneurysm neck (predicted position A). The figure on the right also shows the state in which the position of the microcatheter moved. Similarly, the position of the 1st marker is estimated based on the distance moved by the 2nd marker. In this case, it is estimated to be 8 mm from the aneurysm neck.

Some embodiments of the present invention relate to a method for estimating the tip position from the movement of the 2nd marker when the tip of the catheter is in the coil (aneurysm) and its position is unknown. More specifically, it relates to a method for estimating the tip position of a catheter, comprising a step of storing the positional relationship between the tip of the catheter and the 2nd marker (for example, 3 cm apart), a step of storing the distance a between the neckline of the aneurysm and the 1st marker and the position of the 2nd marker at that time t1, a step of calculating the distance b of movement from the position of the 2nd marker at time t2, and a step of estimating the distance a-b from the aneurysm neckline to the tip of the catheter, and a step of notifying the user of the estimated distance. Here, the catheter tip, 2nd marker, and aneurysm neckline can be automatically detected by image recognition by a computer. Alternatively, they can be specified manually by a pointing device such as a mouse or a touch panel. The estimated distance a-b can be displayed on a display device. In addition, the position of the catheter tip, estimated based on the estimated distance a-b, may be displayed on the display device. An arbitrary threshold value may be set and notified when the distance deviates from it, and the speed and acceleration may be determined in addition to the distance and notified according to the value (rapid or large movements are likely to be dangerous). The distance moved can be a straight line or a distance along the curve of the catheter. Since the curved shape of the catheter may change, the length of the curve may also be used. Furthermore, the distances a, b, and a-b can be probability distributions due to changes in the shape of the catheter. For example, if there are multiple timings to be memorized first, a distribution of the distance a can be made, so the mean and variance can be used to guess the location and predict it as the most likely single point, or it can be displayed as a probability distribution in a heat map or the like. Thus, the estimated distance may be expressed by a probability distribution, and the estimated position of the catheter tip may be colored like a heat map and displayed based on the probability distribution.

In addition to measuring the distance moved, the position of the 2nd marker may be specified by the surgeon or recognized by the computer, and the position may be indicated on the display device in a semi-transparent superimposition display or by an arrow. The surgeon/assistant visually recognizes whether the tip marker has moved forward or backward by recognizing how much the current 2nd marker has shifted from this fixed display.

Some embodiments of the present invention relate to programs for executing the above methods on a computer. Further, some embodiments of the present invention also relate to an image processing apparatus and a method of operating the same for performing the above methods. Such an image processing apparatus may include, for example, a positional storage unit that stores the positional relationship between the tip of the catheter and the 2nd marker (for example, 3 cm apart), a positional storage unit that stores the distance a between the neckline of the aneurysm and the 1st marker and the position of the 2nd marker at that time t1, a distance estimation unit that calculates the distance moved b from the position of the 2nd marker at time t2 and estimates the distance a-b from the aneurysm neckline to the tip of the catheter, and a notification unit that notifies the user of the estimated distance. Furthermore, some embodiments of the present invention also relate to a cerebral aneurysm coil embolization assistance system that is equipped with the image processing apparatus described above and an image capture device that captures X-ray images of a patient (or test subject P) in the state in which a guiding catheter and a delivery wire for an embolization coil are inserted into a vessel, and transmits the images to the above image processing apparatus. In some embodiments of the present invention, instead of or in addition to the notification unit, there may be an output unit that outputs information to a device connected to the image processing apparatus.

### <Processing flow of estimating the catheter tip position using the 2nd marker>

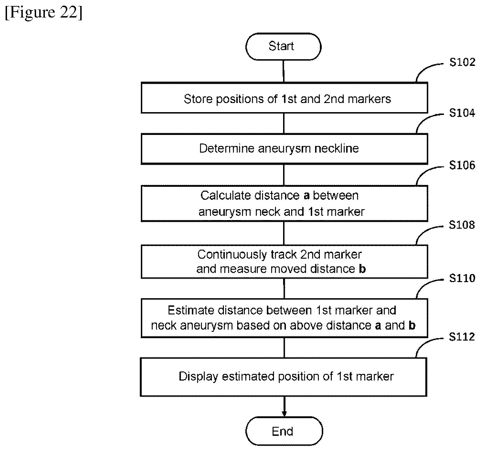

Figure 22 is a flowchart for explaining the flow of estimation processing of the catheter tip position using the 2nd marker, which is performed by the image processing apparatus according to the embodiment. The processing in this flowchart is started, for example, when the image processing apparatus 1 is started, or when the user or the image processing apparatus 1 determines that the processing needs to be started.

First, the image acquisition unit 110, region of interest acquisition unit 111, and optionally tracking unit 112, function to detect and track the device D for examination or treatment in the blood vessel V by video analysis. Then, the positions of the 1st marker and 2nd marker at that time (T = t1) are stored (S102), either automatically by image analysis or by designation of the user (operator) (S102).

Further, the line of the aneurysm neck is then determined automatically or by designation of the user (surgeon) (S104). Then, the distance A between the line of the aneurysm neck and the 1st marker is calculated (S106). At this time, the straight line or curved line distance A between the line of the aneurysm neck and the 1st marker (usually located within the aneurysm) can be measured.

Next, the 2nd marker is continuously tracked and the distance moved b is measured (S108). The 2nd marker is continuously tracked by image analysis and the movement distance b (straight line or curved line distance) of the 2nd marker at T = t2 is measured. This movement distance may be directional (plus/minus).

Then, based on the above distances A and b, the distance of the 1st marker from the aneurysm neck is estimated (S 1 10). For example, the distance of the 1st marker from the aneurysm neck is estimated to be A-b. Alternatively, it may be inferred that the 1st marker is located at a position that has been moved by b from the position of the 1st marker at T = t1 in a direction perpendicular to the aneurysm neck.

The estimated position of the 1st marker is then displayed (S112). For example, the estimated position of the 1st marker at T = t2 or the distance from the aneurysm neck can be displayed by superimposition or displayed in numerical values. If the 1st marker is about to deviate from the aneurysm near the aneurysm neck or is about to hit the aneurysm wall at the back of the aneurysm, the user (surgeon/assistant) may be additionally notified of this. If the analysis is difficult or if the user (surgeon) specifies otherwise, this function can be temporarily stopped. After this processing is completed, the processing in this flowchart may be started again, if necessary.

### (Replay function)

Since medical personnel pay attention to a specific location during a procedure, if they are not paying attention to the device that issues the warning or are looking at a different screen when the system in this disclosure issues a notification, they will try to understand the situation after the warning occurs. However, because real-time video is updated moment by moment, it is often difficult to see the details around the time when the warning is issued. In addition, depending on the nature of the warning, it may be necessary to ascertain the details of the device operation itself, the difference between the time when the warning occurred and the current time, the time that has elapsed since the warning occurred, and the like.

For example, for the guide wire tip, it is desirable to obtain information on the details of the movement of the tip portion. For example, rapid and large movements increase the risk of vascular perforation. In the case of the disappearance of a guiding catheter, it is desirable to obtain information on the position and elapsed time at the time of the catheter's exit from the image. In the case of a coil detector, it is desirable to obtain information on the deviation from the optimal point.

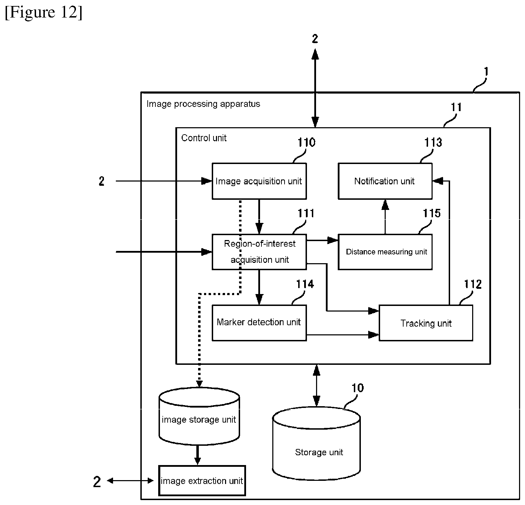

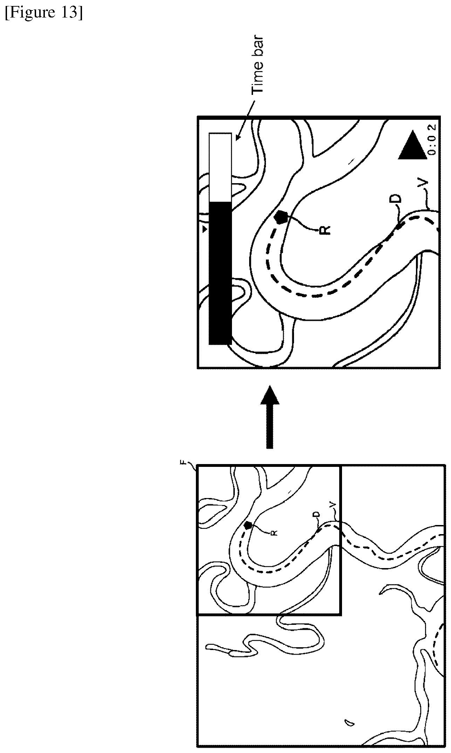

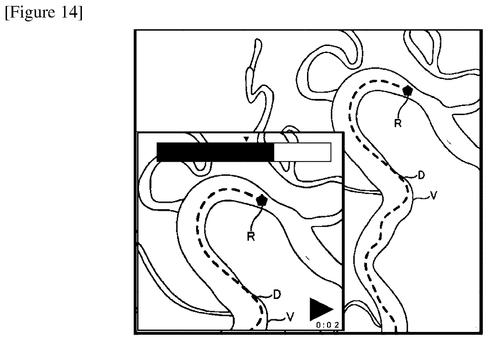

In some embodiments of the present invention, the image processing apparatus is equipped with a function (i.e., replay function) that allows the surgeon to save the image and recognition information and review it when necessary by providing the information as needed. Figure 12 shows the configuration of an image processing apparatus further equipped with an image recording unit (which can be integrated with 10) that stores the images obtained from the image acquisition unit 110 and an image extraction unit 118 that extracts some of the images before and after the time the notification occurred. Figure 13 shows an example of an enlarged display of the replay image at the time the notification occurred, with the region of interest being cropped out at the center. Figure 14 shows an example of a (an enlarged) replay playback window on the real-time display screen. In this example, the replay is enlarged and displayed in an area as far out of the region of interest as possible, which allows one to determine what happened in the area where the warning was issued within the limited area of one screen.

In some embodiments of the present invention, the image processing apparatus further comprises an image recording unit 117 that stores images (including videos) obtained from the image acquisition unit over time (continuously). In some embodiments of the present invention, the image processing apparatus further comprises an image extraction unit 118 that extracts video images from the image storage unit 117 for a certain period of time before and after the notification was issued by the notification unit. The extraction period and playback speed of the images may be automatically determined based on at least one of the movement distance, movement speed, and acceleration of the region of interest when the notification occurred. The information obtained by the region of interest acquisition unit, the tracking unit, the notification unit, the marker detection unit, and/or the distance measuring unit can also be used in the extraction processing. In some embodiments of the present invention, instead of or in addition to the notification unit, there may be an output unit that outputs information to a device connected to the image processing apparatus.

In some embodiments of the present invention, the image processing apparatus can display the extracted image on a display device. The extracted image may be automatically displayed a predetermined number of times repeatedly. The extracted image may be displayed based on any arbitrary operation including playback, stop, fast forward, rewind, frame advance, slow playback, and double-speed playback. This allows the user to easily check the images. In addition, the time elapsed from the time when the notification occurred, a comparison of the position of the region of interest at the time when the notification occurred and after any arbitrary elapsed time (the comparison display may include, for example, the relevant area, differences in the detected position, and the alignment of the images themselves), or the trajectory of the region of interest obtained by the tracking unit may be superimposed on the extracted image and further displayed on the display device.

In some embodiments of the present invention, the image processing apparatus can cut out a portion of an area in the vicinity of the region of interest from the extracted image and display it. The extracted image can be displayed in a position that does not interfere with the display of the region of interest. The extracted image may be enlarged and displayed.

In some embodiments of the present invention, the image processing apparatus can display the extracted image simultaneously with the occurrence of the notification or after a predetermined time has elapsed from the occurrence of the notification. In some embodiments of the present invention, the image processing apparatus can simultaneously display the video images taken from multiple directions.

The replay display described above may be used at a time other than at the time of occurrence of the notification. In other words, the image extraction unit 118 may extract images from the image storage unit 117 not only for a certain period of time before and after the notification is issued by the notification unit, but also for any time or period of time. For example, when the user (surgeon) feels the need to do so, he can specify any arbitrary region of interest to view a replay display of the previous scene. This allows the user to grasp and compare what happened in the region of interest while viewing the display in real time.

Some embodiments of the present invention relate to programs for executing the above methods on a computer. Further, some embodiments of the present invention also relate to an image processing apparatus and a method of operating the same for performing the above methods.

### <Processing flow of the replay display function>

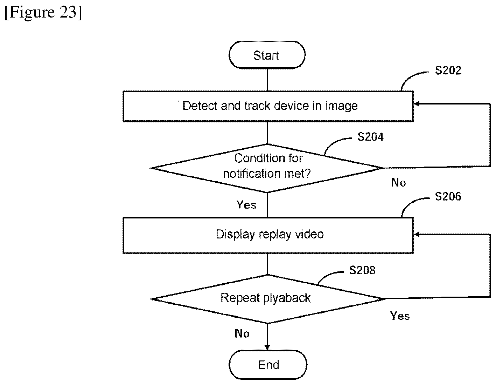

Figure 23 is a flowchart for explaining the flow of processing of the replay function performed by the image processing apparatus according to the embodiment. The processing in this flowchart starts, for example, when the image processing apparatus 1 is started.

First, the image acquisition unit 110, region of interest acquisition unit 111, and optionally tracking unit 112, function to detect and track the device D for examination or treatment in the blood vessel V by video analysis (S202).

Next, it is determined as to whether a notification condition (the distance moved exceeds a threshold value, or such) is met (S204); and if the condition is met, along with the issuance of a notification, a portion of the display screen shows the replay videos before and after the notification condition is met (S206). At this time, the real-time image is displayed as usual, and the replay video may be repeated and displayed several times so that it does not overlap with the area meeting the notification condition, or until the user (surgeon/assistant) wishes (S208). When the repetition is completed, the replay video screen is closed. After completion, the processing in this flowchart may be started again.

### (Estimation of the location of the region of interest outside the frame)

It is dangerous for the region of interest, such as the tip of a guide wire or the tip of a guiding catheter, to move outside the range of the X-ray angle of view (frame out), but the level of risk depends on the amount of movement. Specifically, for example, if the tip of the guide wire is slightly (within 5 mm or such) outside the frame, the possibility of vascular perforation is low, but if the tip is significantly outside the frame (20 mm or more, or such), the risk of vascular perforation is high, and when the region of interest is framed out, it is necessary to pull it back within the angle of view of the X-ray image, but it may not be possible to deal with it immediately depending on the situation. In such cases, it is important to know how far the framed-out region of interest has moved out of the X-ray image angle and how dangerous such movement is. Accordingly, some embodiments of the present invention relate to a device for estimating and displaying the position, speed, and acceleration status of a framed-out region of interest.

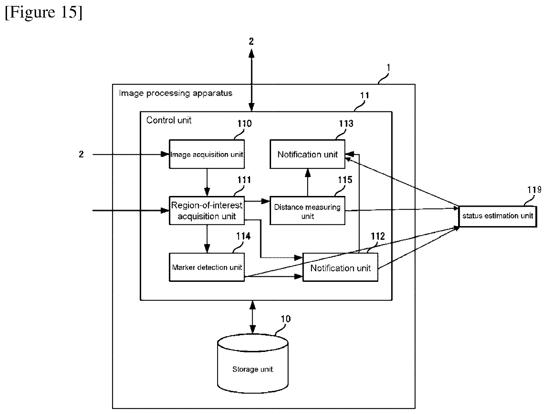

The estimation of the position of such an out-of-frame region of interest is performed, for example, by an image processing apparatus, which is an image processing apparatus comprising an image acquisition unit for acquiring an image including at least a device for examination or treatment in a blood vessel as a subject, a region of interest acquisition unit for acquiring one or more regions in the image that include at least a portion of the device as a region of interest, a tracking unit that tracks each of the regions of interest in the image, and a notification unit that notifies the user of the image processing apparatus when a region including the tip portion of a catheter or the tip portion of a guide wire is set as the region of interest, on the condition that the region of interest disappears from the image, and a status estimation unit that estimates the current position and/or speed of the tip portion of the catheter or the tip portion of the guide wire based on the position, speed and/or acceleration of the tip portion of the catheter or the tip portion of the guide wire immediately before the region of interest disappears from the image (Figure 15). The current position and/or acceleration of the region of interest estimated by the status estimation unit 119 can be displayed on a display device. In some embodiments of the present invention, instead of or in addition to the notification unit, there may be an output unit that outputs information to a device connected to the image processing apparatus.

Here, the status estimation unit 119 can store the output from the region-of-interest acquisition unit, marker detection unit, and tracking unit chronologically from before the frame-out to estimate the position of the framed-out region-of-interest, and use the stored output to calculate the position, speed, acceleration, and other states of the region-of-interest. When the region of interest frames out and tracking on the screen becomes impossible, the position, speed or such of the region of interest is estimated from the state prior to the frame-out and the user is notified. Estimation methods for this include, but are not limited to, learning-based methods using deep learning (CNN, RNN, WaveNet, or such) and Bayesian estimation methods (Kalman filter, extended Kalman filter, ensemble Kalman filter, particle filter, or such).

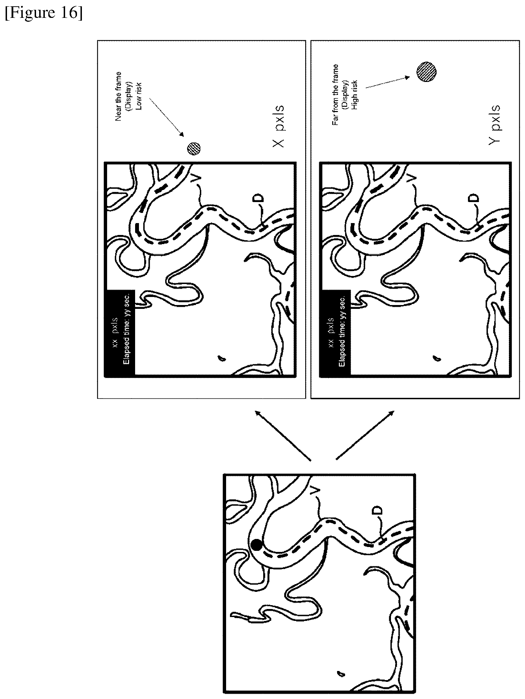

In addition, some embodiments of the present invention relate to a device that calculates the level of risk from the estimated state and issues a notification to the user according to the level of risk. The position, speed, and risk level of the region of interest estimated by the status estimation unit 119 can be displayed on a display device. The display method can be, but is not limited to, display by a point, an arrow, or a heat map. For example, if the position of the estimated region of interest is more than a predetermined distance from the edge of the image, it can be determined to be at high risk (Figure 16). For example, in Figure 16, the position of the estimated region of interest is indicated by a circle, and the further away from the edge of the screen the position is estimated to be, the higher the level of risk is judged to be. In this case, for example, the risk level may be indicated by the color of the circle (for example, green is low risk level and red is high risk level). If the calculated level of risk exceeds a certain threshold, an alert can be displayed on the screen of the display device. Or, an audio notification may be provided in addition.

Accordingly, some embodiments of the present invention relate to an image processing apparatus, which is an image processing apparatus comprising an image acquisition unit for acquiring an image including at least a device for examination or treatment in a blood vessel as a subject, a region of interest acquisition unit for acquiring one or more regions in the image that include at least a portion of the device as a region of interest, and a notification unit that notifies the user of the image processing apparatus when a region including the tip portion of a catheter or the tip portion of a guide wire is set as the region of interest, on the condition that the region of interest disappears from the image, and a status estimation unit 119 that estimates the current position and/or speed of the tip portion of the catheter or the tip portion of the guide wire based on the position, speed and/or speed of the tip portion of the catheter or the tip portion of the guide wire immediately before the region of interest disappears from the image, wherein a warning is issued to the user when the current position and/or speed of the region of interest estimated by the status estimation unit 119 exceeds a predetermined threshold value. This image processing apparatus may further include a tracking unit that tracks each of the said regions of interest in the said image. In some embodiments of the present invention, instead of or in addition to the notification unit, there may be an output unit that outputs information to a device connected to the image processing apparatus.

In addition, this image processing apparatus may further be equipped with a blood vessel recognition unit that recognizes at least some of the blood vessels in the image. A notification or output may be provided if the region of interest falls within a vessel having a diameter below a threshold, or within a manually or automatically designated vessel.

Some embodiments of the present invention relate to programs for executing the above methods on a computer. Further, some embodiments of the present invention also relate to an image processing apparatus and a method of operating the same for performing the above methods.

### <Processing flow of estimating the position of the region of interest outside the frame>

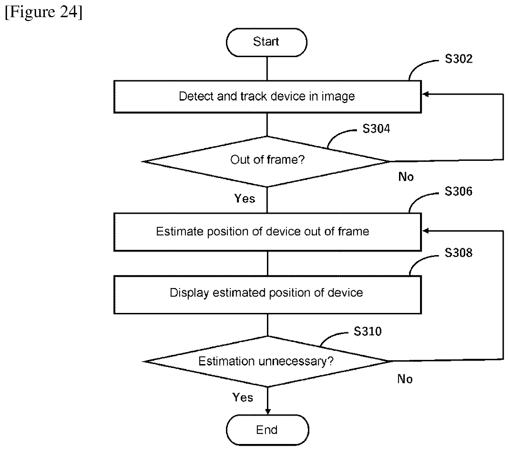

Figure 24 is a flowchart for explaining the processing flow of estimating the position of the region of interest outside the frame, which is executed by the image processing apparatus according to the embodiment. The processing in this flowchart starts, for example, when the image processing apparatus 1 is started.

First, the image acquisition unit 110, region of interest acquisition unit 111, and optionally tracking unit 112, function to detect and track the device D for examination or treatment in the blood vessel V by video analysis (S302).

Next, it is determined as to whether the site of the device D to be notified has gone outside the frame of the screen (S304) or not, and if the condition is met, the location of the device outside the frame is estimated (S306). For example, the position of the device D outside the frame is inferred from the front side of the black part of the guide wire, filter, balloon, or such, which is visible on the screen connected to the device D. The estimation can be done by using the difference in distance, machine learning, or such.

Next, the estimated position of the device is displayed (S308). The estimated position of the device D outside the frame is displayed on the display screen as the actual estimated position or distance from the frame. The further out of the frame, the stronger the display or notification may be.

Finally, whether the estimation is no longer necessary or not is determined based on the reason that the device D has returned to the inside of the frame of the screen, or that the user (surgeon/assistant) has pressed a button that does not require display (S310). After this is completed, processing in this flowchart may be started again.

### (Layout and notification)

In some embodiments of the present invention, the results of image analysis can be displayed on two screens of different sizes. As mentioned above, endovascular surgery is generally performed while viewing multiple screens (for example, four screens), and the surgeon can grasp three-dimensional information by viewing at least two screens (generally, frontal (AP or F direction: Anterior-Posterior, Frontal) and lateral (RL or LAT direction: Right to Left, Lateral). Therefore, it is important to display both frontal and lateral images, and it is very important to display them in an easily viewable manner on a monitor of limited physical size. In actual surgery, monitors are often placed over the patient's bed at a distance of 1 m or more, and instructions are often given to bring the monitor as close as 1 cm to the surgeon. The frontal and lateral views are always viewed from an angle that moves three-dimensionally, for example, 15 degrees to the right and 10 degrees to the head side using the frontal tube. In this way, even when three dimensions are projected onto two dimensions, they are viewed at an angle so that they can be seen clearly even in two dimensions. Furthermore, in the case of four screens, there are the frontal Live and Mask and the lateral Live and Mask. Live is a normal fluoroscopic image, similar to a typical radiograph, which is viewed in real time. Mask is the difference (subtraction) from any arbitrary previous Live image selected by the surgeon. As a result, the bones that were visible in Live disappear, and for example, only the blood vessels and devices that are shown by the contrast medium can be seen, and an image that is easy for the surgeon to understand can be obtained.

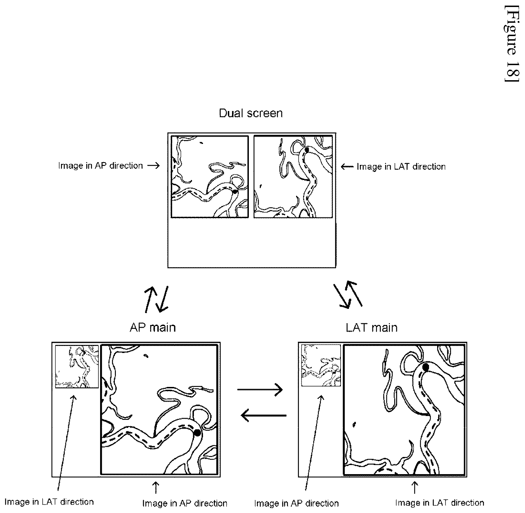

The two screens of different sizes can be switched automatically or by selection of the user (surgeon) (see Figure 18). Since there are physical limits to the screen size and one may want to see one of the screens larger than the other, visibility can be improved by displaying the two screens in different sizes, as shown in Figure 18.

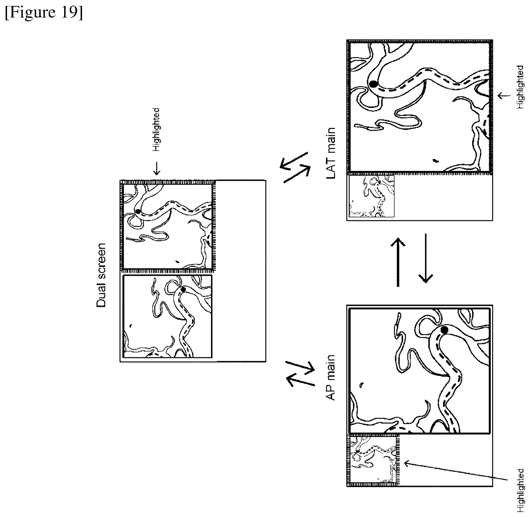

In addition, the screen to be notified can be recognized by the user by making the frame portion of the screen glow, changing the color, or highlighting the screen (see Figure 19). As shown in Figure 19, notifications with colored frames or such make it easier for the user to know which screen to pay attention to. If the notification is issued on the side of the smaller screen, the two screens may be automatically switched (thereby switching the area of attention to a larger, easier-to-see screen). As such, in some embodiments of the present invention, the display device may be equipped with a function to alert the user by making the frame portion of one of the two screens glow, changing the color, or highlighting the screen.

### (Probability-based display)

In some embodiments of the present invention, the region of interest in the screen is output as a probability distribution, so the presence of the region of interest can be expressed in terms of probability. The probability distribution of the region of interest that exists can be displayed as a numerical value, color, bar, or such. In addition, it is also possible to express the probability of whether or not a scene should be notified. Whether or not a scene should be notified can be displayed as a numerical value, color, bar, or such, depending on the probability. It is also possible to display which part of the screen is the responsible part as a probability distribution by means of a heat map or such.

The probability distribution may be converted and displayed in a way that is easy to understand. For example, 0-30% may be displayed as low, 30-70% as middle, and 70-100% as high in text or displayed in three colors. As another example, the area below 70% may be slightly darkened so that the region of interest or the region to be notified appears bright like a spotlight.

Thus, in some embodiments of the present invention, the notification unit can display an image on a display device by a numerical value, color, bar or heat map according to the probability that at least one of the regions of interest satisfies a condition defined for each of the regions of interest, or a numerical value, color, bar or heat map based on any transformation applied to the probability distribution. Further, the notification unit can color the region of interest with a color or heat map according to the probability that at least one of the regions of interest satisfies a condition defined for each of the regions of interest, or with a color or heat map based on an arbitrarily transformed value of the probability distribution, and display it on the display device displaying the image. Alternatively, it can also replace the probability that satisfies a condition with a numerical value or color and display it on the display device displaying the image.

### (Device selection display and recording)

There are a variety of devices used in endovascular therapy, and many different types exist for each of them. Examples of devices include various catheters, catheters with balloons, guide wires, stents, flow-diverting stents (stents with fine mesh), coils, embolic materials (materials such as liquids or particles), and other embolic devices (such as WEB). Further, in each of these devices, there are also various types. For example, in catheters, there are standards for the tip shape, length, lumen, outer lumen, hardness and such. For coils, hundreds of types exist, and there are standards for the manufacturer, thickness, total length, diameter, hardness, and such. It is impossible to memorize all of these, and it is also impossible to know what is in stock, so the surgeon checks with the vendor during the procedure. The combination of coils is also important, and the size of the coils is determined by looking at the images. Since 5 to 15 coils are usually used for an aneurysm, it is necessary to consider which coil to use next, but it is difficult for surgeons and assistants to remember the inventory management, lineups and such. Because new products are introduced and old products are discontinued, it is difficult to keep track of the situation, and the available lineups vary from facility to facility. At present, the selection of devices is made through communication with vendors during treatment, but this is not smooth.

First, a database of the lineup and standards of devices currently available in the country should be created. Inventory information at each facility can also be included. A system that displays this information on a monitor is built. For example, when a microcatheter is selected, the available microcatheter lineups, standards, inventory information and such are listed up and displayed. As additional information, it is also possible to search information on whether the guiding catheter can accommodate that device or multiple devices. For example, if the lumen of a guiding catheter is R and the outer diameters of two devices are r1 and r2, the two devices will fit inside the guiding catheter if R > r1 +r 2. The same idea can be applied to one device or three or more devices.

As another example, in the case of coils, the choice of coil lineups (length, diameter, hardness, shape, or such) is based on the size of the aneurysm and the behavior of the previously placed coil. There are several hundred types of coil lineups, but by specifying the length and such, it is easy to make a list. By displaying them on the monitor, it is easier to make a selection during treatment. Since most coil selections are made with the same or smaller diameters and lengths, the lineup of coils that are most likely to be used can be presented based on the information of coils used up to that point. Presentations that take inventory into consideration can also be made. In addition, lineup candidates can include preferences based on the facility and surgeon (user). Suggestions can be made based on the aneurysm or coil winding method based on image analysis. The surgeon selects the desired device from such a list and performs the surgery. This information is recorded and can be used to automatically create a surgical record, for example, in combination with snapshots of the treatment video.

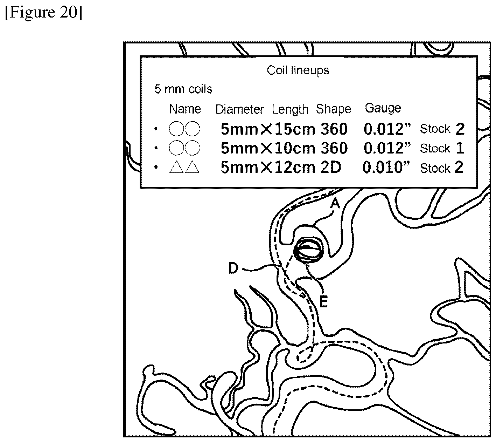

In some embodiments of the present invention, the display device that displays the image can display a product list of devices (for example, various types of catheters and coils) for examination or treatment in the blood vessel (see Figure 20). Further, the display device may also display a product list narrowed down by size or inventory. In addition, the display device may show a list of recommended products based on results of image analysis, facility information, or user preference information.

In some embodiments of the present invention, the image processing apparatus may automatically or based on the user selection create a surgical record that includes information about the device used, the images acquired, and the image analysis results.

Thus, some embodiments of the present invention also relate to a system, which is an endovascular surgical support system that includes a storage unit which stores a product list of devices (for example, various catheters and coils) for examination or treatment in a blood vessel, a recommendation unit that recommends products for use based on image analysis results, facility information, or user preference information, and a display unit that displays the recommended products.

### (Recognizing a device newly entering the screen)

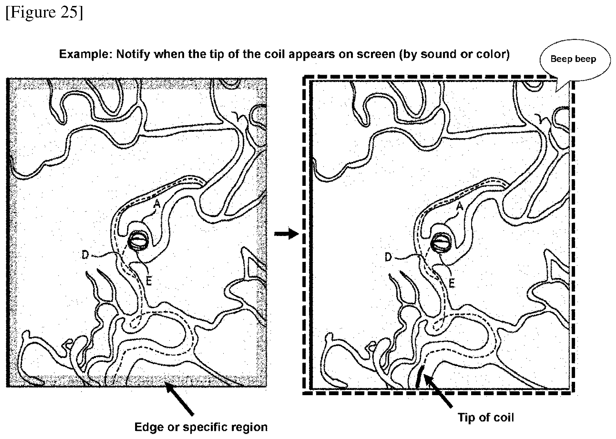

For example, when inserting a device to treat a cerebral aneurysm, the surgeon does not know when the device would reach inside the X-ray screen, so it is necessary to continuously emit X-rays and watch the screen while moving the device forward; typically, the device is moved forward quickly until the device becomes visible and slowly after the device has become visible. At this time, if the appearance of the device is overlooked and the speed is not reduced, there is the risk of vascular perforation or dissection. X-rays are irradiated in a situation in which there usually are no changes in the screen until the device becomes visible, which leads to an increase in radiation exposure for the patient as well as the surgeon, assistants, and medical personnel. Thus, in some embodiments of the present invention, the image processing apparatus can notify the user (surgeon) on the condition that a device that newly appears in the image, such as at the edge or within a set distance from the edge of the image, or within a specific region, is newly detected as a region of interest (Figure 25). More specifically, some embodiments of the present invention relate to an image processing apparatus comprising an image acquisition unit for acquiring an image for examination or treatment in a blood vessel, a region of interest acquisition unit for acquiring, when a device for examination or treatment in a blood vessel is included in the image, one or more regions that include at least a portion of the device as a region of interest, and a notification unit for notifying a user of the image processing apparatus when at least one of the regions of interest satisfies a condition defined for each of the regions of interest, wherein the notification unit notifies the user on the condition that a device or a part thereof that newly appears in an image or within a specific region, or a portion thereof, is newly detected as a region of interest. This reduces the burden of the surgeon when performing endovascular therapy.

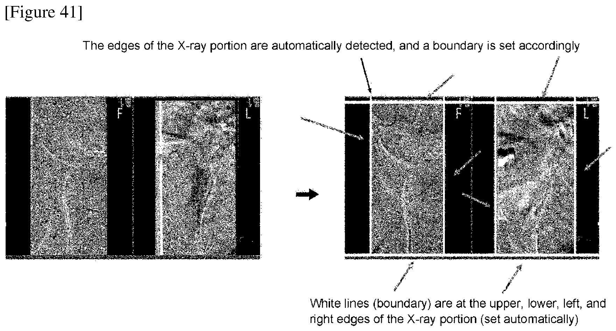

In addition, the device appears in a maximum of four screens-a frontal live image, a lateral live image, a frontal mask image, and a lateral mask image-and notification may be given on all screens or on only the first one. For example, in many cases, the timings at which the device appears from below differ between a frontal image and a lateral image; in this case, if notifications are issued on both, there will be notifications in succession, which is not comfortable for the user. Therefore, a notification may be issued when the device becomes visible in one of the screens, but a notification is omitted even if the same device appears later in the other screen. The frontal or lateral, live and mask images are often the same size, but sometimes the mask image is enlarged. In this case, since the device first appears in the live image, notification may be issued in the live image, whereas a notification is omitted even if the device later appears in the mask image. In this way, with respect to the appearance of a device on a screen, whether to notify only once or multiple times with respect to multiple screens may be set in accordance with the surgeon's preference. Furthermore, since there are cases in which the edge changes due to narrowing of the X-ray, or the like, the boundary may be automatically set to the range of the X-ray when an edge function button is pressed, for example (Figure 41). If the scene changes temporarily due to contrast imaging, or the like, the change may be detected so that a notification is not issued. Notifications may be suppressed in accordance with the image quality. For example, notifications may be temporarily suppressed if the mask image shifts and becomes difficult to see, or if the entire screen moves due to the patient's body movement.

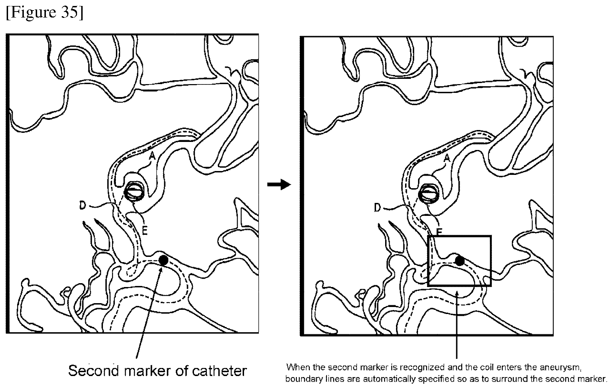

In addition, in some embodiments of the present invention, the image processing apparatus may further include a tracking unit for tracking the region of interest in the image. Here, the notification unit can notify the user on the condition that a device that newly appears at the edge or within a set distance from the edge of the image, or within a specific region, is newly detected as a region of interest. At the edge of or within a set distance from the edge of the image refers to, for example, a region within 10%, within 8%, within 5%, or within 3% of the entire length in the vertical or horizontal direction as seen from the edge of the image, but is not limited thereto. The specific region may be specified by the user or be automatically specified by the image processing apparatus. The specification by the user can be performed using a pointing device, such as a mouse or a touch panel. The specific region may be a region that the image processing apparatus determines automatically as a region that should be checked. The region that should be checked can be determined based on, for example, the tip of a guide wire being within a set range, the tip of a guiding catheter being within a set range, the tip of a stent delivery wire being within a set range, a coil marker entering within a set range, a second marker entering within a set range, the range in which a second marker of the catheter moves in order to discern the timing when the coil can be detached, a filter being within a set range, the tip of a catheter being within a set range, an embolic material being within a set range, a device being in a range remaining inside a large blood vessel, an aneurysm, or an important blood vessel, but no limitation is imposed thereby. For example, automatic determination of the region that should be checked can be performed based on a model created by means of machine learning, but no limitation is imposed thereby. The region may be manually specified by the user, or be specified automatically or semi-automatically. For example, when the second marker of the catheter is recognized and the coil enters the aneurysm or the coil marker is detected, a region is automatically set so as to surround the second marker, or, a region is proposed and the region is set when the user hits OK (Figure 35). Alternatively, for example, a rectangular region with a dimension of constant a x constant b is automatically specified at the tip of the guide wire such that the guide wire is at the center. The region may be automatically determined, be set when the user hits OK, or be set after modifying the length and width, or after translation, as necessary. The constants a/b may be changed depending on the case, the magnification rate, or the preference of the surgeon. Furthermore, the region is not limited to a rectangular shape and may be a circle, an ellipse, any closed curve, a straight line, or any curve line. In the case of a straight line or any curved line, the region is determined to be divided there. In the case of a straight line, it can be extended to divide the screen into two regions. In addition, the region may be divided along a blood vessel by means of a straight line or any curved line. That is, the region may be divided according to whether it is distal or proximal to the region delineated by the straight line or curved line, along the blood vessel. These are illustrative examples, and no limitation is imposed thereby. In some embodiments of the present invention, an output unit that outputs information to a device connected to the image processing apparatus may be provided instead of, or in addition to, the notification unit. Specifically, for example, information may be output to a catheterization robot via the output unit to notify the surgeon through the robot, to automatically stop the advancement of the device temporarily, or to insert the device and automatically advance it until the device appears on the screen, and stop or slow down the advancement when the device appears by means of an output of the output unit. As another example, an AR/VR/MR device may be connected, and, when an output of the appearance of the device is received from the output unit, the output may be displayed or notified on the AR/VR/MR device. Of course, the present invention is not limited to this example, and includes all outputs to any other device.

### (Recognizing a device entering a specific region)

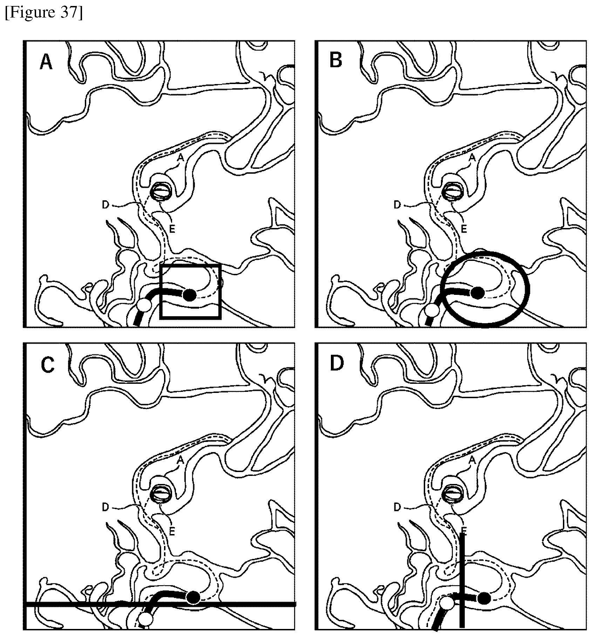

For example, when inserting a coil into an aneurysm, the guiding catheter or the intermediate catheter may move, the stent may move, the balloon may move, or the tip of the guide wire that guides the balloon may move, but the surgeon may not notice movements or changes because they are focused on the aneurysm. In some embodiments of the present invention, the image processing apparatus can notify the user (surgeon) on the condition that a device that newly appears in the image, such as within a specific region, is newly detected as a region of interest (Figure 37). More specifically, some embodiments of the present invention relate to an image processing apparatus comprising an image acquisition unit for acquiring an image for examination or treatment in a blood vessel, a region of interest acquisition unit for acquiring, when a device for examination or treatment in a blood vessel is included in the image, one or more regions that include at least a portion of the device as a region of interest, and a notification unit for notifying a user of the image processing apparatus when at least one of the regions of interest satisfies a condition defined for each of the regions of interest, wherein the notification unit notifies the user on the condition that a device or a part thereof that newly appears in an image or within a specific region, or a portion thereof, is newly detected as a region of interest. This reduces the burden of the surgeon when performing endovascular therapy. In addition, in some embodiments of the present invention, the image processing apparatus may further include a tracking unit for tracking the region of interest in the image. Here, the notification unit can notify the user on the condition that a device that newly appears at the edge or within a set distance from the edge of the image, or within a specific region, is newly detected as a region of interest.

The specific region may be specified by the user or be automatically specified by the image processing apparatus. The specification by the user can be performed using a pointing device, such as a mouse or a touch panel. The specific region may be a region that the image processing apparatus determines automatically as a region that should be checked. The region that should be checked can be determined based on, for example, the tip of a guide wire being within a set range, the tip of a guiding catheter being within a set range, the tip of a stent delivery wire being within a set range, a coil marker entering within a set range, a second marker entering within a set range, the range in which a second marker of the catheter moves in order to discern the timing when the coil can be detached, a filter being within a set range, the tip of a catheter being within a set range, an embolic material being within a set range, a device being in a range remaining inside a large blood vessel, an aneurysm, or an important blood vessel, but no limitation is imposed thereby. For example, automatic determination of the region that should be checked can be performed based on a model created by means of machine learning, but no limitation is imposed thereby. The region may be manually specified by the user, or be specified automatically or semi-automatically. For example, when the second marker of the catheter is recognized and the coil enters the aneurysm or the coil marker is detected, a region is automatically set so as to surround the second marker, or, a region is proposed and the region is set when the user hits OK (Figure 35). Alternatively, for example, a rectangular region with a dimension of constant a x constant b is automatically specified at the tip of the guide wire such that the guide wire is at the center. The region may be automatically determined, be set when the user hits OK, or be set after modifying the length and width, or after translation, as necessary. The constants a/b may be changed depending on the case, the magnification rate, or the preference of the surgeon. Furthermore, the region is not limited to a rectangular shape and may be a circle, an ellipse, any closed curve, a straight line, or any curve line. In the case of a straight line or any curved line, the region is determined to be divided there. In the case of a straight line, it can be extended to divide the screen into two regions. In addition, the region may be divided along a blood vessel by means of a straight line or any curved line. That is, the region may be divided according to whether it is distal or proximal to the region delineated by the straight line or curved line, along the blood vessel.

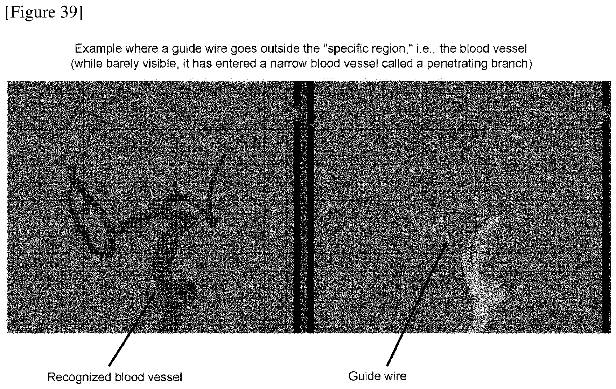

The specific region may be inside or outside a blood vessel. For example, in the case of outside of a blood vessel (Figure 39), notification is issued when the tip of the guide wire enters the specific region, that is, goes outside the blood vessel. In clinical practice, a guide wire is often inside a small blood vessel (penetrating branch), but when only either the front view or the side view is being watched, recognition may be delayed and the blood vessel may be damaged; this can be let known at an early stage by means of a notification. Of course, since the blood vessel is being penetrated, early notification can minimize complications in such cases as well.

Small blood vessels such as penetrating branches may be too small to be discerned in an X-ray image. In this case, since automatic segmentation of blood vessels would not include penetrating branches, the outside of this segmentation may be defined as the specific region in order to issue a notification when a guide wire enters a penetrating branch, for example. If penetrating branches or relatively small blood vessels (such as the ophthalmic artery or the small posterior communicating artery) can be seen in the X-ray image, a certain threshold value may be set, and blood vessels that are smaller than that may be excluded from the segmentation, so that it is possible to issue a notification when a guide wire wonders into those blood vessels. Furthermore, if there is a blood vessel that the user prefers not to be entered, the user can specify the blood vessel, thereby setting a specific region so that a notification is issued when it is entered. The specific region may be a region including lesions such as aneurysms or stenosis, or the region opposite thereof (inverted region). For example, if an aneurysm is specified (specified by the user or automatically recognized by means of segmentation of the aneurysm), and a notification is sounded when a guide wire enters the aneurysm. As a result, entry into the aneurysm can be notified, thereby prompting caution in subsequent operations. When it is desired to bring the guide wire to a distal portion of the parent vessel of the aneurysm, issuing a notice allows the user to be notified that the guide wire has entered a dangerous location (entered an undesirable location). These are illustrative examples, and no limitation is imposed thereby.

In addition, all of the specific regions may be automatically surrounded or the specific regions may be selectively surrounded in a maximum of four screens, namely, the frontal live image, the lateral live image, the frontal mask image, and the lateral mask image. For example, in the case of the tip of a guiding catheter, it suffices if it is visible in either the frontal image or the lateral image, so that the guiding catheter that is in a higher position of the two screens may be selected to set the specific region. For example, in the case of notification of a coil marker surrounding the second marker (Figure 34), a specific region may be selected only in the front view or the lateral view, in whichever it is easier to see the coil marker. The visibility of the coil marker may be determined, for example, by selecting the one in which the coil marker is more visible and appears longer than the second marker at the time of detection, or the one with the higher degree of certainty according to deep learning, but no limitation is imposed thereby.

If the scene changes temporarily due to contrast imaging, or the like, the change may be detected so that a notification is not issued. Notifications may be suppressed in accordance with the image quality. For example, notifications may be temporarily suppressed if the mask image shifts and becomes difficult to see, or if the entire screen moves due to the patient's body movement. In some embodiments of the present invention, an output unit that outputs information to a device connected to the image processing apparatus may be provided instead of, or in addition to, the notification unit. Specifically, for example, information may be output to a catheterization robot via the output unit to notify the surgeon through the robot, to automatically stop the advancement of the device temporarily, or to insert the device and automatically advance it until the device appears on the screen, and stop or slow down the advancement when the device appears by means of an output of the output unit. As another example, an AR/VR/MR device may be connected, and, when an output of the appearance of the device is received from the output unit, the output may be displayed or notified on the AR/VR/MR device. Of course, the present invention is not limited to this example, and includes all outputs to any other device.

In the case that the user sets a specific region, if there is only one device, the device may be set as the region of interest to automatically form a relationship between the specific region and the region of interest of the device. If multiple devices are included, the user may specify one, or the region of interest of a specific device may be automatically set based on the importance of the device, the position of the specific region in the screen, the surgery scene, or the like. For example, between a guide wire and a guiding catheter, the guide wire may be selected, deeming the guide wire more important. Alternatively, in the case that a guiding catheter and a guide wire enter a specific region simultaneously, normally, the guide wire is further advanced in many cases, so that the guiding catheter may be selected. In addition, if the specific region is located toward the bottom of the screen, it is likely that the specific region is set with the intention toward a guiding catheter rather than a guide wire, so that the guiding catheter may be set as the region of interest. Additionally, in a case where a coil is about to be inserted in a cerebral aneurysm embolization surgery, if a guiding catheter and a guide wire are included in the specific region, the guiding catheter may be specified as the region of interest based on the surgery scene. The aforementioned are illustrative examples; determination can be made based on the type of device, the location of the specific region, the surgery scene, the user's preference, or a previous method employed by the user.

In addition, this image processing apparatus may further comprise a blood vessel recognition unit for recognizing at least some of the blood vessels in the image. A notification may be issued or an output may be performed if the region of interest enters a blood vessel having a diameter less than or equal to a threshold value, or a blood vessel that has been specified automatically or manually.

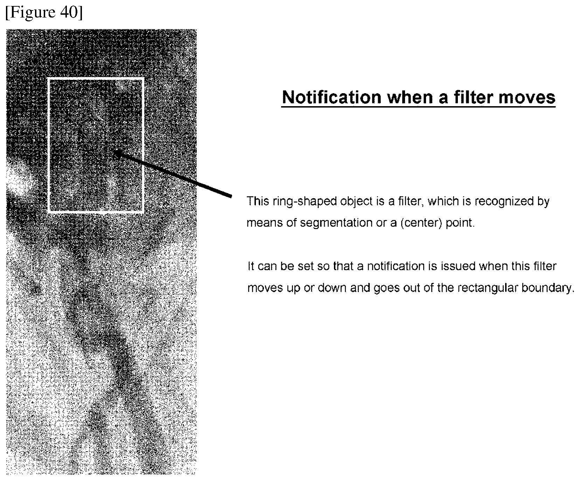

For example, a region including at least a portion of a device for examination or treatment in a blood vessel, selected from a group consisting of a guiding catheter, a guide wire, an intermediate catheter, a microcatheter, a suction catheter for thrombectomy, a marker, a coil, a stent, a filter, an embolic material, an aneurysm embolization device, and a balloon, may be set as the region of interest. For example, a region including at least a portion of the device is the tip of a device for examination or treatment in a blood vessel. In Figure 40, a notification is issued when a filter enters a specific region (outside of the square) during carotid artery stenting. That is, a notification is issued when the filter has moved a certain amount.

In addition, some embodiments of the present invention relate to a method comprising: acquiring an image that includes at least a device for examination or treatment in a blood vessel as a subject; acquiring one or more regions that include at least a portion of the device in the image as a region of interest; and notifying the user on the condition that a device that newly appears at an edge of an image is newly detected as a region of interest, as well as to a program for executing the above method on a computer. Further, some embodiments of the present invention also relate to an image processing apparatus and a method of operating the same for performing the above methods.

### <Processing flow of recognizing a device newly entering the screen)

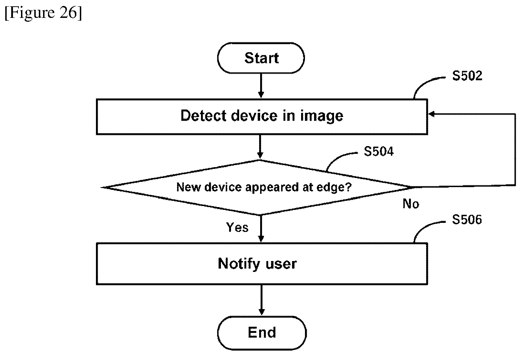

Figure 26 is a flowchart for explaining the flow of processing of the recognition of a device newly entering the screen performed by the image processing apparatus according to the embodiment. Processing in this flowchart starts, for example, when the image processing apparatus is started.

First, the image acquisition unit and region of interest acquisition unit function to detect the device for examination or treatment in the blood vessel by means of image analysis. Next, it is determined as to whether a notification condition or an output condition (a device that newly appeared at the edge of the image is newly detected as a region of interest) is met; and if the condition is met, a notification is issued or an output is performed.

Some embodiments of the present invention relate to an image processing apparatus comprising a storage device and a processor connected to the storage device, wherein the processor acquires an image for examination or treatment in a blood vessel, acquires, when a device for examination or treatment in a blood vessel is included in the image, one or more regions that include at least a portion of the device as a region of interest, and notifies a user of the image processing apparatus when at least one of the regions of interest satisfies a condition defined for each of the regions of interest, and wherein the notification is issued to the user on the condition that a device or a part thereof that newly appears in an image or within a specific region, or a portion thereof, is newly detected as a region of interest.

Furthermore, some embodiments of the present invention relate to an image processing apparatus comprising a storage device and a processor connected to the storage device, wherein the processor acquires an image for examination or treatment in a blood vessel, acquires, when a device for examination or treatment in a blood vessel is included in the image, one or more regions that include at least a portion of the device as a region of interest, and, when at least one of the regions of interest satisfies a condition defined for each of the regions of interest, outputs that information to an external device, and wherein the output is performed with respect to the external device on the condition that a device or a part thereof that newly appears in an image or within a specific region, or a portion thereof, is newly detected as a region of interest.

### (Notification based on specification of a specific boundary line)

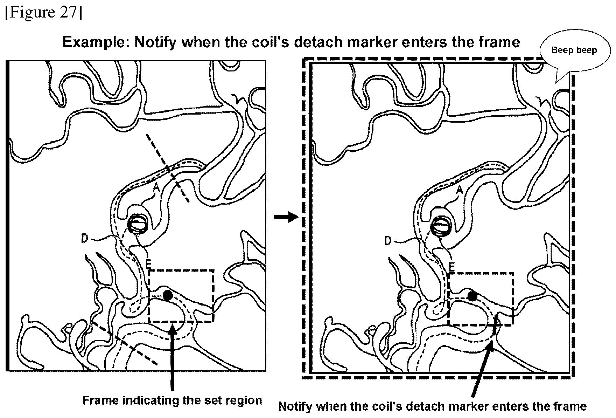

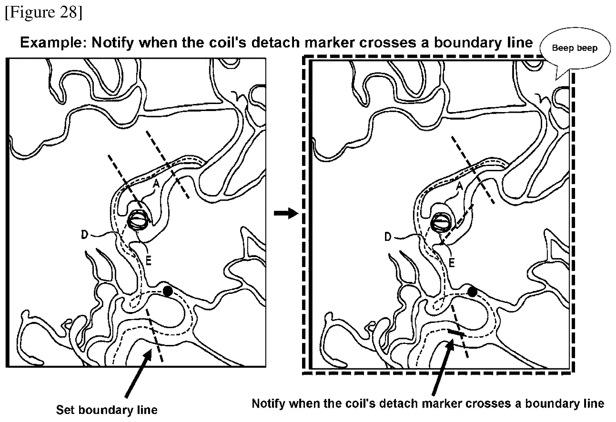

In some embodiments of the present invention, the image processing apparatus can notify the user (surgeon) on the condition that a region of interest passes, or is predicted to pass, a specific boundary line specified on an image. More specifically, some embodiments of the present invention relate to an image processing apparatus comprising an image acquisition unit for acquiring an image that includes at least a device for examination or treatment in a blood vessel as a subject, a region of interest acquisition unit for acquiring one or more regions that include at least a portion of the device included in the image as a region of interest, and a notification unit for notifying a user of the image processing apparatus when at least one of the regions of interest satisfies a condition defined for each of the regions of interest, wherein the notification unit notifies the user on the condition that the region of interest passes, or is predicted to pass, a specific boundary line specified on the image. This makes it possible to flexibly set conditions for the image processing apparatus to issue a notification (Figures 27 and 28). Prediction of passage can be performed based on the distance between the region of interest and the boundary line, or on the distance/velocity of the region of interest. This image processing apparatus may further comprise a tracking unit for tracking each of the regions of interest in the image. In some embodiments of the present invention, an output unit that outputs information to a device connected to the image processing apparatus may be provided instead of, or in addition to, the notification unit.

In addition, this image processing apparatus may further comprise a blood vessel recognition unit for recognizing at least some of the blood vessels in the image. A notification may be issued or an output may be performed if the region of interest enters a blood vessel having a diameter less than or equal to a threshold value, or a blood vessel that has been specified automatically or manually.

In some embodiments of the present invention, the specific boundary line may be specified by the user or be automatically specified by the image processing apparatus. If specification is to be performed by the user, various pointing devices, such as a mouse or a touch panel, can be used. If specification is to be performed automatically, the condition, location, etc., for carrying out such a specification may be set in advance. The specific boundary line may be represented by a straight line, a curve, a circle, a rectangle, or any other polygon or closed curve. For example, in the case that the boundary line is a closed curve, "pass" can mean both entering and exiting a region specified by the closed curve. Such a boundary line may be used, for example, to detect entry of a device into a blood vessel where entry thereto is not desirable. For example, a boundary line may be set at the entrance of a bifurcation to a particular blood vessel, to detect entry of the device into such a blood vessel.

The specific boundary line may be automatically specified when the region of interest has not moved for a certain period of time. The specific boundary line may be automatically specified in accordance with the situation of the surgery, or, is automatically proposed, which can then be accepted or rejected by the user. The specific boundary line may be automatically removed in accordance with the situation of the surgery, or, removal may be automatically proposed, which can then be accepted or rejected by the user.

If a display device that displays images has multiple screens, the specific boundary line specified in one screen may be automatically specified in a corresponding location on the other screens.

In some embodiments, the notification unit notifies the user only when the condition is met for the first time, for each of one or more regions of interest. That is, if device A and device B are set as regions of interest, a notification is issued when device A crosses the boundary line for the first time, and also when device B crosses the boundary line for the first time. In addition, in some embodiments, the notification unit notifies the user only when the condition is met for the first time after a particular point in time, for each of the one or more regions of interest. In some embodiments of the present invention, an output unit that outputs information to a device connected to the image processing apparatus may be provided instead of, or in addition to, the notification unit.

Additionally, in some embodiments, the notification unit notifies the user only when the condition is met for the first time, for all of the one or more regions of interest. That is, if device A and device B are set as regions of interest, a notification is issued only when one of device A and device B crosses the boundary line for the first time, but a notification is not issued when the other crosses the boundary line later. In addition, in some embodiments, the notification unit notifies the user only when the condition is met for the first time after a particular point in time, for all of the one or more regions of interest. In some embodiments of the present invention, an output unit that outputs information to a device connected to the image processing apparatus may be provided instead of, or in addition to, the notification unit.

Additionally, in some embodiments, the notification unit notifies the user only when the condition is met for one or more specific regions of interest specified by the user. That is, if device A and device B are set as regions of interest, and the user has specified notifications only for device A, a notification is issued only when device A crosses the boundary line for the first time, and a notification is not issued when device B crosses the boundary line. In some embodiments of the present invention, an output unit that outputs information to a device connected to the image processing apparatus may be provided instead of, or in addition to, the notification unit.

Furthermore, in some embodiments, the notification unit notifies the user only when the condition is met for one or more regions of interest that have been specified automatically. That is, if device A and device B are set as regions of interest, and the image processing apparatus is performing notifications only for device A, a notification is issued only when device A crosses the boundary line for the first time, and a notification is not issued when device B crosses the boundary line. In some embodiments of the present invention, an output unit that outputs information to a device connected to the image processing apparatus may be provided instead of, or in addition to, the notification unit.

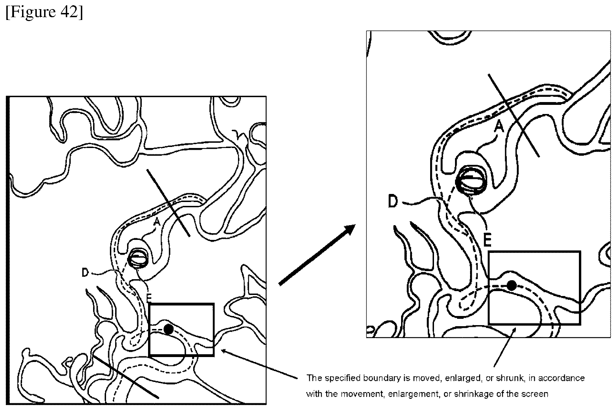

In some embodiments, the boundary line is superimposed and displayed on an X-ray image. The boundary line may be redrawn in accordance with changes in the extent or magnification of the X-ray image. This can be done automatically, or be proposed to the user, and be redrawn if the user presses OK and kept as is if the user presses No. For example, if the extent of the X-ray image is rotated or moved up, down, left, or right, the boundary line is redrawn so as to move or rotate accordingly. In addition, for example, if the extent of the X-ray image is moved, enlarged, or shrunk, the boundary line is redrawn so as to be moved, enlarged, or shrunk accordingly (Figure 42) . In some embodiments, the specific boundary line is redrawn after interruption and reacquisition of the X-ray image. Notification may be issued by means of generation of a warning sound or by changing the display mode of the boundary line. Furthermore, different methods of notification may be used in accordance with the direction in which a region of interest passes the specific boundary line specified on the image. For example, if a boundary line is set at the entrance of a bifurcation to a particular blood vessel to detect entry of a device into a blood vessel where entry is not desirable, different notifications (different sounds or colors) may be used when entry of a device into such a blood vessel is detected, and when the device is returned outside of such a blood vessel. In some embodiments, the direction in which a region of interest passes a specific boundary line specified on the image may be automatically specified in accordance with the situation of the surgery, or, is automatically proposed, which can then be accepted or rejected by the user. Furthermore, different methods of notification may be used in accordance with the speed at which a region of interest crosses the boundary line.

In some embodiments, the boundary line may be automatically deleted when the scene changes, or deletion may be proposed to the user. If proposed, it will be deleted if the user hits OK. For example, if the working angle is changed, the shape of the blood vessel changes on the 2D screen of the X-ray image, so that it becomes meaningless to maintain the boundary, and thus the boundary is deleted. Alternatively, in cases such as when the stand is moved and the screen moves a great distance so that the boundary goes outside of the X-ray screen, the boundary is deleted. The aforementioned is merely an example; deletion of the boundary line may also be proposed when switching scenes, when there is much noise, when there are large body movements of the patient, and the like.

In some embodiments, the notification unit may display the distance between the region of interest and the specific boundary line on a display device that displays the image, and change the manner in which the distance is displayed on the display device according to the length of the distance between the region of interest and the specific boundary line. The notification unit may notify the user on the condition that the value obtained by dividing the distance between the region of interest and the specific boundary line by the movement speed of the region of interest in the image is less than a predetermined threshold value. In some embodiments of the present invention, an output unit that outputs information to a device connected to the image processing apparatus may be provided instead of, or in addition to, the notification unit.

In addition, some embodiments of the present invention relate to a method comprising: acquiring an image that includes at least a device for examination or treatment in a blood vessel as a subject; acquiring one or more regions that include at least a portion of the device in the image as a region of interest; tracking each of the regions of interest in the image; and notifying the user, or outputting to an external device, on the condition that the region of interest passes, or is predicted to pass, a specific boundary line specified on the image, as well as to a program for executing the above method on a computer. Prediction of passage can be performed based on the distance between the region of interest and the boundary line, or on the distance/velocity of the region of interest. Further, some embodiments of the present invention also relate to an image processing apparatus and a method of operating the same for performing the above methods.

Some embodiments of the present invention relate to an image processing apparatus comprising a storage device and a processor connected to the storage device, wherein the processor acquires an image that includes at least a device for examination or treatment in a blood vessel as a subject, acquires one or more regions that include at least a portion of the device in the image as a region of interest, and tracks each of the regions of interest in the image, and wherein the user is notified on the condition that the region of interest passes, or is predicted to pass, a specific boundary line specified on the image.

Furthermore, some embodiments of the present invention relate to an image processing apparatus comprising a storage device and a processor connected to the storage device, wherein the processor acquires an image that includes at least a device for examination or treatment in a blood vessel as a subject, acquires one or more regions that include at least a portion of the device in the image as a region of interest, and tracks each of the regions of interest in the image, and wherein the output to an external device is performed on the condition that the region of interest passes, or is predicted to pass, a specific boundary line specified on the image.

### (Specifying the region of interest)

For example, if it is necessary for a user (surgeon) to specify a device on a screen using a pointing device such as a mouse or a touch panel during surgery, it is not easy to precisely select a moving device during surgery. For example, there are cases in which a device exhibits unintended movement during catheter treatment. Therefore, if a device could be selected within a certain area of a screen by enclosing the area with a given shape, such as a rectangle, the device is less likely to be missed, even if moving. Thus, in some embodiments of the present invention, the image processing apparatus can acquire a region of interest included in a region specified by the user on the image as the region of interest. More specifically, some embodiments of the present invention relate to an image processing apparatus, comprising an image acquisition unit for acquiring an image that includes at least a device for examination or treatment in a blood vessel as a subject, a region of interest acquisition unit for acquiring one or more regions that include at least a portion of the device included in the image as a region of interest, and a notification unit for notifying a user of the image processing apparatus when at least one of the regions of interest satisfies a condition defined for each of the regions of interest, wherein a region of interest included in a region specified by a user on the image is acquired as the region of interest. This image processing apparatus may further comprise a tracking unit for tracking each of the regions of interest in the image. In some embodiments of the present invention, an output unit that outputs information to a device connected to the image processing apparatus may be provided instead of, or in addition to, the notification unit.

In addition, this image processing apparatus may further comprise a blood vessel recognition unit for recognizing at least some of the blood vessels in the image. A notification may be issued or an output may be performed if the region of interest enters a blood vessel having a diameter less than or equal to a threshold value, or a blood vessel that has been specified automatically or manually.

In some embodiments, the region specified by the user on the image is represented by a rectangle, a circle, or any closed curve. Candidates for regions that can be specified by the user may be automatically displayed. In addition, in order to facilitate the user's specification on the screen, the image may be temporarily frozen when the user specifies a region. Alternatively, the image playback may be temporarily slowed (played in slow motion) when the user specifies a region. Furthermore, a portion of the image may be displayed enlarged when the user specifies a region. In some embodiments, candidates for regions that can be specified by the user may be automatically displayed in accordance with the situation of the surgery.

In some embodiments, if a region including multiple regions of interest is specified, only the region of interest that should be checked may be selected automatically. That is, even if multiple regions of interest are included in the specified region, unimportant regions of interest are excluded from the specification. The condition for determining a region of interest that should be checked may be, for example, set in advance by the user. Alternatively, this condition may be automatically set by means of machine learning. For example, the region of interest that should be checked may be determined in accordance with the situation of the surgery. The situation of the surgery can be automatically recognized by the image processing apparatus. The region that should be checked can be determined based on, for example, the tip of a guide wire being within a set range, the tip of a stent delivery wire being within a set range, the range in which a second marker of the catheter moves in order to discern the timing when the coil may be detached, a filter being within a set range, the tip of a catheter being within a set range, an embolic material being within a set range, a device being in a range remaining inside a large blood vessel, an aneurysm, or an important blood vessel.

Furthermore, some embodiments of the present invention relate to a method comprising: acquiring an image that includes at least a device for examination or treatment in a blood vessel as a subject; acquiring one or more regions that include at least a portion of the device included in the image as a region of interest; tracking each of the regions of interest in the image; and notifying a user of the image processing apparatus when at least one of the regions of interest satisfies a condition defined for each of the regions of interest, wherein a region of interest included in a region specified by a user on the image is acquired as the region of interest, as well as to a program for executing the above method on a computer. Further, some embodiments of the present invention also relate to an image processing apparatus and a method of operating the same for performing the above methods.

Some embodiments of the present invention relate to an image processing apparatus comprising a storage device and a processor connected to the storage device, wherein the processor acquires an image that includes at least a device for examination or treatment in a blood vessel as a subject, acquires one or more regions that include at least a portion of the device included in the image as a region of interest, tracks each of the regions of interest in the image, and notifies a user of the image processing apparatus when at least one of the regions of interest satisfies a condition defined for each of the regions of interest, and wherein a region of interest included in a region specified by a user on the image is acquired as the region of interest.

Some embodiments of the present invention relate to an image processing apparatus, comprising a storage device and a processor connected to the storage device, wherein the processor acquires an image that includes at least a device for examination or treatment in a blood vessel as a subject, acquires one or more regions that include at least a portion of the device included in the image as a region of interest, tracks each of the regions of interest in the image, and, when at least one of the regions of interest satisfies a condition defined for each of the regions of interest, outputs that information to an external device, and wherein a region of interest included in a region specified by a user on the image is acquired as the region of interest.

### (Recognizing the situation of the surgery)

When recognizing a device in real time during surgery, inferences made under situations such as angiography, puncture, noise generation, and the like, may lead to erroneous recognition results. Therefore, it is desirable to automatically recognize situations of specific surgeries and take measures such as stopping the device recognition depending on the situation. Thus, some embodiments of the present invention relate to an image processing apparatus comprising an image acquisition unit for acquiring an image including at least a device for examination or treatment in a blood vessel as a subject, and a situation recognition unit for recognizing the situation of a specific surgery based on the image.

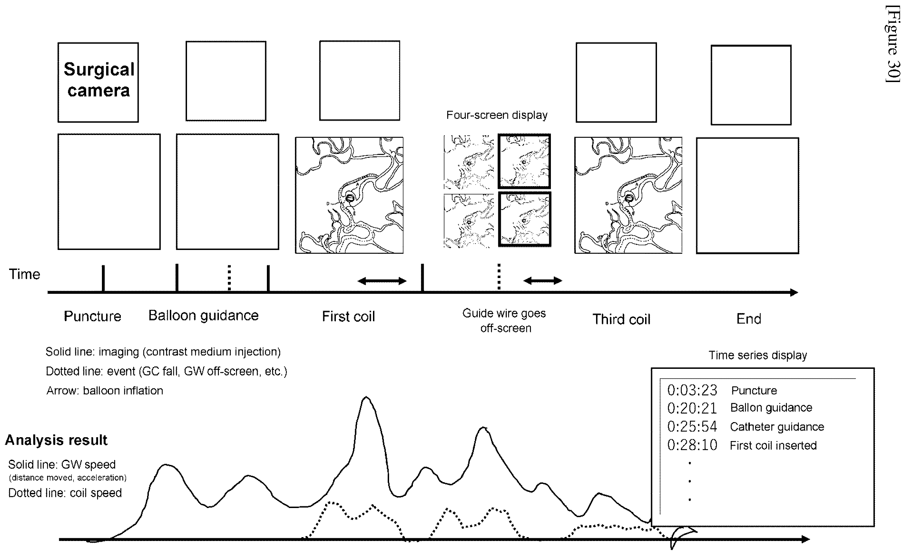

For example, the specific surgery situation may be at least one situation selected from a group consisting of the content of the surgery, the disease name, vascular leakage, vascular perforation, occurrence of thrombus, disappearance of peripheral blood vessels, guiding a guide wire/a catheter/a balloon, aspiration by catheter, stent placement, balloon inflation, coil insertion, insertion of an aneurysm embolization device (for example, a pouch-shaped embolization device WEB (Terumo Corporation) or a self-expanding implant PulsRider (Cerenovus)), the timing when a coil may be detached, guiding/placing/retrieving a filter, injection of liquid embolic material, injection of contrast agent, continuation of a still image for a certain period of time or more, discrimination between a mask image and a live image, determination of imaging site/angle, switching of images, and occurrence of noise. The specific surgery situation can be recognized by means of pattern matching, image recognition, time-series image recognition, time-series differencing, or object detection algorithms. For example, switching of a scene can be recognized by taking a time-series difference, and determining that the scene (surgery situation) has switched when the difference is greater than a certain threshold value. In addition, noise can be classified as no noise, slight noisy, or noisy, and still images may be subjected to classification learning by means of machine learning techniques such as CNN, in order to recognize noise. Balloon inflation can be determined by recognizing the presence of an inflated balloon through object recognition such as SSD in machine learning or learning through segmentation using U-Net (Figure 30). Figure 30 shows an example in which X-ray images or videos of hands, etc., of specific surgeries, such as puncture, balloon guidance, and coil insertion, are recognized, displayed, and recorded. Events such as imaging (contrast medium injection) or a guiding catheter or guide wire going off-screen, time periods during which a balloon is inflated, and the like, may also be displayed. For example, by clicking, it is possible to jump to the corresponding event and quickly recap the surgery. Two or four screens are displayed using thumbnails, as needed. If an event occurs, the screen in which it occurred is clearly displayed, such as by surrounding it with a different color frame. By recording the timeline of these events, as well as the types and number of events, it is possible to record important elements of the surgery (e.g., time until the guiding catheter was raised, the number of times that the guiding catheter fell, the number of balloon inflations and average inflation time). These will serve as feedback to the surgeon and assistants, and is expected to lead to subsequent improvements in procedure. In addition, as shown in Figure 30, changes in the speed of the guide wire and the coil may also be recorded (which can be displayed if the guide wire and the coil can be recognized and tracked by means of deep learning, or the like). By means of the foregoing, or, by converting the foregoing to a histogram or mean/variance, it is possible to express the characteristics of the surgery or of the surgeon's skill. For example, if the guide wire is moved slowly near an aneurysm but is moved relatively quickly in other areas, it suggests a high level of skill. This can be compared between different surgeons to ascertain the characteristics of their skills and provide an educational guide on what could be done to become a more skilled surgeon. Also, for example, by tracking the progress of the same surgeon for three years, it is possible to show how much improvement has been made (a record of one's own technical growth as a surgeon). In addition, there are cases in which the hands are imaged with a surgical camera, and by recording this on the same timeline, it is possible to see the situation of the hands when a certain event occurs, for example. Another benefit is that it is possible to easily review the movements of the hands when a skilled surgeon inserts a catheter into an aneurysm (in the prior art, because the timelines of the surgical camera and the X-ray images do not match, it is difficult to check how the hands were actually moving with respect to an operation that is visible in the X-ray images). Furthermore, as described in the lower right of Figure 30, it is possible to create a list of events (puncture, guiding of the guiding catheter, etc.) and the time course. This can assist in the creation of a surgical record. Furthermore, times when the picture is not moving can also be recorded. This would mean that X-ray fluoroscopy is occurring but the picture is not moving, which indicates that there is unnecessary fluoroscopy taking place. While it is difficult to constantly determine whether there is unnecessary X-ray fluoroscopy occurring during surgery, if is confirmed later with this system that there was a relatively long period of unnecessary X-ray fluoroscopy, feedback is provided to the surgeon that the fluoroscopy time should be shortened. As a result, X-ray fluoroscopy time can be gradually shortened, which could lead to reduction in exposure to the patient and medical personnel.

In some embodiments, the image processing device may further comprise a region of interest acquisition unit for acquiring one or more regions that include at least a portion of the device in the image as a region of interest, and a notification unit for notifying a user of the image processing apparatus when at least one of the regions of interest satisfies a condition defined for each of the regions of interest. In addition, in some embodiments, the device to be recognized can be specified in accordance with the specific surgery situation that has been recognized. For example, when performing carotid artery stenting, a coil normally does not appear, so even if a coil is erroneously recognized, it can be ignored, thereby improving recognition accuracy. In addition, if an aneurysm is recognized and if it can be known which process is currently being performed, it is possible to determine what type of device should be located where, thereby improving accuracy. In some embodiments of the present invention, an output unit that outputs information to a device connected to the image processing apparatus may be provided instead of, or in addition to, the notification unit.

In some embodiments, a specific boundary line for notifying a user on the condition that a region of interest passes the specific boundary line specified on the image may be automatically specified, or proposed around the region of interest, in accordance with the specific surgery situation that has been recognized.

For example, if occurrence of noise is recognized by the situation recognition unit, acquisition of the region of interest can be stopped. In addition, if continuation of a still image of a certain period of time or longer is recognized, a recommendation to reduce exposure can be notified to the user.

In some embodiments, when injection of a contrast medium is recognized, when a 3D image is acquired, or when a cone beam CT (CBCT) is acquired, acquisition of the region of interest may be stopped, or, may be acquired but not notified.

In some embodiments, the image processing apparatus further includes a recording unit for recording the specific surgery situation that is recognized.

Furthermore, some embodiments of the present invention relate to a method comprising: acquiring an image including at least a device for examination or treatment in a blood vessel as a subject, and recognizing the situation of a specific surgery based on the image, as well as to a program for executing the above method on a computer. Further, some embodiments of the present invention also relate to an image processing apparatus and a method of operating the same for performing the above methods.

Some embodiments of the present invention relate to an image processing apparatus comprising a storage device and a processor connected to the storage device, wherein the processor acquires an image that includes at least a device for examination or treatment in a blood vessel as a subject, and recognizes a specific surgery situation based on the image.

### (Surgery record)

Surgical videos are important from the point of education to medical personnel and explanation to patients and their families. However, for example, in the case of a cerebrovascular surgery, there are videos corresponding to a maximum of four screens, each being one to two hours long, so that viewing and editing takes time. Therefore, it is desirable to use AI to automatically detect events and classify scenes, save videos for surgical record-keeping, education, and explanations, and save stills of important images. Thus, some embodiments of the present invention relate to an image processing apparatus, comprising an image acquisition unit for acquiring an image that includes at least a device for examination or treatment in a blood vessel as a subject, a region of interest acquisition unit for acquiring one or more regions that include at least a portion of the device included in the image as a region of interest, and a recording unit for recording, when at least one of the regions of interest satisfies a condition defined for each of the regions of interest, the situation of a surgery corresponding to that condition.

The condition for recording include at least one procedure selected from a group consisting of, for example, puncture, arrival of a guiding catheter to the treatment site, balloon guidance, balloon inflation, stent deployment, catheter guidance, insertion of the first coil, insertion of the second and subsequent coils, location of the coil within the aneurysm, deviation of the coil to the parent vessel, catheter removal, coil removal, and end of procedure. The surgery situation to be recorded may include still images, video, and/or text information explaining the surgery situation. A video is, for example, a video of a certain period of time before and after the point in time at which the condition is met. The recorded information may be modifiable by the user.

In some embodiments, the image processing apparatus further includes a report creation unit for creating reports based on recorded information. The report creation unit can create a summary of a surgery based on, for example, recorded still images, video, and/or text information explaining the surgery situation.

In some embodiments, the image processing apparatus further includes a notification unit for notifying the user when a specific surgery situation has been recorded. In addition, in some embodiments, the image processing apparatus further includes a notification unit for notifying the user in accordance with the time that has elapsed after a specific surgery situation was recorded. The time that has elapsed after a specific surgery situation was recorded may be determined based on the balloon inflation time, the guide wire travel time, speed, and acceleration, or the coil travel time. For example, if X-rays are being emitted but nothing is moving in the screen, that is, if the image is still, it means that there is an increase in unnecessary X-ray exposure to the patient and medical personnel. Notifying this in real time or as a post-operative record can lead to reduction in the exposure dose of the entire surgery. For example, if the time and circumstances of this unnecessary X-ray irradiation are recorded as a post-operative record, the physician can strive to avoid such unnecessary X-ray irradiation in the future. The time required for each procedure is also important. For example, when inflating a balloon, the blood vessel is blocked, and, depending on the part of the blood vessel, it is said that inflation should not be continued for more than 3 to 5 minutes (continuation leads to cerebral ischemia and cerebral infarction). By graphing the number of balloon inflations and the respective inflation times in real time or post-operatively, feedback is provided earlier on when the balloon should be deflated. As other examples of the time required for various procedures, the times for balloon guidance, guiding the catheter to an aneurysm, and insertion of a coil, in aneurysm coil embolization can be measured to visualize where it took time in the case. As a result, it is possible to know how much time it took compared to the average or to experienced physicians, thereby leading to shorter surgery times and radiation exposure. In addition, by tracking the tip of the guide wire/catheter, for example, it can be expected that an experienced physician would operate the device carefully near an aneurysm (low speed and acceleration), and quickly in other areas. Conversely, if this balance is off, there is room for improvement. Furthermore, by visualizing these various parameters for each surgeon, it becomes possible to evaluate surgeons and to know which areas each surgeon should improve. Therefore, it becomes possible to visualize improvements in the technique of a surgeon, and also to visualize when techniques are being maintained. In some embodiments of the present invention, an output unit that outputs information to a device connected to the image processing apparatus may be provided instead of, or in addition to, the notification unit.

In some embodiments, the image processing apparatus further includes a notification unit for notifying the user in accordance with the number of times that a specific surgery situation has been recorded. An example of a specific surgery situation is the insertion of coils. In some embodiments of the present invention, an output unit that outputs information to a device connected to the image processing apparatus may be provided instead of, or in addition to, the notification unit.

In some embodiments, images are acquired from video recorded in real time or from previously recorded video.

Some embodiments of the present invention relate to an image processing apparatus, comprising a storage device and a processor connected to the storage device, wherein the processor acquires an image that includes at least a device for examination or treatment in a blood vessel as a subject, acquires one or more regions that include at least a portion of the device in the image as a region of interest, and, when at least one of the regions of interest meets a condition defined for each of the regions of interest, records the situation of a surgery corresponding to that condition.

### (Application of scene classification)

In the case of real-time surgical support, when setting an ROI, the boundary (ROI) may be proposed, made to appear, deleted, moved, enlarged, or shrunk, in accordance with the scene classification. In addition, the presence/absence and the types of recognition and notification may be changed; for example, if the scene can be classified as being during contrast imaging, recognition or notification may be stopped. This is because, in general, the purpose of contrast imaging is to see blood vessels, and devices rarely move at that time, so there is little need for a notification. However, a notification may be made. This is because, while rare, a device may move due to contrast imaging.

In addition, when making a mask image, the surgeon or an assistant needs to select a frame of the appropriate time phase after contrast imaging, which takes time and is cumbersome. By applying the scene classification function, in the case of contrast imaging, it is possible to automatically select a frame that seems to be appropriate for creating a mask image, such as a frame in which arteries can be best seen, any frame before or after such a frame, a frame in which the lesion can be best seen, or a frame close to the timing used for the mask image during the surgery. The surgeon, the assistants, and medical personnel involved in the surgery can specify the foregoing as the base for the mask image, or select an appropriate frame from the frames before or after. Additionally, it is not necessarily limited to the arterial phase; it is possible to take a frame of a time phase deemed to be appropriate for the surgery, such as the capillary phase or the venous phase.

Furthermore, some embodiments of the present invention relate to a method comprising: acquiring an image that includes at least a device for examination or treatment in a blood vessel as a subject; acquiring one or more regions that include at least a portion of the device included in the image as a region of interest; and recording, when at least one of the regions of interest satisfies a condition defined for each of the regions of interest, the situation of a surgery corresponding to that condition, as well as to a program for executing the above method on a computer. Further, some embodiments of the present invention also relate to an image processing apparatus and a method of operating the same for performing the above methods.

Some embodiments of the present invention relate to an image processing apparatus, comprising a storage device and a processor connected to the storage device, wherein the processor acquires an image that includes at least a device for examination or treatment in a blood vessel as a subject, acquires one or more regions that include at least a portion of the device in the image as a region of interest, and, when at least one of the regions of interest meets a condition defined for each of the regions of interest, records the situation of a surgery corresponding to that condition.

### (Displaying marks corresponding to the Y coordinate)

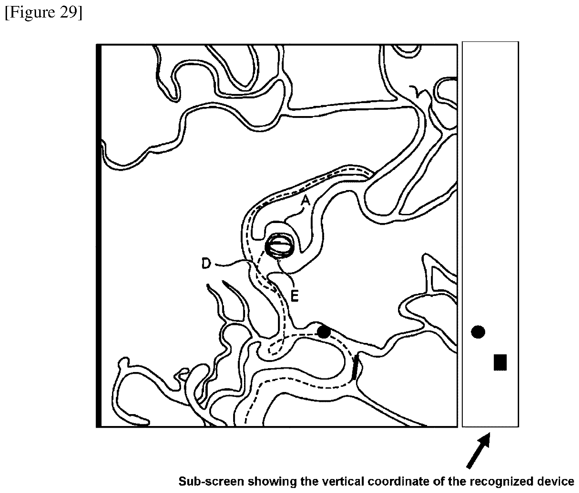

In an image containing multiple devices, if various pieces of information in the screen increases excessively, it may become difficult to ascertain momentary situations. Therefore, a sub-screen that displays only the vertical position of a device is provided next to the main surgery screen to solve such a problem (Figure 29). Thus, some embodiments of the present invention relate to an image processing apparatus, comprising an image acquisition unit for acquiring an image that includes at least a device for examination or treatment in a blood vessel as a subject, a region of interest acquisition unit for acquiring one or more regions that include at least a portion of the device included in the image as a region of interest, and a notification unit for notifying a user of the image processing apparatus when at least one of the regions of interest satisfies a condition defined for each of the regions of interest, wherein the image processing apparatus further comprises a display unit that causes a display device connected to the image processing apparatus to display the image including the region of interest, and a mark corresponding only to the vertical coordinate of the region of interest next to the image. This image processing apparatus may further comprise a tracking unit for tracking each of the regions of interest in the image. In some embodiments of the present invention, an output unit that outputs information to a device connected to the image processing apparatus may be provided instead of, or in addition to, the notification unit.

In addition, this image processing apparatus may further comprise a blood vessel recognition unit for recognizing at least some of the blood vessels in the image. A notification may be issued or an output may be performed if the region of interest enters a blood vessel having a diameter less than or equal to a threshold value, or a blood vessel that has been specified automatically or manually.

In some embodiments, the trajectory of the mark may be displayed for a certain period of time. In addition, the display mode of the mark may be different for each of multiple regions of interest. Here, the display mode may be color, shape, pattern, symbol, text, caption, or animation.

In some embodiments, the image processing apparatus can automatically recognize and highlight a region of interest that should be checked. The condition for determining a region of interest that should be checked may be, for example, set in advance by the user. Alternatively, this condition may be automatically set by means of machine learning. For example, the region of interest that should be checked may be determined in accordance with the situation of the surgery. The situation of the surgery can be automatically recognized by the image processing apparatus.

In some embodiments, only regions of interest specified by the user are displayed on the sub-screen. In addition, in some embodiments, only regions of interest selected automatically are displayed on the sub-screen.

Furthermore, in some embodiments, the image processing apparatus can notify the user if a mark exceeds a user-specified threshold value. The specification of a threshold value by the user can be performed by, for example, the user using a pointing device, such as a mouse or a touch panel, and setting a specific range on the sub-screen.

Furthermore, some embodiments of the present invention relate to a method comprising: acquiring an image that includes at least a device for examination or treatment in a blood vessel as a subject; acquiring one or more regions that include at least a portion of the device included in the image as a region of interest; tracking each of the regions of interest in the image; and notifying a user of the image processing apparatus when at least one of the regions of interest satisfies a condition defined for each of the regions of interest, wherein a display device connected to the image processing apparatus is caused to display the image including the region of interest, and a mark corresponding only to the vertical coordinate of the region of interest next to the image, as well as to a program for executing the above method on a computer. Further, some embodiments of the present invention also relate to an image processing apparatus and a method of operating the same for performing the above methods.

Some embodiments of the present invention relate to an image processing apparatus, comprising a storage device and a processor connected to the storage device, wherein the processor acquires an image that includes at least a device for examination or treatment in a blood vessel as a subject, acquires one or more regions that include at least a portion of the device included in the image as a region of interest, tracks each of the regions of interest in the image, notifies a user of the image processing apparatus when at least one of the regions of interest satisfies a condition defined for each of the regions of interest, and causes a display device connected to the image processing apparatus to display the image including the region of interest, and a mark corresponding only to the vertical coordinate of the region of interest next to the image.

### (Tracking and displaying multiple regions of interest)

Some embodiments of the present invention relate to an image processing apparatus, comprising an image acquisition unit for acquiring an image that includes at least a device for examination or treatment in a blood vessel as a subject, a region of interest acquisition unit for acquiring one or more regions that include at least a portion of the device included in the image as a region of interest, a tracking unit for tracking each of the regions of interest in the image, and a notification unit for notifying a user of the image processing apparatus when at least one of the regions of interest satisfies a condition defined for each of the regions of interest, wherein the image processing apparatus further comprises a display unit for displaying, if a plurality of the regions of interest are tracked, each of the multiple regions of interest using a different display mode. In such embodiments, since multiple regions of interest are each displayed using different display modes, it becomes easy to identify individual devices. The display mode may be, for example, color, shape, pattern, symbol, text, caption, or animation.

In some embodiments, regions of interest that should be checked are automatically recognized and highlighted. In addition, in some embodiments, the regions of interest that should be checked are determined in accordance with the situation of the surgery. The condition for determining a region of interest that should be checked may be, for example, set in advance by the user. Alternatively, this condition may be automatically set by means of machine learning. For example, the region of interest that should be checked may be determined in accordance with the situation of the surgery. The situation of the surgery can be automatically recognized by the image processing apparatus.

Furthermore, in some embodiments, the image processing apparatus may display only regions of interest specified by the user. In addition, the image processing apparatus may display only regions of interest selected automatically. When selecting automatically, a condition of making such a selection can be set in advance.

Some embodiments of the present invention relate to an image processing apparatus comprising a storage device and a processor connected to the storage device, wherein the processor acquires an image that includes at least a device for examination or treatment in a blood vessel as a subject, acquires one or more regions that include at least a portion of the device included in the image as a region of interest, tracks each of the regions of interest in the image, and notifies a user of the image processing apparatus when at least one of the regions of interest satisfies a condition defined for each of the regions of interest, and wherein, if a plurality of the regions of interest are tracked, each of the multiple regions of interest is displayed using a different display mode.

### (Exemplary system)

Some embodiments of the present invention relate to vascular catheterization support systems, for example, catheterization support systems for cerebral, cardiac, peripheral limbs, and abdominal vessels, in particular, cerebral blood vessels. Such a system is a system that can comprise an image processing apparatus and an image capture device that captures and transmits to the image processing apparatus X-ray images of a patient with one or more devices inserted into a blood vessel, wherein the image processing apparatus is equipped with an image acquisition unit that acquires X-ray images over time of a region (for example, a fixed region) that includes at least an area of interest for achieving a surgical objective and a device inserted into a blood vessel, a region of interest acquisition unit that acquires one or more regions which include at least a portion of the device included in the image as a region of interest, and a notification unit that notifies a user of the image processing apparatus when at least one of the regions of interest meets a condition defined for each of the regions of interest, and wherein the one or more of the devices are a catheter, guide wire, stent and/or balloon, and if a region including the tip portion of a catheter or the tip portion of a guide wire, both ends of the stent, and both ends of the balloon is set as the region of interest, the user is notified on the condition that the region of interest disappears from the image, the distance between the region of interest and the edge of the image is less than a predetermined threshold distance, or that the region of interest has shifted a certain distance. This system may further comprise a tracking unit for tracking each of the regions of interest in the image. In some embodiments of the present invention, an output unit that outputs information to a device connected to the image processing apparatus may be provided instead of, or in addition to, the notification unit.

In addition, this image processing apparatus may further comprise a blood vessel recognition unit for recognizing at least some of the blood vessels in the image. A notification may be issued or an output may be performed if the region of interest enters a blood vessel having a diameter less than or equal to a threshold value, or a blood vessel that has been specified automatically or manually.

Here, the notification unit may display the distance between the region of interest and the edge of the image or the distance between the marker and the region of interest on a display device that displays the image, and the notification unit can change the manner in which the distance is displayed on the display device according to the length of the distance, and the change in the display mode may include changing the font, size, or color of the characters displayed according to the length of the distance, changing the color of the entire screen or a portion of the screen of the display device according to the length of the distance, displaying a graphic on the entire screen or outside the frame of the display device or in a part of the screen, enlarging and displaying the region of interest according to the length of the distance, or changing the color or size of the mark attached to the region of interest according to the length of the distance. The notification unit may also sound a notification tone in accordance with the length of the distance. Furthermore, the distance may be determined by either a straight-line distance or a distance along a blood vessel.

Some embodiments of the present invention also relate to an aneurysm coil embolization assistance system, in particular, a cerebral aneurysm coil embolization assistance system. Such a system is a system that can be equipped with an image processing apparatus and an image capture device that captures and transmits to the image processing apparatus X-ray images of a patient in a state with a guiding catheter and a delivery wire for an embolic coil inserted into a blood vessel, wherein the image processing apparatus is equipped with an image acquisition unit that acquires X-ray images over time of a fixed area that includes at least the aneurysm in the patient's blood vessel, a catheter inserted into the blood vessel and a delivery wire for an embolization coil, a region of interest acquisition unit that acquires one or more areas that include at least a portion of the guiding catheter in the images as a region of interest, a marker detection unit that detects a marker provided on the delivery wire, which is a marker that approaches one or more regions of interest set in a portion of the catheter guiding the delivery wire, and a notification unit that notifies the user of the timing when the embolic coil may be detached from the delivery wire, triggered by the superimposition of the marker and the region of interest. This system may further comprise a tracking unit for tracking each of the regions of interest and the marker in the image. In some embodiments of the present invention, an output unit that outputs information to a device connected to the image processing apparatus may be provided instead of, or in addition to, the notification unit.

In addition, this image processing apparatus may further comprise a blood vessel recognition unit for recognizing at least some of the blood vessels in the image. A notification may be issued or an output may be performed if the region of interest enters a blood vessel having a diameter less than or equal to a threshold value, or a blood vessel that has been specified automatically or manually.

Here, the notification unit may display the distance between the region of interest and the edge of the image or the distance between the marker and the region of interest on a display device that displays the image, and the notification unit can change the manner in which the distance is displayed on the display device according to the length of the distance, and the change in the display mode may include changing the font, size, or color of the characters displayed according to the length of the distance, changing the color of the entire screen or a portion of the screen of the display device according to the length of the distance, displaying a graphic on the entire screen or outside the frame of the display device or in a part of the screen, enlarging and displaying the region of interest according to the length of the distance, or changing the color or size of the mark attached to the region of interest according to the length of the distance. The notification unit may also sound a notification tone in accordance with the length of the distance. Furthermore, the distance may be determined by either a straight line distance or a distance along a blood vessel.

### <Processing flow of the image processing method executed by the image processing apparatus 1>

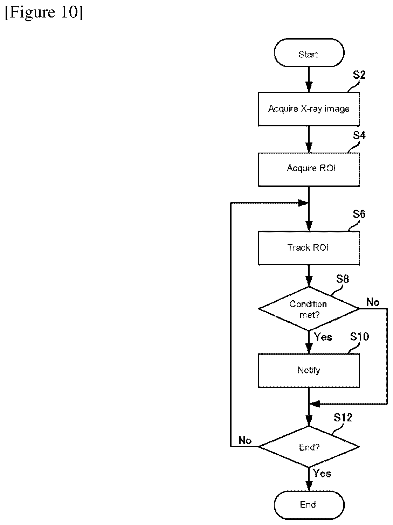

Figure 10 is a flowchart illustrating the flow of the image analysis processing performed by the image processing apparatus 1 according to the embodiment. Processing in this flowchart starts, for example, when the image processing apparatus 1 is started.

The image acquisition unit 110 acquires an X-ray image created based on the absorption rate of X-rays, which includes at least a blood vessel V and a device D for examination or treatment in the blood vessel V as a subject (S2). The region-of-interest acquisition unit 111 acquires one or more regions including at least a portion of the device D in the X-ray image as the region-of-interest R (S4).

The tracking unit 112 tracks each of the regions of interest R in the X-ray image (S6). If at least one of the regions of interest R satisfies a condition defined for each of the regions of interest R (Yes in S8), the notification unit 113 notifies the user of the image processing apparatus 1 of that fact (S10). If all the regions of interest R fail to satisfy the defined condition (No in S8), the notification unit 113 skips the notification processing.

Until the image processing is completed (No in S12), the image processing apparatus 1 returns to step S6 and repeats the processing from step S6 to step S10. When the image processing is completed (Yes in S12), the processing in this flowchart is terminated.

### <Diagnosis and comparison of angiography>

In angiographic examination and treatment, blood vessels are visualized by contrast imaging to diagnose lesions, but sometimes the images are overlooked or must be judged by comparing them with images taken the same day before or on a different day, which can be time-consuming and difficult to compare. In addition, since the images are projected in two dimensions, it can be difficult to make a judgment. Therefore, some embodiments of the present invention relate to an image diagnosis device that points out lesions or sites of lesion including, without limitations thereto, cerebral aneurysm, stenosis, occlusion, thrombus formation, vascular perforation (spillage of the contrast medium), shunt disease, nutrient vessel and tumor thickening of tumor vessels, venous thrombosis, avascular area of the capillary phase (finding of vascular occlusion), collateral circulation, and such, as determined by deep learning and other methods used in contrast-enhanced angiography. Thus, in some embodiments of the present invention, the image processing apparatus can further comprise a lesion recognition unit that recognizes a lesion selected from the group consisting of aneurysm, stenosis, vasospasm, dissection, occlusion, recanalization, thrombosis, site of thrombus and location of both ends, vascular perforation, leakage of contrast medium out of a blood vessel, calcification of a blood vessel, arteriosclerosis, shunt disease and its feeding and draining vessels, blood (contrast medium) backflow, and cerebral arteriovenous malformations, dural arteriovenous fistulas, avascular region, characteristics anatomy of bone (internal auditory canal, ocular fundus, supraorbital margin, pyramidal body, foramen magnum occipitalis, cervical spine, clavicle, rib and spine numbers, femoral head, pelvis), feeding vessels of tumor and tumor staining, venous occlusion, venous sinus thrombosis, avascular area of capillary phase, vascular occlusion, coil shape and distribution within the aneurysm, balloon position, inflation, and shape, coil deviation into the normal vessel, insufficient expansion of stent, degree of adherence to the vessel and torsion of stent, stent migration, positional relationship between puncture site and blood vessel (no stenosis, none near bifurcation), vessel tortuosity, type of aortic arch (how far down from the top of the aortic arch the right brachiocephalic artery is), extent of penetration of liquid embolic material, delay or stagnation of blood (contrast medium) flow, blood vessel variations (anterior communicating artery, anterior cerebral artery A1, posterior communicating artery, posterior cerebral artery P1, posterior inferior cerebellar artery, anterior inferior cerebellar artery, superior cerebellar artery, superficial temporal artery, presence and development of each venous sinus and each venous vein), moyamoya vessels of moyamoya disease (stenosis and occlusion of the tip of the internal carotid artery and development of collateral vessels beyond it), location of arterial bifurcations and segments (internal carotid artery conus, cavernous sinus, ophthalmic artery, middle cerebral artery M1 bifurcation), past surgery devices (clips, coils, plates, shunt tubes/valves, ventricular tubes and cistern tubes), position/openness of WEB devices, foreign bodies (dentures, plates), and collateral circulation in the said images. The system may not go so far as to point out the lesion, but may notify when an abnormal finding is suspected, or point out which areas in the region may be abnormal. In such cases, the physician can make the final decision. Similarly, some embodiments of the present invention relate to an image diagnosis device that points out changes in performing imaging of a blood vessel, when comparing with the previous imaging or imaging performed on a different day. Thus, in some embodiments of the present invention, the image processing apparatus can further comprise an image recognition unit that compares the angiographic image in the image with a previously acquired and stored angiographic image and notifies the change. For example, changes in the degree of vasospasm, changes in thrombus formation (appearance, disappearance, enlargement, reduction, or such), release of occluded vessels, vessel occlusion, coil deviation, stent movement, or such can be noted.

### <Effects of the image processing apparatus 1 according to the embodiment>

As explained above, the image processing apparatus 1 according to the embodiment can provide technology to support judgment of the area of interest in catheter examination or treatment of blood vessels by allowing the user, a medical professional, to concentrate on the work in the area of interest.

Although the present invention has been described above using the embodiments, the technical scope of the invention is not limited to the scope described in the above embodiments, and various variations and modifications are possible within the gist thereof. For example, all or part of the device can be functionally or physically distributed or integrated in any arbitrary unit. New embodiments resulting from any combination of multiple embodiments are also included in the present invention. The effects of a new embodiment created by the combination have the effect of the original embodiment.

### <Example of a first modification>

In the above, the examination and treatment of cerebral blood vessels were mainly described. However, the applicable subject of the present invention is not limited to the cerebrovascular system, but can be applied to examination and treatment of blood vessels including the circulatory system such as the heart, peripheral limbs, abdomen and such.

### <Example of a second modification>

In the above, the explanation was given using the example of a device with two screens as shown in Figures 7(a)-(b), but the number of screens is not limited to this, and for example, it can be one screen, or three screens or more.

### <Example of a third modification>

In the above, the case in which the X-ray imaging apparatus 3 captures images of the surgical site of the test subject P was explained. However, the imaging apparatus for capturing images of the test subject P's surgical site is not limited to the X-ray imaging apparatus 3. Other modalities such as MRI (Magnetic Resonance Imaging) and ultrasonography may also be used to capture images of the surgical site.

### <Examples of the embodiments>

Some or all of the above embodiments may also be described in the Supplementary Notes below, but the disclosure of this application is not limited to the following Supplementary Notes.

### (Supplementary Note 1)

An image processing apparatus, comprising:
an image acquisition unit for acquiring an image including at least a device for examination or treatment in a blood vessel as a subject,
a region of interest acquisition unit for acquiring one or more regions in the image that include at least a portion of the device as a region of interest,
a tracking unit that tracks each of the regions of interest in the image, and
a notification unit that notifies the user of the image processing apparatus when at least one of the regions of interest satisfies a condition determined for each of the regions of interest.

### (Supplementary Note 2)

The image processing apparatus according to Supplementary Note 1, wherein
when an area including a tip portion of a catheter or a tip portion of a guide wire is set as the region of interest, the notification unit notifies the user on the condition that the region of interest disappears from the image.

### (Supplementary Note 3)

The image processing apparatus according to Supplementary Note 1, wherein
when a region including the tip portion of a catheter or the tip portion of a guide wire is set as the region of interest, the notification unit notifies the user on the condition that the distance between the region of interest and an edge of the image is less than a predetermined threshold distance.

### (Supplementary Note 4)

The image processing apparatus according to any one of Supplementary Notes 1 to 3.
the notification unit notifies the user on the condition that at least one of moving distance, movement speed, and acceleration of the region of interest in the image exceeds a predetermined threshold value.

### (Supplementary Note 5)

The image processing apparatus according to any one of Supplementary Notes 1 to 4, wherein
the notification unit displays the distance between the region of interest and the edge of the image on a display device that displays the image.

### (Supplementary Note 6)

The image processing apparatus according to Supplementary Note 5, wherein
the notification unit changes the manner in which the distance is displayed on the display device according to the length of the distance between the region of interest and the edge of the image.

### (Supplementary Note 7)

The image processing apparatus according to any one of Supplementary Notes 1 to 6, wherein
the notification unit notifies the user on the condition that the value obtained by dividing the distance between the region of interest and the edge of the image by movement speed of the region of interest in the image is less than a predetermined threshold value.

### (Supplementary Note 8)

The image processing apparatus according to any one of Supplementary Notes 1 to 7, which is further equipped with
a marker detection unit that detects a marker established on a delivery wire of an embolic coil that approaches a region of interest configured in a portion of a microcatheter that guides the delivery wire, wherein
the tracking unit further tracks the detected marker, and
the notification unit notifies the user when the embolic coil may be detached from the delivery wire, as triggered by the superimposition of the marker and the region of interest.

### (Supplementary Note 9)

The image processing apparatus according to Supplementary Note 8, wherein
the notification unit notifies the user when the marker has passed the region of interest.

### (Supplementary Note 10)

The image processing apparatus according to Supplementary Note 8 or 9, wherein
the notification unit displays on a display device the distance that the marker should move before detaching the embolic coil from the delivery wire.

### (Supplementary Note 11)

The image processing apparatus according to any one of Supplementary Notes 1 to 10, wherein
when a feature value indicating the shape of the device included in the region of interest satisfies a predetermined condition, the notification unit notifies the user of the image processing apparatus of this fact.

### (Supplementary Note 12)

The image processing apparatus according to Supplementary Note 11, wherein the feature value is a curvature, and
the notification unit notifies the user of the device included in the region of interest on the condition that the curvature of the device exceeds a predetermined threshold curvature or that the curvature is changing but the tip is not moving.

### (Supplementary Note 13)

The image processing apparatus according to Supplementary Note 11 or 12, wherein
the notification unit notifies the user on the condition that the length of the device in the image or region of interest minus the length of the centerline of the blood vessel in the image or region of interest exceeds a predetermined threshold length.

### (Supplementary Note 14)

The image processing apparatus according to any one of Supplementary Notes 1 to 13, wherein the notification unit notifies the user by coloring the region of interest with a different color from the image, changing the font, size, or color of the characters displayed, changing the color of the entire screen or part of the screen of the display device, displaying a graphic on the entire screen, outside the frame, or in some other location of the display device, enlarging the region of interest, or changing the color or size of a mark attached to the region of interest.

### (Supplementary Note 15)

The image processing apparatus according to any one of Supplementary Notes 1 to 14, wherein
the notification unit notifies the user when a region including the tip portion of the catheter or the tip portion of the guide wire is set as the region of interest, on the condition that the region of interest has moved, or the region of interest exceeds a specific range specified on the image.

### (Supplementary Note 16)

The image processing apparatus according to any one of Supplementary Notes 1 to 15, further comprising an image storage unit for storing an image or a video obtained from an image acquisition unit over time.

### (Supplementary Note 17)

The image processing apparatus according to Supplementary Note 16, further comprising an image extraction unit that extracts video images from the image storage unit for a certain period of time before and after the notification is issued by the notification unit, or at any time point or any period of time specified by the user.

### (Supplementary Note 18)

The image processing apparatus according to Supplementary Note 17, wherein the image extraction period is automatically determined based on at least one of the movement distance, the movement speed, and the acceleration of the region of interest when the notification occurs.

### (Supplementary Note 19)

The image processing apparatus according to Supplementary Note 17 or 18, wherein the extracted video is displayed on a display device.

### (Supplementary Note 20)

The image processing apparatus according to Supplementary Note 19, wherein the extracted video is automatically and repeatedly displayed a predetermined number of times.

### (Supplementary Note 21)

The image processing apparatus according to Supplementary Note 20, wherein the extracted video is displayed based on any arbitrary operation including playback, stop, fast forward, rewind, frame advance, slow playback, and double-speed playback.

### (Supplementary Note 22)

The image processing apparatus according to any one of Supplementary Notes 17 to 21, wherein the time elapsed from the time when the notification occurred, a comparison of the position of the region of interest at the time when the notification occurred and after any arbitrary elapsed time, or the trajectory of the region of interest obtained by the tracking unit is superimposed on the extracted image and further displayed.

### (Supplementary Note 23)

The image processing apparatus according to any one of Supplementary Notes 17 to 22, wherein the extracted video is displayed by cutting out a portion of an area in the vicinity of the region of interest.

### (Supplementary Note 24)

The image processing apparatus according to any one of Supplementary note 17 to 23, wherein the extracted video is displayed at a position that does not interfere with the display of the region of interest.

### (Supplementary Note 25)

The image processing apparatus according to any one of Supplementary Notes 17 to 24, wherein the extracted video is enlarged and displayed.

### (Supplementary Note 26)

The image processing apparatus according to any one of Supplementary Notes 17 to 25, wherein the extracted video is displayed at the same time as the occurrence of the notification or after a predetermined time has elapsed from the occurrence of the notification.

### (Supplementary Note 27)

The image processing apparatus according to any one of Supplementary Notes 17 to 26, wherein images taken from multiple directions are simultaneously displayed.

### (Supplementary Note 28)

The image processing apparatus according to any one of Supplementary Notes 2 to 27, which is further equipped with a status estimation unit that estimates the current position and/or speed of the tip portion of the catheter or the tip portion of the guide wire that has disappeared from the image based on the position, speed and/or acceleration of the tip portion of the catheter or the tip portion of the guide wire immediately before the region of interest disappears from the image.

### (Supplementary Note 29)

The image processing apparatus according to Supplementary Note 28, wherein a warning is issued to the user when the current position and/or speed of the region of interest estimated by the status estimation unit exceeds a predetermined threshold value.

### (Supplementary Note 30)

The image processing apparatus according to any one of Supplementary Notes 1 to 29, wherein the display device displaying the image displays two images on two screens of different sizes.

### (Supplementary Note 31)

The image processing apparatus according to Supplementary Note 30, wherein the display device calls attention to the user by making the frame portion of one of the two screens glow, changing the color, or highlighting the screen.

### (Supplementary Note 32)

The image processing apparatus according to any one of Supplementary Notes 1 to 31, wherein the display device displaying the image displays a product list of devices for endovascular examination or treatment.

### (Supplementary Note 33)

The image processing apparatus according to Supplementary Note 32, wherein the display device displays a list of products narrowed down by size or inventory.

### (Supplementary Note 34)

The image processing apparatus according to Supplementary Note 32 or 33, wherein the display device displays a list of recommended products based on a result of image analysis, facility information, or user preference information.

### (Supplementary Note 35)

The image processing apparatus according to any one of Supplementary Notes 1 to 34, which automatically or based on the user selection creates a surgical record that includes information of the device used, information of the images acquired, and a result of the image analysis.

### (Supplementary Note 36)

The image processing apparatus according to any one of Supplementary Notes 1 to 35, wherein the notification unit displays on a display device a numerical value, color, bar or heat map according to the probability that at least one of the regions of interest satisfies a condition defined for each of the regions of interest, or a numerical value, color, bar or heat map based on any transformation applied to the probability distribution.

### (Supplementary Note 37)

The image processing apparatus according to any one of Supplementary Notes 1 to 35, wherein the notification unit colors the region of interest with a color or heat map according to the probability that at least one of the regions of interest satisfies a condition defined for each of the regions of interest, or with a color or heat map based on an arbitrarily transformed value of the probability distribution, and displays it on the display device displaying the image, or alternatively, replaces the probability that satisfies the condition with a numerical value or color and displays it on the display device displaying the image.

### (Supplementary Note 38)

The image processing apparatus according to any one of Supplementary Notes 1 to 37, wherein the notification unit notifies the user when a region including the tip portion of the catheter or the tip portion of the guide wire is set as the region of interest, on the condition that the region of interest has moved, or the region of interest exceeds a specific range specified on the image.

### (Supplementary Note 39)

The image processing apparatus according to Supplementary Note 38, wherein the boundary line of the specific range is represented by a straight line, a curve, a circle, a rectangle, or any other polygon.

### (Supplementary Note 40)

The image processing apparatus according to Supplementary Note 38 or 39, wherein the specific range is superimposed and displayed on an X-ray image.

### (Supplementary Note 41)

The image processing according to any one of Supplementary Notes 38 to 40, wherein the notification unit displays the distance between the region of interest and the edge portion of the specific range on a display device displaying the image.

### (Supplementary Note 42)

The image processing apparatus according to Supplementary Note 41, wherein the notification unit changes the display mode of the distance in the display device according to the length of the distance between the region of interest and the edge portion of the specific range.

### (Supplementary Note 43)

The image processing apparatus according to any one of Supplementary Notes 38 to 42, wherein the notification unit notifies the user on the condition that the value obtained by dividing the distance between the region of interest and the edge of the specific range by the movement speed of the region of interest in the image is less than a predetermined threshold value.

### (Supplementary Note 44)

The image processing apparatus according to any one of Supplementary Notes 3 to 43, wherein the distance is determined by either a straight line distance or a distance along a blood vessel.

### (Supplementary Note 45)

The image processing apparatus according to any one of Supplementary Notes 1 to 44, comprising a storage unit that acquires and stores the position and/or shape of a device for examination or treatment in a blood vessel at any point in time, wherein the stored device position and/or shape are superimposed on images since the acquisition.

### (Supplementary Note 46)

The image processing apparatus according to any one of Supplementary Notes 1 to 42, further comprising a lesion recognition unit that recognizes a lesion selected from the group consisting of cerebral aneurysm, stenosis, occlusion, thrombus formation, vascular perforation, spillage of the contrast medium, shunt disease, nutrient vessel and tumor thickening of tumor vessels, venous thrombosis, avascular area of the capillary phase, vascular occlusion, and collateral circulation in the image.

### (Supplementary Note 47)

The image processing apparatus according to any one of Supplementary Notes 1 to 43, further comprising an image recognition unit that compares the angiographic image in the image with a previously acquired and stored angiographic image and notifies the change.

### (Supplementary Note 48)

An image processing method, in which a processor of an image processing apparatus performs
a step of acquiring an image that includes at least a device for examination or treatment in a blood vessel as a subject;
a step of acquiring one or more regions that include at least a portion of the device in the image as a region of interest;
a step of tracking each of the regions of interest in the image; and
a step of notifying a user of the image processing apparatus when at least one of the regions of interest satisfies a condition defined for each of the regions of interest.

### (Supplementary Note 49)

A program that realizes on a computer,
a function of acquiring an image that includes at least a device for examination or treatment in a blood vessel as a subject,
a function of acquiring one or more regions that include at least a portion of the device in the X-ray image as a region of interest, and
a function of tracking each of the regions of interest in the X-ray image, and
a function of notifying a user of the computer when at least one of the regions of interest satisfies a condition defined for each of the regions of interest.

### (Supplementary Note 50)

An image processing system, which is equipped with
the image processing apparatus according to any one of Supplementary Notes 1 to 44 and
an imaging apparatus that captures an image of a person in a state in which a device for examination or treatment in a blood vessel is inserted and transmits the image to the image processing apparatus.

### (Supplementary Note 51)

A cerebrovascular catheterization surgery support system, which is a system equipped with
an image processing apparatus and
an image capture device that captures and transmits to the image processing apparatus X-ray images of a patient with one or more devices inserted into a blood vessel,
   wherein,
the image processing apparatus is equipped with
an image acquisition unit that acquires X-ray images over time of a fixed region that includes at least an area of interest for achieving a surgical objective and a device inserted into a blood vessel,
a region of interest acquisition unit for acquiring one or more regions in the image that include at least a portion of the device as a region of interest,
a tracking unit that tracks each of the regions of interest in the image, and
a notification unit that notifies a user of the image processing apparatus when at least one of the regions of interest meets a condition defined for each of the regions of interest,
   and wherein,
one or more of the devices are a catheter, guide wire, stent and/or balloon, and
a region including the tip portion of the catheter or the tip portion of the guide wire, both ends of the stent, and both ends of the balloon is set as the region of interest, and the user is notified on the condition that the region of interest disappears from the image, or that the distance between the region of interest and the edge of the image is less than a predetermined threshold distance.

### (Supplementary Note 52)

A cerebral aneurysm coil embolization assistance system, which is a system equipped with
an image processing apparatus and
an image capture device that captures and transmits to the image processing apparatus X-ray images of a patient in a state with a guiding catheter and a delivery wire for an embolic coil inserted into a blood vessel,
   wherein,
the image processing apparatus is equipped with
an image acquisition unit that acquires X-ray images over time of a fixed area that includes at least the aneurysm in the patient's blood vessel, a catheter inserted into the blood vessel, and a delivery wire for an embolization coil,
a region of interest acquisition unit that acquires one or more areas that include at least a portion of the guiding catheter in the image as a region of interest,
a marker detection unit that detects a marker provided on the delivery wire, which is a marker that approaches one or more regions of interest set in a portion of the catheter guiding the delivery wire,
a tracking unit that tracks each of the regions of interest and the markers in the image, and
a notification unit that notifies the user of the timing when the embolic coil may be detached from the delivery wire, triggered by the superimposition of the marker and the region of interest.

### (Supplementary Note 53)

The system according to Supplementary Note 51 or 52, wherein
the notification unit displays the distance between the region of interest and the edge portion of the image or the distance between the marker and the region of interest on a display device that displays the image, and wherein
the notification unit can change the manner in which the distance is displayed on the display device according to the length of the distance, wherein the change in the display mode includes changing the font, size, or color of the characters displayed according to the length of the distance, changing the color of the entire screen or a portion of the screen of the display device according to the length of the distance, displaying a graphic on the entire screen or outside the frame of the display device or in a part of the screen, enlarging and displaying the region of interest according to the length of the distance, or changing the color or size of the mark attached to the region of interest according to the length of the distance.

### (Supplementary Note 54)

The system according to any one of Supplementary Notes 51 to 53, wherein the notification unit can sound a notification tone or transmit vibration according to the length of the distance.

### (Supplementary Note 55)

The system according to any one of Supplementary Notes 51 to 54, wherein the distance is determined by either a straight line distance or a distance along a blood vessel.

### (Supplementary Note 56)

A cerebral aneurysm coil embolization assistance system, which is a system equipped with
an image processing apparatus and
an image capture device that captures and transmits to the image processing apparatus X-ray images of a patient in a state with a guiding catheter and a delivery wire for an embolic coil inserted into a blood vessel,
   wherein,
the image processing apparatus is equipped with
a positional relationship storage unit that stores the positional relationship between the tip of the catheter and a 2nd marker,
a positional storage unit that stores the distance a between the neckline of the aneurysm and a 1st marker and the position of the 2nd marker at that time tl,
a distance estimation unit that calculates the distance moved b from the position of the 2nd marker at time t2 and estimates the distance a-b from the aneurysm neckline to the tip of the catheter, and
a notification unit that notifies the user of the estimated distance.

### (Supplementary Note 57)

The system according to Supplementary Note 56, wherein the estimated distance is expressed by a probability distribution.

### (Supplementary Note 58)

The system according to Supplementary Note 56 or 57, wherein the estimated position of the catheter tip is colored and displayed based on a probability distribution.

### (Supplementary Note 59)

An endovascular surgical support system, comprising a storage unit that stores a product list of devices for examination or treatment in a blood vessel, a recommendation unit that recommends products for use based on image analysis results, facility information, or user preference information, and a display unit that displays the recommended products.

### (Supplementary Note A1)

An image processing apparatus, comprising:
an image acquisition unit that can acquire an image including at least a device for examination or treatment in a blood vessel as a subject.

### (Supplementary Note A2)

The image processing apparatus according to Supplementary Note A1, further comprising:
an image acquisition unit for acquiring an image including at least a device for examination or treatment in a blood vessel as a subject,
a region of interest acquisition unit for acquiring one or more regions in the image that include at least a portion of the device as a region of interest, and
a notification unit that notifies the user of the image processing apparatus when at least one of the regions of interest satisfies a condition determined for each of the regions of interest,
   wherein,
the notification unit notifies the user on the condition that a device that newly appears in an image is newly detected as a region of interest.

### (Supplementary Note A3)

The image processing apparatus according to Supplementary Note A2, wherein the notification unit notifies the user on the condition that a device that newly appears at the edge of the image or within a specific region is newly detected as a region of interest.

### (Supplementary Note A4)

The image processing apparatus according to Supplementary Note A3, wherein the specific region is specified by a user.

### (Supplementary Note A5)

The image processing apparatus according to Supplementary Note A3, wherein the specific region is specified automatically.

### (Supplementary Note A6)

The image processing apparatus according to Supplementary Note A5, wherein the specific region is a region that the image processing apparatus determines automatically as a region that should checked.

### (Supplementary Note A7)

The image processing apparatus according to any one of Supplementary Notes A2 to A6, further comprising a tracking unit for tracking the region of interest in the image.

### (Supplementary Note A8)

The image processing apparatus according to any one of Supplementary Notes A2 to A7, wherein a region including at least a portion of a device for examination or treatment in a blood vessel, selected from a group consisting of a guiding catheter, a guide wire, an intermediate catheter, a microcatheter, a suction catheter for thrombectomy, a marker, a coil, a stent, a filter, an embolic material, an aneurysm embolization device, and a balloon, is set as the region of interest.

### (Supplementary Note A9)

The image processing apparatus according to any one of Supplementary Notes A2 to A8, wherein the region including at least a portion of the device includes the tip of a device for examination or treatment in a blood vessel.

### (Supplementary Note A10)

The image processing apparatus according to Supplementary Note A1, further comprising
an image acquisition unit for acquiring an image including at least a device for examination or treatment in a blood vessel as a subject,
a region of interest acquisition unit for acquiring one or more regions in the image that include at least a portion of the device as a region of interest,
a tracking unit that tracks each of the regions of interest in the image, and
a notification unit that notifies the user of the image processing apparatus when at least one of the regions of interest satisfies a condition determined for each of the regions of interest,
   wherein,
the notification unit notifies the user on the condition that the region of interest passes a specific boundary line specified on an image.

### (Supplementary Note A11)

The image processing apparatus according to Supplementary Note A10, wherein the specific boundary line is specified by a user.

### (Supplementary Note A12)

The image processing apparatus according to Supplementary Note A10, wherein the specific boundary line is specified automatically.

### (Supplementary Note A13)

The image processing apparatus according to any one of Supplementary Notes A9 to A12, wherein the specific boundary line is represented by a straight line, a curve, a circle, a rectangle, or any other polygon.

### (Supplementary Note A14)

The image processing apparatus according to any one of Supplementary Notes A9 to A13, wherein the specific boundary line is used to detect entry of the device into a blood vessel where entry thereto is not desirable.

### (Supplementary Note A15)

The image processing apparatus according to any one of Supplementary Notes A9 to A14, wherein the notification unit notifies the user only when the condition is met for the first time, for each of one or more regions of interest.

### (Supplementary Note A16)

The image processing apparatus according to any one of Supplementary Notes A9 to A15, wherein the notification unit notifies the user only when the condition is met for the first time, for all of the one or more regions of interest.

### (Supplementary Note A17)

The image processing apparatus according to any one of Supplementary Notes A9 to A16, wherein the notification unit notifies the user only when the condition is met for one or more specific regions of interest specified by the user.

### (Supplementary Note A18)

The image processing apparatus according to any one of Supplementary Notes A9 to A16, wherein the notification unit notifies the user only when the condition is met for one or more specific regions of interest specified automatically.

### (Supplementary Note A19)

The image processing apparatus according to any one of Supplementary Notes A9 to A18, wherein the specific boundary line is superimposed and displayed on an X-ray image.

### (Supplementary Note A20)

The image processing apparatus according to any one of Supplementary Notes A9 to A19, wherein the specific boundary line is redrawn in accordance with changes in the extent or magnification of the X-ray image.

### (Supplementary Note A21)

The image processing apparatus according to any one of Supplementary Notes A9 to A20, wherein the notification is issued by means of generation of a warning sound or by changing a display mode of a boundary line.

### (Supplementary Note A22)

The image processing apparatus according to any one of Supplementary Notes A9 to A21, wherein different methods of notification are used in accordance with the direction in which the region of interest passes a specific boundary line specified on an image.

### (Supplementary Note A23)

The image processing apparatus according to any one of Supplementary Notes A9 to A22, wherein different methods of notification are used in accordance with the speed at which the region of interest crosses the specific boundary line.

### (Supplementary Note A24)

The image processing apparatus according to any one of Supplementary Notes A9 to A23, wherein the notification unit displays the distance between the region of interest and the specific boundary line on a display device for displaying the image.

### (Supplementary Note A25)

The image processing apparatus according to any one of Supplementary Notes A9 to A24, wherein the notification unit changes the display mode of the distance in the display device according to the length of the distance between the region of interest and the specific boundary line.

### (Supplementary Note A26)

The image processing apparatus according to any one of Supplementary Notes A9 to A25, wherein the notification unit notifies the user on the condition that the value obtained by dividing the distance between the region of interest and the specific boundary line by the movement speed of the region of interest in the image is less than a predetermined threshold value.

### (Supplementary Note A27)

The image processing apparatus according to Supplementary Note A1, further comprising:
an image acquisition unit for acquiring an image including at least a device for examination or treatment in a blood vessel as a subject,
a region of interest acquisition unit for acquiring one or more regions in the image that include at least a portion of the device as a region of interest,
a tracking unit that tracks each of the regions of interest in the image, and
a notification unit that notifies the user of the image processing apparatus when at least one of the regions of interest satisfies a condition determined for each of the regions of interest,
   wherein,
a region of interest included in a region specified by a user on the image is acquired as the region of interest.

### (Supplementary Note A28)

The image processing apparatus according to Supplementary Note A27, wherein a region specified by a user on the image is represented by a rectangle, a circle, or any closed curve.

### (Supplementary Note A29)

The image processing apparatus according to Supplementary Note A27 or A28, wherein candidates for regions that can be specified by a user are automatically displayed.

### (Supplementary Note A30)

The image processing apparatus according to any one of Supplementary Notes A27 to A29, wherein the image temporarily becomes a still image when a user specifies a region.

### (Supplementary Note A31)

The image processing apparatus according to any one of Supplementary Notes A27 to A29, wherein the image playback is temporarily slowed when a user specifies a region.

### (Supplementary Note A32)

The image processing apparatus according to any one of Supplementary Notes A27 to A31, wherein a portion of the image is displayed enlarged when a user specifies a region.

### (Supplementary Note A33)

The image processing apparatus according to any one of Supplementary Notes A27 to A32, wherein, if a region including multiple regions of interest is specified, only the region of interest that should be checked is selected automatically.

### (Supplementary Note A34)

The image processing apparatus according to Supplementary Note A33, wherein the region that should be checked is determined based on, for example, the tip of a guide wire being within a set range, the tip of a stent delivery wire being within a set range, the range in which a second marker of a catheter moves in order to discern the timing when a coil may be detached, a filter being within a set range, the tip of a catheter being within a set range, an embolic material being within a set range, a device being in a range remaining inside a large blood vessel, an aneurysm, or an important blood vessel.

### (Supplementary Note A35)

The image processing apparatus according to Supplementary Note A33 or A34, wherein a region of interest that should be checked is determined in accordance with the situation of a surgery.

### (Supplementary Note A36)

The image processing apparatus according to any one of Supplementary Notes A27 to A35, wherein the specified region is automatically adjusted when the image is changed.

### (Supplementary Note A37)

The image processing apparatus according to Supplementary Note A1, further comprising
an image acquisition unit for acquiring an image including at least a device for examination or treatment in a blood vessel as a subject, and
a situation recognition unit for recognizing a specific surgery situation based on the image.

### (Supplementary Note A38)

The image processing apparatus according to Supplementary Note A37, wherein the specific surgery situation is at least one situation selected from a group consisting of the content of a surgery, disease name, vascular leakage, vascular perforation, occurrence of thrombus, disappearance of peripheral blood vessels, guiding a guide wire/a catheter/a balloon, aspiration by catheter, stent placement, balloon inflation, coil insertion, insertion of an aneurysm embolization device, timing when a coil may be detached, guiding/placing/retrieving a filter, injection of liquid embolic material, injection of contrast agent, continuation of a still image for a certain period of time or more, discrimination between a mask image and a live image, determination of imaging site/angle, switching of images, and occurrence of noise.

### (Supplementary Note A39)

The image processing apparatus according to Supplementary Note A37 or A38, further comprising
a region of interest acquisition unit for acquiring one or more regions in the image that include at least a portion of the device as a region of interest, and
a notification unit that notifies the user of the image processing apparatus when at least one of the regions of interest satisfies a condition determined for each of the regions of interest.

### (Supplementary Note A40)

The image processing apparatus according to Supplementary Note A38, wherein acquisition of the region of interest is stopped if occurrence of noise is recognized.

### (Supplementary Note A41)

The image processing apparatus according to Supplementary Note A38, wherein a recommendation to reduce exposure is notified if continuation of a still image of a certain period of time or more is recognized.

### (Supplementary Note A42)

The image processing apparatus according to any one of Supplementary Notes A37 to A41, further comprising a recording unit for recording the specific surgery situation that is recognized.

### (Supplementary Note A43)

The image processing apparatus according to any one of Supplementary Notes A37 to A42, wherein the specific surgery situation is recognized by means of pattern matching, image recognition, time-series image recognition, time-series differencing, or object detection algorithms.

### (Supplementary Note A44)

The image processing apparatus according to any one of Supplementary Notes A37 to A43, wherein the device to be recognized is specified in accordance with the specific surgery situation that has been recognized.

### (Supplementary Note A45)

The image processing apparatus according to Supplementary Note A1, further comprising:
an image acquisition unit for acquiring an image including at least a device for examination or treatment in a blood vessel as a subject,
a region of interest acquisition unit for acquiring one or more regions in the image that include at least a portion of the device as a region of interest, and
a recording unit for recording, when at least one of the regions of interest satisfies a condition defined for each of the regions of interest, the situation of a surgery corresponding to that condition.

### (Supplementary Note A46)

The image processing apparatus according to Supplementary Note A45, wherein the conditions include at least one procedure selected from a group consisting of puncture, arrival of a guiding catheter to the treatment site, balloon guidance, balloon inflation, stent deployment, catheter guidance, insertion of the first coil, insertion of the second and subsequent coils, location of the coil within the aneurysm, deviation of the coil to the parent vessel, catheter removal, coil removal, and end of procedure.

### (Supplementary Note A47)

The image processing apparatus according to Supplementary Note A45 or A46, wherein the surgery situation to be recorded includes still images, video, and/or text information explaining the surgery situation.

### (Supplementary Note A48)

The image processing apparatus according to Supplementary Note A47, wherein the video is a video of a certain period of time before and after the point in time at which the condition is met.

### (Supplementary Note A49)

The image processing apparatus according to any one of Supplementary Notes A45 to A48, wherein the recorded information is modifiable by a user.

### (Supplementary Note A50)

The image processing apparatus according to any one of Supplementary Notes A45 to A49, further comprising a report creation unit for creating reports based on the recorded information.

### (Supplementary Note A51)

The image processing apparatus according to Supplementary Note A50, wherein the report creation unit creates a summary of a surgery based on recorded still images, video, and/or text information explaining the surgery situation.

### (Supplementary Note A52)

The image processing apparatus according to any one of Supplementary Notes A45 to A51, further comprising a notification unit for notifying the user when a specific surgery situation has been recorded.

### (Supplementary Note A53)

The image processing apparatus according to any one of Supplementary Notes A45 to A52, further comprising a notification unit for notifying the user in accordance with the time that has elapsed after a specific surgery situation was recorded.

### (Supplementary Note A54)

The image processing apparatus according to Supplementary Note A53, wherein the time that has elapsed after a specific surgery situation was recorded is determined based on balloon inflation time, guide wire travel time, speed, and acceleration, or coil travel time.

### (Supplementary Note A55)

The image processing apparatus according to any one of Supplementary Notes A45 to A54, further comprising a notification unit for notifying the user in accordance with the number of times that a specific surgery situation has been recorded.

### (Supplementary Note A56)

The image processing apparatus according to Supplementary Note A55, wherein the specific surgery situation is coil insertion.

### (Supplementary Note A57)

The image processing apparatus according to any one of Supplementary Notes A45 to A56, wherein the images are acquired from video recorded in real time or from previously recorded video.

### (Supplementary Note A58)

The image processing apparatus according to Supplementary Note A1, further comprising
an image acquisition unit for acquiring an image including at least a device for examination or treatment in a blood vessel as a subject,
a region of interest acquisition unit for acquiring one or more regions in the image that include at least a portion of the device as a region of interest,
a tracking unit that tracks each of the regions of interest in the image, and
a notification unit that notifies the user of the image processing apparatus when at least one of the regions of interest satisfies a condition determined for each of the regions of interest,
   wherein,
the image processing apparatus further comprises a display unit that causes a display device connected to the image processing apparatus to display the image including the region of interest, and a mark corresponding only to the vertical coordinate of the region of interest next to the image.

### (Supplementary Note A59)

The image processing apparatus according to Supplementary Note A58, wherein a trajectory of a mark is displayed for a certain period of time.

### (Supplementary Note A60)

The image processing apparatus according to Supplementary Note A60 or A61, wherein the display mode of the mark is different for each region of interest.

### (Supplementary Note A61)

The image processing apparatus according to Supplementary Note A60, wherein the display mode is color, shape, pattern, symbol, text, caption, or animation.

### (Supplementary Note A62)

The image processing apparatus according to any one of Supplementary Notes A58 to A61, wherein a region of interest that should be checked is automatically recognized and highlighted.

### (Supplementary Note A63)

The image processing apparatus according to Supplementary Note A62, wherein a region of interest that should be checked is determined in accordance with the situation of a surgery.

### (Supplementary Note A64)

The image processing apparatus according to any one of Supplementary Notes A58 to A63, wherein only regions of interest specified by the user are displayed.

### (Supplementary Note A65)

The image processing apparatus according to any one of Supplementary Notes A58 to A64, wherein only automatically selected regions of interest are displayed.

### (Supplementary Note A66)

The image processing apparatus according to any one of Supplementary Notes A58 to A65, wherein the user is notified if a mark exceeds a user-specified threshold value.

### (Supplementary Note A67)

The image processing apparatus according to Supplementary Note A1, further comprising
an image acquisition unit for acquiring an image including at least a device for examination or treatment in a blood vessel as a subject,
a region of interest acquisition unit for acquiring one or more regions in the image that include at least a portion of the device as a region of interest,
a tracking unit that tracks each of the regions of interest in the image, and
a notification unit that notifies the user of the image processing apparatus when at least one of the regions of interest satisfies a condition determined for each of the regions of interest,
   wherein,
the image processing apparatus further comprises a display unit for displaying, if a plurality of the regions of interest are tracked, each of the multiple regions of interest using a different display mode.

### (Supplementary Note A68)

The image processing apparatus according to Supplementary Note A67, wherein the display mode is color, shape, pattern, symbol, text, caption, or animation.

### (Supplementary Note A69)

The image processing apparatus according to Supplementary Note A67 or A68, wherein a region of interest that should be checked is automatically recognized and highlighted.

### (Supplementary Note A70)

The image processing apparatus according to any one of Supplementary Notes A67 to A69, wherein a region of interest that should be checked is determined in accordance with the situation of a surgery.

### (Supplementary Note A71)

The image processing apparatus according to any one of Supplementary Notes A67 to A70, wherein only regions of interest specified by the user are displayed.

### (Supplementary Note A72)

The image processing apparatus according to any one of Supplementary Notes A67 to A71, wherein only automatically selected regions of interest are displayed.

### (Supplementary Note B1)

An image processing apparatus, comprising:
an image acquisition unit that can acquire an image including at least a device for examination or treatment in a blood vessel as a subject.

### (Supplementary Note B2)

The image processing apparatus according to Supplementary Note B1, further comprising:
an image acquisition unit for acquiring an image for examination or treatment in a blood vessel,
a region of interest acquisition unit for acquiring, when a device for examination or treatment in a blood vessel is included in the image, one or more regions that include at least a portion of the device as a region of interest, and
a notification unit that notifies the user of the image processing apparatus when at least one of the regions of interest satisfies a condition determined for each of the regions of interest,
   wherein,
the notification unit notifies the user on the condition that a device or a part thereof that newly appears in an image or a specific region is newly detected as a region of interest.

### (Supplementary Note B3)

The image processing apparatus according to Supplementary Note B1, further comprising:
an image acquisition unit for acquiring an image for examination or treatment in a blood vessel,
a region of interest acquisition unit for acquiring, when a device for examination or treatment in a blood vessel is included in the image, one or more regions that include at least a portion of the device as a region of interest, and
an output unit that, when at least one of the regions of interest satisfies a condition determined for each of the regions of interest, outputs the information to a device connected to the image processing apparatus,
   wherein,
the output unit outputs to the device on the condition that a device or a part thereof that newly appears in an image or a specific region is newly detected as a region of interest.

### (Supplementary Note B4)

The image processing apparatus according to Supplementary Note B2 or B3, wherein the notification unit notifies the user on the condition that a device or a part thereof that newly appears at the edge of, or within a set distance from the edge of the image is newly detected as a region of interest.

### (Supplementary Note B5)

The image processing apparatus according to Supplementary Note B2 or B3, wherein the output unit outputs to the device on the condition that a device or a part thereof that newly appears at the edge or within a set distance from the edge of the image, or within a specific region, or a portion thereof, is newly detected as a region of interest.

### (Supplementary Note B6)

The image processing apparatus according to Supplementary Note B2 or B3, wherein, if a display device that displays the images has multiple screens, processing is performed for each screen.

### (Supplementary Note B7)

The image processing apparatus according to Supplementary Note B6, wherein the multiple screens display two or more images selected from a frontal live image, a lateral live image, a frontal mask image, and a lateral mask image.

### (Supplementary Note B8)

The image processing apparatus according to Supplementary Note B6, wherein information of multiple screens is used to improve processing accuracy.

### (Supplementary Note B9)

The image processing apparatus according to Supplementary Note B2 or B3, wherein the specific region is specified by a user.

### (Supplementary Note B10)

The image processing apparatus according to Supplementary Note B2 or B3, wherein the specific region is specified automatically.

### (Supplementary Note B11)

The image processing apparatus according to Supplementary Note B2 or B3, wherein the specific region is a region that the image processing apparatus determines automatically as a region that should be checked.

### (Supplementary Note B12)

The image processing apparatus according to Supplementary Note B2 or B3, further comprising a tracking unit for tracking the region of interest in the image.

### (Supplementary Note B13)

The image processing apparatus according to Supplementary Note B2 or B3, further comprising a blood vessel recognition unit for recognizing at least some of the blood vessels in the image.

### (Supplementary Note B14)

The image processing apparatus according to Supplementary Note B2 or B3, wherein a notification is issued or an output is performed if the region of interest enters a blood vessel having a diameter less than or equal to a threshold value, or a blood vessel that has been specified automatically or manually.

### (Supplementary Note B15)

The image processing apparatus according to Supplementary Note B2 or B3, wherein a region including at least a portion of a device for examination or treatment in a blood vessel, selected from a group consisting of a guiding catheter, a guide wire, an intermediate catheter, a microcatheter, a suction catheter for thrombectomy, a marker, a coil, a stent, a filter, an embolic material, an aneurysm embolization device, and a balloon, is set as the region of interest.

### (Supplementary Note B16)

The image processing apparatus according to Supplementary Note B2 or B3, wherein the region including at least a portion of the device includes the tip of a device for examination or treatment in a blood vessel.

### (Supplementary Note B17)

The image processing apparatus according to Supplementary Note B1, further comprising:
an image acquisition unit for acquiring an image including at least a device for examination or treatment in a blood vessel as a subject,
a region of interest acquisition unit for acquiring one or more regions in the image that include at least a portion of the device as a region of interest, and
a notification unit that notifies the user of the image processing apparatus when at least one of the regions of interest satisfies a condition determined for each of the regions of interest,
   wherein,
the notification unit notifies the user on the condition that the region of interest passes, or is predicted to pass, a specific boundary line specified on an image.

### (Supplementary Note B18)

The image processing apparatus according to Supplementary Note B1, further comprising:
an image acquisition unit for acquiring an image including at least a device for examination or treatment in a blood vessel as a subject,
a region of interest acquisition unit for acquiring one or more regions in the image that include at least a portion of the device as a region of interest, and
an output unit that, when at least one of the regions of interest satisfies a condition determined for each of the regions of interest, outputs the information to a device connected to the image processing apparatus,
   wherein,
the output unit notifies the user on the condition that the region of interest passes, or is predicted to pass, a specific boundary line specified on an image.

### (Supplementary Note B19)

The image processing apparatus according to Supplementary Note B17 or B18, further comprising a tracking unit for tracking the region of interest in the image.

### (Supplementary Note B20)

The image processing apparatus according to Supplementary Note B17 or B18, further comprising a blood vessel recognition unit for recognizing at least some of the blood vessels in the image.

### (Supplementary Note B21)

The image processing apparatus according to Supplementary Note B17 or B18, wherein a notification is issued or an output is performed if the region of interest enters a blood vessel having a diameter less than or equal to a threshold value, or a blood vessel that has been specified automatically or manually.

### (Supplementary Note B22)

The image processing apparatus according to Supplementary Note B 17 or B 18, wherein prediction of passage is performed based on distance between the region of interest and the boundary line, or on the distance/velocity of the region of interest.

### (Supplementary Note B23)

The image processing apparatus according to Supplementary Note B 17 or B 18, wherein the specific boundary line is specified by a user.

### (Supplementary Note B24)

The image processing apparatus according to Supplementary Note B 17 or B 18, wherein the specific boundary line is specified automatically.

### (Supplementary Note B25)

The image processing apparatus according to Supplementary Note B 17 or B 18, wherein the specific boundary line is automatically specified when the region of interest has not moved for a certain period of time.

### (Supplementary Note B26)

The image processing apparatus according to Supplementary Note B 17 or B 18, wherein the specific boundary line is automatically specified in accordance with the situation of the surgery, or, is automatically proposed, which can then be accepted or rejected by the user.

### (Supplementary Note B27)

The image processing apparatus according to Supplementary Note B 17 or B18, wherein the specific boundary line is automatically removed in accordance with the situation of the surgery, or, removal may be automatically proposed, which can then be accepted or rejected by the user.

### (Supplementary Note B28)

The image processing apparatus according to Supplementary Note B 17 or B18, wherein, if a display device that displays the images has multiple screens, the specific boundary line specified in one screen is automatically specified in a corresponding location on the other screens.

### (Supplementary Note B29)

The image processing apparatus according to Supplementary Note B 17 or B18, wherein, if a display device that displays the images has multiple screens, processing is performed for each screen.

### (Supplementary Note B30)

The image processing apparatus according to Supplementary Note B29, wherein the multiple screens display two or more images selected from a frontal live image, a lateral live image, a frontal mask image, and a lateral mask image.

### (Supplementary Note B31)

The image processing apparatus according to Supplementary Note B29, wherein information of multiple screens is used to improve processing accuracy.

### (Supplementary Note B32)

The image processing apparatus according to Supplementary Note B17 or B18, wherein the specific boundary line is represented by a straight line, a curve, a circle, a rectangle, or any other polygon.

### (Supplementary Note B33)

The image processing apparatus according to Supplementary Note B17 or B18, wherein the boundary line can be moved, deformed, enlarged, or shrunk by means of user operation.

### (Supplementary Note B34)

The image processing apparatus according to Supplementary Note B17 or B18, wherein the specific boundary line is used to detect entry of the device into a blood vessel where entry thereto is not desirable, a lesion site such as an aneurysm or stenosis, or to outside of the blood vessel.

### (Supplementary Note B35)

The image processing apparatus according to Supplementary Note B17 or B18, wherein the notification unit or output unit notifies or outputs only when the condition is met for the first time, for each of one or more regions of interest.

### (Supplementary Note B36)

The image processing apparatus according to Supplementary Note B17 or B18, wherein the notification unit or output unit notifies or outputs only when the condition is met for the first time after a particular point in time, for each of one or more regions of interest.

### (Supplementary Note B37)

The image processing apparatus according to Supplementary Note B17 or B18, wherein the notification unit or output unit notifies or outputs only when the condition is met for the first time, for all of one or more regions of interest.

### (Supplementary Note B38)

The image processing apparatus according to Supplementary Note B17 or B18, wherein the notification unit or output unit notifies or outputs only when the condition is met for the first time after a particular point in time, for all of one or more regions of interest.

### (Supplementary Note B39)

The image processing apparatus according to Supplementary Note B17 or B18, wherein the notification unit or output unit notifies or outputs only when the condition is met for one or more specific regions of interest specified by the user.

### (Supplementary Note B40)

The image processing apparatus according to Supplementary Note B17 or B18, wherein the notification unit or output unit notifies or outputs only when the condition is met for one or more regions of interest specified automatically.

### (Supplementary Note B41)

The image processing apparatus according to Supplementary Note B17 or B18, wherein the specific boundary line is superimposed and displayed on an X-ray image.

### (Supplementary Note B42)

The image processing apparatus according to Supplementary Note B17 or B18, wherein the specific boundary line is redrawn in accordance with movements or changes in the extent or magnification of the X-ray image.

### (Supplementary Note B43)

The image processing apparatus according to Supplementary Note B17 or B18, wherein the specific boundary line is redrawn after interruption and reacquisition of the X-ray image.

### (Supplementary Note B44)

The image processing apparatus according to Supplementary Note B17, wherein the notification is issued by means of generation of a warning sound or by changing a display mode of a boundary line.

### (Supplementary Note B45)

The image processing apparatus according to Supplementary Note B17, wherein the notification conveys, by means of voice, some or all of: which screen, which device, and what type of event.

### (Supplementary Note B46)

The image processing apparatus according to Supplementary Note B17, wherein different methods of notification are used in accordance with the direction in which the region of interest passes a specific boundary line specified on an image.

### (Supplementary Note B47)

The image processing apparatus according to Supplementary Note B17 or B18, wherein the direction in which the region of interest passes a specific boundary line specified on an image is automatically recognized in accordance with the situation of the surgery, or, is automatically proposed, which can then be accepted or rejected by the user.

### (Supplementary Note B48)

The image processing apparatus according to Supplementary Note B17, wherein different methods of notification are used in accordance with the speed at which the region of interest crosses the specific boundary line.

### (Supplementary Note B49)

The image processing apparatus according to Supplementary Note B17, wherein the notification unit displays the distance between the region of interest and the specific boundary line on a display device for displaying the image.

### (Supplementary Note B50)

The image processing apparatus according to Supplementary Note B17, wherein the notification unit changes the display mode of the distance in the display device according to the length of the distance between the region of interest and the specific boundary line.

### (Supplementary Note B51)

The image processing apparatus according to Supplementary Note B17 or B18, wherein the notification unit or output unit notifies or outputs on the condition that the value obtained by dividing the distance between the region of interest and the specific boundary line by the movement speed of the region of interest in the image is less than a predetermined threshold value.

### (Supplementary Note B52)

The image processing apparatus according to Supplementary Note B1, further comprising:
an image acquisition unit for acquiring an image including at least a device for examination or treatment in a blood vessel as a subject,
a region of interest acquisition unit for acquiring one or more regions in the image that include at least a portion of the device as a region of interest, and
a notification unit that notifies the user of the image processing apparatus when at least one of the regions of interest satisfies a condition determined for each of the regions of interest,
   wherein,
a region of interest included in a region specified by a user on the image is acquired as the region of interest.

### (Supplementary Note B53)

The image processing apparatus according to Supplementary Note B1, further comprising:
an image acquisition unit for acquiring an image including at least a device for examination or treatment in a blood vessel as a subject,
a region of interest acquisition unit for acquiring one or more regions in the image that include at least a portion of the device as a region of interest, and
an output unit that, when at least one of the regions of interest satisfies a condition determined for each of the regions of interest, outputs the information to a device connected to the image processing apparatus,
   wherein,
a region of interest included in a region specified by a user on the image is acquired as the region of interest.

### (Supplementary Note B54)

The image processing apparatus according to Supplementary Note B52 or B53, further comprising a tracking unit for tracking the region of interest in the image.

### (Supplementary Note B55)

The image processing apparatus according to Supplementary Note B52 or B53, further comprising a blood vessel recognition unit for recognizing at least some of the blood vessels in the image.

### (Supplementary Note B56)

The image processing apparatus according to Supplementary Note B52 or B53, wherein a notification is issued or an output is performed if the region of interest enters a blood vessel having a diameter less than or equal to a threshold value, or a blood vessel that has been specified automatically or manually.

### (Supplementary Note B57)

The image processing apparatus according to Supplementary Note B52 or B53, wherein a region specified by a user on the image is represented by a rectangle, a circle, or any closed curve.

### (Supplementary Note B58)

The image processing apparatus according to Supplementary Note B52 or B53, wherein candidates for regions that can be specified by a user are automatically displayed.

### (Supplementary Note B59)

The image processing apparatus according to Supplementary Note B52 or B53, wherein candidates for regions that can be specified by a user are automatically displayed in accordance with the situation of a surgery.

### (Supplementary Note B60)

The image processing apparatus according to Supplementary Note B52 or B53, wherein the image temporarily becomes a still image when a user specifies a region.

### (Supplementary Note B61)

The image processing apparatus according to Supplementary Note B52 or B53, wherein the image playback is temporarily slowed when a user specifies a region.

### (Supplementary Note B62)

The image processing apparatus according to Supplementary Note B52 or B53, wherein a portion of the image is displayed enlarged when a user specifies a region.

### (Supplementary Note B63)

The image processing apparatus according to Supplementary Note B52 or B53, wherein, if a display device that displays the images has multiple screens, processing is performed for each screen.

### (Supplementary Note B64)

The image processing apparatus according to Supplementary Note B63, wherein the multiple screens display two or more images selected from a frontal live image, a lateral live image, a frontal mask image, and a lateral mask image.

### (Supplementary Note B65)

The image processing apparatus according to Supplementary Note B63, wherein information of multiple screens is used to improve processing accuracy.

### (Supplementary Note B66)

The image processing apparatus according to Supplementary Note B52 or B53, wherein, if a region including multiple regions of interest is specified, only the region of interest that should be checked is selected automatically.

### (Supplementary Note B67)

The image processing apparatus according to Supplementary Note B66, wherein the region of interest that should be checked is determined based on importance of multiple devices, position of the region of interest in the screen, and the surgery scene.

### (Supplementary Note B68)

The image processing apparatus according to Supplementary Note B66, wherein a region of interest that should be checked is determined in accordance with the situation of a surgery.

### (Supplementary Note B69)

The image processing apparatus according to Supplementary Note B52 or B53, wherein the specified region is automatically adjusted when the image is changed.

### (Supplementary Note B70)

The image processing apparatus according to Supplementary Note B 1, further comprising:
an image acquisition unit for acquiring an image including at least a device for examination or treatment in a blood vessel as a subject, and
a situation recognition unit for recognizing a specific surgery situation based on the image.

### (Supplementary Note B71)

The image processing apparatus according to Supplementary Note B70, wherein the specific surgery situation is at least one situation selected from a group consisting of the content of a surgery, disease name, vascular leakage, vascular perforation, occurrence of thrombus, disappearance of peripheral blood vessels, guiding a guide wire/a catheter/a balloon, aspiration by catheter, stent placement, balloon inflation, coil insertion, insertion of an aneurysm embolization device, timing when a coil may be detached, guiding/placing/retrieving a filter, injection of liquid embolic material, injection of contrast agent, continuation of a still image for a certain period of time or more, discrimination between a mask image and a live image, determination of imaging site/angle, switching of images, and occurrence of noise.

### (Supplementary Note B72)

The image processing apparatus according to Supplementary Note B70 or B71, further comprising:
a region of interest acquisition unit for acquiring one or more regions in the image that include at least a portion of the device as a region of interest, and
a notification unit that notifies the user of the image processing apparatus when at least one of the regions of interest satisfies a condition determined for each of the regions of interest.

### (Supplementary Note B73)

The image processing apparatus according to Supplementary Note B70, wherein acquisition of the region of interest is stopped if occurrence of noise is recognized.

### (Supplementary Note B74)

The image processing apparatus according to Supplementary Note B70, wherein, when injection of a contrast medium is recognized, when a 3D image is acquired, or when a cone beam CT (CBCT) is acquired, acquisition of the region of interest is stopped, or, is acquired but not notified.

### (Supplementary Note B75)

The image processing apparatus according to Supplementary Note B70, wherein a recommendation to reduce exposure is notified if continuation of a still image of a certain period of time or more is recognized.

### (Supplementary Note B76)

The image processing apparatus according to Supplementary Note B70 or B71, further comprising a recording unit for recording the specific surgery situation that is recognized.

### (Supplementary Note B77)

The image processing apparatus according to Supplementary Note B70 or B71, wherein the specific surgery situation is recognized by means of pattern matching, image recognition, time-series image recognition, time-series differencing, or object detection algorithms.

### (Supplementary Note B78)

The image processing apparatus according to Supplementary Note B70 or B71, wherein the device to be recognized is specified in accordance with the specific surgery situation that has been recognized.

### (Supplementary Note B79)

The image processing apparatus according to Supplementary Note B70 or B71, wherein a specific boundary line for notifying a user on the condition that a region of interest passes the specific boundary line specified on the image is automatically specified or proposed around the region of interest, in accordance with the specific surgery situation that has been recognized.

### (Supplementary Note B80)

The image processing apparatus according to Supplementary Note B70 or B71, wherein, if a display device that displays the images has multiple screens, processing is performed for each screen.

### (Supplementary Note B81)

The image processing apparatus according to Supplementary Note B80, wherein the multiple screens display two or more images selected from a frontal live image, a lateral live image, a frontal mask image, and a lateral mask image.

### (Supplementary Note B82)

The image processing apparatus according to Supplementary Note B80, wherein information of multiple screens is used to improve processing accuracy.

### (Supplementary Note B83)

The image processing apparatus according to Supplementary Note B 1, further comprising:
an image acquisition unit for acquiring an image including at least a device for examination or treatment in a blood vessel as a subject,
a region of interest acquisition unit for acquiring one or more regions in the image that include at least a portion of the device as a region of interest, and
a recording unit for recording, when at least one of the regions of interest satisfies a condition defined for each of the regions of interest, the situation of a surgery corresponding to that condition.

### (Supplementary Note B84)

The image processing apparatus according to Supplementary Note B83, wherein the conditions include at least one procedure selected from a group consisting of puncture, arrival of a guiding catheter to the treatment site, balloon guidance, balloon inflation, stent deployment, catheter guidance, insertion of the first coil, insertion of the second and subsequent coils, location of the coil within the aneurysm, deviation of the coil to the parent vessel, catheter removal, coil removal, and end of procedure.

### (Supplementary Note B85)

The image processing apparatus according to Supplementary Note B83 or B84, wherein the surgery situation to be recorded includes still images, video, and/or text information explaining the surgery situation.

### (Supplementary Note B86)

The image processing apparatus according to Supplementary Note B83 or B84, wherein the video is a video of a certain period of time before and after the point in time at which the condition is met.

### (Supplementary Note B87)

The image processing apparatus according to Supplementary Note B83 or B84, wherein the recorded information is modifiable by a user.

### (Supplementary Note B88)

The image processing apparatus according to Supplementary Note B83 or B84, further comprising a report creation unit for creating reports based on the recorded information.

### (Supplementary Note B89)

The image processing apparatus according to Supplementary Note B83 or B84, wherein the report creation unit creates a summary of a surgery based on recorded still images, video, and/or text information explaining the surgery situation.

### (Supplementary Note B90)

The image processing apparatus according to Supplementary Note B83 or B84, further comprising a notification unit for notifying the user or an output unit that performs an output when a specific surgery situation has been recorded.

### (Supplementary Note B91)

The image processing apparatus according to Supplementary Note B83 or B84, further comprising a notification unit for notifying the user or an output unit that performs an output in accordance with the time that has elapsed after a specific surgery situation was recorded.

### (Supplementary Note B92)

The image processing apparatus according to Supplementary Note B91, wherein the time that has elapsed after a specific surgery situation was recorded is determined based on balloon inflation time, guide wire travel time, speed, and acceleration, or coil travel time.

### (Supplementary Note B93)

The image processing apparatus according to Supplementary Note B83 or B84, further comprising a notification unit for notifying the user or an output unit that performs an output in accordance with the number of times that a specific surgery situation has been recorded.

### (Supplementary Note B94)

The image processing apparatus according to Supplementary Note B83 or B84, wherein the specific surgery situation is coil insertion.

### (Supplementary Note B95)

The image processing apparatus according to Supplementary Note B83 or B84, wherein the images are acquired from video recorded in real time or from previously recorded video.

### (Supplementary Note B96)

The image processing apparatus according to Supplementary Note B83 or B84, wherein, if a display device that displays the images has multiple screens, processing is performed for each screen.

### (Supplementary Note B97)

The image processing apparatus according to Supplementary Note B96, wherein the multiple screens display two or more images selected from a frontal live image, a lateral live image, a frontal mask image, and a lateral mask image.

### (Supplementary Note B98)

The image processing apparatus according to Supplementary Note B96, wherein information of multiple screens is used to improve processing accuracy.

### (Supplementary Note B99)

The image processing apparatus according to Supplementary Note B1, further comprising:
an image acquisition unit for acquiring an image including at least a device for examination or treatment in a blood vessel as a subject,
a region of interest acquisition unit for acquiring one or more regions in the image that include at least a portion of the device as a region of interest, and
a notification unit that notifies the user of the image processing apparatus when at least one of the regions of interest satisfies a condition determined for each of the regions of interest,
   wherein,
the image processing apparatus further comprises a display unit that causes a display device connected to the image processing apparatus to display the image including the region of interest, and a mark corresponding only to the vertical coordinate of the region of interest next to the image.

### (Supplementary Note B100)

The image processing apparatus according to Supplementary Note B1, further comprising:
an image acquisition unit for acquiring an image including at least a device for examination or treatment in a blood vessel as a subject,
a region of interest acquisition unit for acquiring one or more regions in the image that include at least a portion of the device as a region of interest, and
an output unit that, when at least one of the regions of interest satisfies a condition determined for each of the regions of interest, outputs the information to a device connected to the image processing apparatus,
   wherein,
the image processing apparatus further comprises a display unit that causes a display device connected to the image processing apparatus to display the image including the region of interest, and a mark corresponding only to the vertical coordinate of the region of interest next to the image.

### (Supplementary Note B101)

The image processing apparatus according to Supplementary Note B99 or B100, further comprising a tracking unit for tracking the region of interest in the image.

### (Supplementary Note B102)

The image processing apparatus according to Supplementary Note B99 or B100, further comprising a blood vessel recognition unit for recognizing at least some of the blood vessels in the image.

### (Supplementary Note B103)

The image processing apparatus according to Supplementary Note B99 or B100, wherein a notification is issued or an output is performed if the region of interest enters a blood vessel having a diameter less than or equal to a threshold value, or a blood vessel that has been specified automatically or manually.

### (Supplementary Note B104)

The image processing apparatus according to Supplementary Note B99 or B100, wherein a trajectory of a mark is displayed for a certain period of time.

### (Supplementary Note B105)

The image processing apparatus according to Supplementary Note B99 or B100, wherein the display mode of the mark is different for each region of interest.

### (Supplementary Note B106)

The image processing apparatus according to Supplementary Note B99 or B100, wherein the display mode is color, shape, pattern, symbol, text, caption, or animation.

### (Supplementary Note B107)

The image processing apparatus according to Supplementary Note B99 or B100, wherein a region of interest that should be checked is automatically recognized and highlighted.

### (Supplementary Note B108)

The image processing apparatus according to Supplementary Note B92, wherein a region of interest that should be checked is determined in accordance with the situation of a surgery.

### (Supplementary Note B109)

The image processing apparatus according to Supplementary Note B99 or B100, wherein only regions of interest specified by the user are displayed.

### (Supplementary Note B110)

The image processing apparatus according to Supplementary Note B99 or B100, wherein only automatically selected regions of interest are displayed.

### (Supplementary Note B111)

The image processing apparatus according to Supplementary Note B99 or B100, wherein the user is notified if a mark exceeds a user-specified threshold value.

### (Supplementary Note B112)

The image processing apparatus according to Supplementary Note B99 or B100, wherein, if a display device that displays the images has multiple screens, processing is performed for each screen.

### (Supplementary Note B113)

The image processing apparatus according to Supplementary Note B112, wherein the multiple screens display two or more images selected from a frontal live image, a lateral live image, a frontal mask image, and a lateral mask image.

### (Supplementary Note B114)

The image processing apparatus according to Supplementary Note B112, wherein information of multiple screens is used to improve processing accuracy.

### (Supplementary Note B115)

The image processing apparatus according to Supplementary Note B1, further comprising:
an image acquisition unit for acquiring an image including at least a device for examination or treatment in a blood vessel as a subject,
a region of interest acquisition unit for acquiring one or more regions in the image that include at least a portion of the device as a region of interest,
a tracking unit that tracks each of the regions of interest in the image, and
a notification unit that notifies the user of the image processing apparatus when at least one of the regions of interest satisfies a condition determined for each of the regions of interest,
   wherein,
the image processing apparatus further comprises a display unit for displaying, if a plurality of the regions of interest are tracked, each of the multiple regions of interest using a different display mode.

### (Supplementary Note B116)

The image processing apparatus according to Supplementary Note B1, further comprising:
an image acquisition unit for acquiring an image including at least a device for examination or treatment in a blood vessel as a subject,
a region of interest acquisition unit for acquiring one or more regions in the image that include at least a portion of the device as a region of interest,
a tracking unit that tracks each of the regions of interest in the image, and
an output unit that, when at least one of the regions of interest satisfies a condition determined for each of the regions of interest, outputs the information to a device connected to the image processing apparatus,
   wherein,
the image processing apparatus further comprises a display unit for displaying, if a plurality of the regions of interest are tracked, each of the multiple regions of interest using a different display mode.

### (Supplementary Note B 117)

The image processing apparatus according to Supplementary Note B 1 15 or B 116, further comprising a blood vessel recognition unit for recognizing at least some of the blood vessels in the image.

### (Supplementary Note B118)

The image processing apparatus according to Supplementary Note B 1 17, wherein a notification is issued or an output is performed if the region of interest enters a blood vessel having a diameter less than or equal to a threshold value, or a blood vessel that has been specified automatically or manually.

### (Supplementary Note B119)

The image processing apparatus according to Supplementary Note B 1 15 or B116, wherein the display mode is color, shape, pattern, symbol, text, caption, or animation.

### (Supplementary Note B120)

The image processing apparatus according to Supplementary Note B 1 15 or B116, wherein a region of interest that should be checked is automatically recognized and highlighted.

### (Supplementary Note B121)

The image processing apparatus according to Supplementary Note B 120, wherein a region of interest that should be checked is determined in accordance with the situation of a surgery.

### (Supplementary Note B122)

The image processing apparatus according to Supplementary Note B 1 15 or B116, wherein only regions of interest specified by the user are displayed.

### (Supplementary Note B123)

The image processing apparatus according to Supplementary Note B 1 15 or B 1 16, wherein only automatically selected regions of interest are displayed.

### (Supplementary Note B124)

The image processing apparatus according to Supplementary Note B 1 15 or B 116, wherein, if a display device that displays the images has multiple screens, processing is performed for each screen.

### (Supplementary Note B125)

The image processing apparatus according to Supplementary Note B 124, wherein the multiple screens display two or more images selected from a frontal live image, a lateral live image, a frontal mask image, and a lateral mask image.

### (Supplementary Note B126)

The image processing apparatus according to Supplementary Note B 124, wherein information of multiple screens is used to improve processing accuracy.

### (Supplementary Note B127)

A method of creating a machine learning model, wherein cerebral blood vessel images acquired from multiple directions are used as input to perform training.

### (Supplementary Note B128)

The image processing apparatus according to Supplementary Note B 1, further comprising:
an image acquisition unit for acquiring an image including at least a device for examination or treatment in a blood vessel as a subject,
a region of interest acquisition unit for acquiring one or more regions in the image that include at least a portion of the device as a region of interest, and
a notification unit that notifies the user of the image processing apparatus when at least one of the regions of interest satisfies a condition determined for each of the regions of interest,
   wherein,
the notification unit performs notification if the region of interest includes a guide wire and the length of the guide wire or the area of segmentation in a specific region including the tip of the guide wire exceeds a certain threshold value.

### (Supplementary Note B129)

The image processing apparatus according to Supplementary Note B1, further comprising:
an image acquisition unit for acquiring an image including at least a device for examination or treatment in a blood vessel as a subject,
a region of interest acquisition unit for acquiring one or more regions in the image that include at least a portion of the device as a region of interest, and
an output unit that, when at least one of the regions of interest satisfies a condition determined for each of the regions of interest, outputs the information to a device connected to the image processing apparatus,
   wherein,
the output unit performs output if the region of interest includes a guide wire and the length of the guide wire or the area of segmentation in a specific region including the tip of the guide wire exceeds a certain threshold value.

### DESCRIPTIONS OF THE REFERENCE SYMBOLS

- 1: Image processing apparatus
- 10: Storage unit
- 11: Control unit
- 110: Image acquisition unit
- 111: Region-of-interest acquisition unit
- 112: Tracking unit
- 113: Notification unit
- 114: Marker detection unit
- 115: Distance measuring unit
- 116: Output unit
- 117: Image recording unit
- 118: Image extraction unit
- 119: Status estimation unit
- 2: Display device
- 3: X-ray image capture device
- 4: External device
- 20: CPU
- 21: ROM
- 22: RAM
- 23: Storage
- 24: Input/output interface
- 25: Input unit
- 26: Output unit
- 27: Storage medium
- 30: X-ray irradiator
- 31: X-ray detector
- 32: Bed
- D: Device
- E: Coil for embolization
- P: Test subject
- S: Image processing system

## Claims

1. An image processing apparatus, comprising:
an image acquisition unit that can acquire an image including at least a device for examination or treatment in a blood vessel as a subject.

2. The image processing apparatus according to claim 1, further comprising:
an image acquisition unit for acquiring an image for examination or treatment in a blood vessel,
a region of interest acquisition unit for acquiring, when a device for examination or treatment in a blood vessel is included in the image, one or more regions that include at least a portion of the device as a region of interest, and
a notification unit that notifies the user of the image processing apparatus when at least one of the regions of interest satisfies a condition determined for each of the regions of interest,
wherein,
the notification unit notifies the user on the condition that a device or a part thereof that newly appears in an image or a specific region is newly detected as a region of interest.

3. The image processing apparatus according to claim 1, further comprising:
an image acquisition unit for acquiring an image for examination or treatment in a blood vessel,
a region of interest acquisition unit for acquiring, when a device for examination or treatment in a blood vessel is included in the image, one or more regions that include at least a portion of the device as a region of interest, and
an output unit that, when at least one of the regions of interest satisfies a condition determined for each of the regions of interest, outputs the information to a device connected to the image processing apparatus,
wherein,
the output unit outputs to the device on the condition that a device or a part thereof that newly appears in an image or a specific region is newly detected as a region of interest.

4. The image processing apparatus according to claim 2 or 3, wherein the notification unit notifies the user on the condition that a device or a part thereof that newly appears at the edge of, or within a set distance from the edge of the image is newly detected as a region of interest.

5. The image processing apparatus according to claim 2 or 3, wherein the output unit outputs to the device on the condition that a device or a part thereof that newly appears at the edge or within a set distance from the edge of the image, or within a specific region, or a portion thereof, is newly detected as a region of interest.

6. The image processing apparatus according to claim 2 or 3, wherein, if a display device that displays the images has multiple screens, processing is performed for each screen.

7. The image processing apparatus according to claim 6, wherein the multiple screens display two or more images selected from a frontal live image, a lateral live image, a frontal mask image, and a lateral mask image.

8. The image processing apparatus according to claim 6, wherein information of multiple screens is used to improve processing accuracy.

9. The image processing apparatus according to claim 2 or 3, wherein the specific region is specified by a user.

10. The image processing apparatus according to claim 2 or 3, wherein the specific region is specified automatically.

11. The image processing apparatus according to claim 2 or 3, wherein the specific region is a region that the image processing apparatus determines automatically as a region that should be checked.

12. The image processing apparatus according to claim 2 or 3, further comprising a tracking unit for tracking the region of interest in the image.

13. The image processing apparatus according to claim 2 or 3, further comprising a blood vessel recognition unit for recognizing at least some of the blood vessels in the image.

14. The image processing apparatus according to claim 2 or 3, wherein a notification is issued or an output is performed if the region of interest enters a blood vessel having a diameter less than or equal to a threshold value, or a blood vessel that has been specified automatically or manually.

15. The image processing apparatus according to claim 2 or 3, wherein a region including at least a portion of a device for examination or treatment in a blood vessel, selected from a group consisting of a guiding catheter, a guide wire, an intermediate catheter, a microcatheter, a suction catheter for thrombectomy, a marker, a coil, a stent, a filter, an embolic material, an aneurysm embolization device, and a balloon, is set as the region of interest.

16. The image processing apparatus according to claim 2 or 3, wherein the region including at least a portion of the device includes the tip of a device for examination or treatment in a blood vessel.

17. The image processing apparatus according to claim 1, further comprising:
an image acquisition unit for acquiring an image including at least a device for examination or treatment in a blood vessel as a subject,
a region of interest acquisition unit for acquiring one or more regions in the image that include at least a portion of the device as a region of interest, and
a notification unit that notifies the user of the image processing apparatus when at least one of the regions of interest satisfies a condition determined for each of the regions of interest,
wherein,
the notification unit notifies the user on the condition that the region of interest passes, or is predicted to pass, a specific boundary line specified on an image.

18. The image processing apparatus according to claim 1, further comprising:
an image acquisition unit for acquiring an image including at least a device for examination or treatment in a blood vessel as a subject,
a region of interest acquisition unit for acquiring one or more regions in the image that include at least a portion of the device as a region of interest, and
an output unit that, when at least one of the regions of interest satisfies a condition determined for each of the regions of interest, outputs the information to a device connected to the image processing apparatus,
wherein,
the notification unit notifies the user on the condition that the region of interest passes, or is predicted to pass, a specific boundary line specified on an image.

19. The image processing apparatus according to claim 17 or 18, further comprising a tracking unit for tracking the region of interest in the image.

20. The image processing apparatus according to claim 17 or 18, further comprising a blood vessel recognition unit for recognizing at least some of the blood vessels in the image.

21. The image processing apparatus according to claim 17 or 18, wherein a notification is issued or an output is performed if the region of interest enters a blood vessel having a diameter less than or equal to a threshold value, or a blood vessel that has been specified automatically or manually.

22. The image processing apparatus according to claim 17 or 18, wherein prediction of passage is performed based on distance between the region of interest and the boundary line, or on the distance/velocity of the region of interest.

23. The image processing apparatus according to claim 17 or 18, wherein the specific boundary line is specified by a user.

24. The image processing apparatus according to claim 17 or 18, wherein the specific boundary line is specified automatically.

25. The image processing apparatus according to claim 17 or 18, wherein the specific boundary line is automatically specified when the region of interest has not moved for a certain period of time.

26. The image processing apparatus according to claim 17 or 18, wherein the specific boundary line is automatically specified in accordance with the situation of the surgery, or, is automatically proposed, which can then be accepted or rejected by the user.

27. The image processing apparatus according to claim 17 or 18, wherein the specific boundary line is automatically removed in accordance with the situation of the surgery, or, is automatically proposed, which can then be accepted or rejected by the user.

28. The image processing apparatus according to claim 17 or 18, wherein, if a display device that displays the images has multiple screens, the specific boundary line specified in one screen is automatically specified in a corresponding location on the other screens.

29. The image processing apparatus according to claim 17 or 18, wherein, if a display device that displays the images has multiple screens, processing is performed for each screen.

30. The image processing apparatus according to claim 29, wherein the multiple screens display two or more images selected from a frontal live image, a lateral live image, a frontal mask image, and a lateral mask image.

31. The image processing apparatus according to claim 29, wherein information of multiple screens is used to improve processing accuracy.

32. The image processing apparatus according to claim 17 or 18, wherein the specific boundary line is represented by a straight line, a curve, a circle, a rectangle, or any other polygon.

33. The image processing apparatus according to claim 17 or 18, wherein the boundary line can be moved, deformed, enlarged, or shrunk by means of user operation.

34. The image processing apparatus according to claim 17 or 18, wherein the specific boundary line is used to detect entry of the device into a blood vessel where entry thereto is not desirable, a lesion site such as an aneurysm or stenosis, or to outside of the blood vessel.

35. The image processing apparatus according to claim 17 or 18, wherein the notification unit or output unit notifies or outputs only when the condition is met for the first time, for each of one or more regions of interest.

36. The image processing apparatus according to claim 17 or 18, wherein the notification unit or output unit notifies or outputs only when the condition is met for the first time after a particular point in time, for each of one or more regions of interest.

37. The image processing apparatus according to claim 17 or 18, wherein the notification unit or output unit notifies or outputs only when the condition is met for the first time, for all of one or more regions of interest.

38. The image processing apparatus according to claim 17 or 18, wherein the notification unit or output unit notifies or outputs only when the condition is met for the first time after a particular point in time, for all of one or more regions of interest.

39. The image processing apparatus according to claim 17 or 18, wherein the notification unit or output unit notifies or outputs only when the condition is met for one or more specific regions of interest specified by the user.

40. The image processing apparatus according to claim 17 or 18, wherein the notification unit or output unit notifies or outputs only when the condition is met for one or more regions of interest specified automatically.

41. The image processing apparatus according to claim 17 or 18, wherein the specific boundary line is superimposed and displayed on an X-ray image.

42. The image processing apparatus according to claim 17 or 18, wherein the specific boundary line is redrawn in accordance with movements or changes in the extent or magnification of the X-ray image.

43. The image processing apparatus according to claim 17 or 18, wherein the specific boundary line is redrawn after interruption and reacquisition of the X-ray image.

44. The image processing apparatus according to claim 17, wherein the notification is issued by means of generation of a warning sound or by changing a display mode of a boundary line.

45. The image processing apparatus according to claim 17, wherein the notification conveys, by means of voice, some or all of: which screen, which device, and what type of event.

46. The image processing apparatus according to claim 17, wherein different methods of notification are used in accordance with the direction in which the region of interest passes a specific boundary line specified on an image.

47. The image processing apparatus according to claim 17 or 18, wherein the direction in which the region of interest passes a specific boundary line specified on an image is automatically recognized in accordance with the situation of the surgery, or, is automatically proposed, which can then be accepted or rejected by the user.

48. The image processing apparatus according to claim 17, wherein different methods of notification are used in accordance with the speed at which the region of interest crosses the specific boundary line.

49. The image processing apparatus according to claim 17, wherein the notification unit displays the distance between the region of interest and the specific boundary line on a display device for displaying the image.

50. The image processing apparatus according to claim 17, wherein the notification unit changes the display mode of the distance in the display device according to the length of the distance between the region of interest and the specific boundary line.

51. The image processing apparatus according to claim 17 or 18, wherein the notification unit or output unit notifies or outputs on the condition that the value obtained by dividing the distance between the region of interest and the specific boundary line by the movement speed of the region of interest in the image is less than a predetermined threshold value.

52. The image processing apparatus according to claim 1, further comprising:
an image acquisition unit for acquiring an image including at least a device for examination or treatment in a blood vessel as a subject,
a region of interest acquisition unit for acquiring one or more regions in the image that include at least a portion of the device as a region of interest, and
a notification unit that notifies the user of the image processing apparatus when at least one of the regions of interest satisfies a condition determined for each of the regions of interest,
wherein,
a region of interest included in a region specified by a user on the image is acquired as the region of interest.

53. The image processing apparatus according to claim 1, further comprising:
an image acquisition unit for acquiring an image including at least a device for examination or treatment in a blood vessel as a subject,
a region of interest acquisition unit for acquiring one or more regions in the image that include at least a portion of the device as a region of interest, and
an output unit that, when at least one of the regions of interest satisfies a condition determined for each of the regions of interest, outputs the information to a device connected to the image processing apparatus,
wherein,
a region of interest included in a region specified by a user on the image is acquired as the region of interest.

54. The image processing apparatus according to claim 52 or 53, further comprising a tracking unit for tracking the region of interest in the image.

55. The image processing apparatus according to claim 52 or 53, further comprising a blood vessel recognition unit for recognizing at least some of the blood vessels in the image.

56. The image processing apparatus according to claim 52 or 53, wherein a notification is issued or an output is performed if the region of interest enters a blood vessel having a diameter less than or equal to a threshold value, or a blood vessel that has been specified automatically or manually.

57. The image processing apparatus according to claim 52 or 53, wherein a region specified by a user on the image is represented by a rectangle, a circle, or any closed curve.

58. The image processing apparatus according to claim 52 or 53, wherein candidates for regions that can be specified by a user are automatically displayed.

59. The image processing apparatus according to claim 52 or 53, wherein candidates for regions that can be specified by a user are automatically displayed in accordance with the situation of a surgery.

60. The image processing apparatus according to claim 52 or 53, wherein the image temporarily becomes a still image when a user specifies a region.

61. The image processing apparatus according to claim 52 or 53, wherein the image playback is temporarily slowed when a user specifies a region.

62. The image processing apparatus according to claim 52 or 53, wherein a portion of the image is displayed enlarged when a user specifies a region.

63. The image processing apparatus according to claim 52 or 53, wherein, if a display device that displays the images has multiple screens, processing is performed for each screen.

64. The image processing apparatus according to claim 63, wherein the multiple screens display two or more images selected from a frontal live image, a lateral live image, a frontal mask image, and a lateral mask image.

65. The image processing apparatus according to claim 63, wherein information of multiple screens is used to improve processing accuracy.

66. The image processing apparatus according to claim 52 or 53, wherein, if a region including multiple regions of interest is specified, only the region of interest that should be checked is selected automatically.

67. The image processing apparatus according to claim 66, wherein the region of interest that should be checked is determined based on importance of multiple devices, position of the region of interest in the screen, and the surgery scene.

68. The image processing apparatus according to claim 66, wherein a region of interest that should be checked is determined in accordance with the situation of a surgery.

69. The image processing apparatus according to claim 52 or 53, wherein the specified region is automatically adjusted when the image is changed.

70. The image processing apparatus according to claim 1, further comprising:
an image acquisition unit for acquiring an image including at least a device for examination or treatment in a blood vessel as a subject, and
a situation recognition unit for recognizing a specific surgery situation based on the image.

71. The image processing apparatus according to claim 70, wherein the specific surgery situation is at least one situation selected from a group consisting of the content of a surgery, disease name, vascular leakage, vascular perforation, occurrence of thrombus, disappearance of peripheral blood vessels, guiding a guide wire/a catheter/a balloon, aspiration by catheter, stent placement, balloon inflation, coil insertion, insertion of an aneurysm embolization device, timing when a coil may be detached, guiding/placing/retrieving a filter, injection of liquid embolic material, injection of contrast agent, continuation of a still image for a certain period of time or more, discrimination between a mask image and a live image, determination of imaging site/angle, switching of images, and occurrence of noise.

72. The image processing apparatus according to claim 70 or 71, further comprising:
a region of interest acquisition unit for acquiring one or more regions in the image that include at least a portion of the device as a region of interest, and
a notification unit that notifies the user of the image processing apparatus when at least one of the regions of interest satisfies a condition determined for each of the regions of interest.

73. The image processing apparatus according to claim 70, wherein acquisition of the region of interest is stopped if occurrence of noise is recognized.

74. The image processing apparatus according to claim 70, wherein, when injection of a contrast medium is recognized, when a 3D image is acquired, or when a cone beam CT (CBCT) is acquired, acquisition of the region of interest is stopped, or, is acquired but not notified.

75. The image processing apparatus according to claim 70, wherein a recommendation to reduce exposure is notified if continuation of a still image of a certain period of time or more is recognized.

76. The image processing apparatus according to claim 70 or 71, further comprising a recording unit for recording the specific surgery situation that is recognized.

77. The image processing apparatus according to claim 70 or 71, wherein the specific surgery situation is recognized by means of pattern matching, image recognition, time-series image recognition, time-series differencing, or object detection algorithms.

78. The image processing apparatus according to claim 70 or 71, wherein the device to be recognized is specified in accordance with the specific surgery situation that has been recognized.

79. The image processing apparatus according to claim 70 or 71, wherein a specific boundary line for notifying a user on the condition that a region of interest passes the specific boundary line specified on the image is automatically specified or proposed around the region of interest, in accordance with the specific surgery situation that has been recognized.

80. The image processing apparatus according to claim 70 or 71, wherein, if a display device that displays the images has multiple screens, processing is performed for each screen.

81. The image processing apparatus according to claim 80, wherein the multiple screens display two or more images selected from a frontal live image, a lateral live image, a frontal mask image, and a lateral mask image.

82. The image processing apparatus according to claim 80, wherein information of multiple screens is used to improve processing accuracy.

83. The image processing apparatus according to claim 1, further comprising:
an image acquisition unit for acquiring an image including at least a device for examination or treatment in a blood vessel as a subject,
a region of interest acquisition unit for acquiring one or more regions in the image that include at least a portion of the device as a region of interest, and
a recording unit for recording, when at least one of the regions of interest satisfies a condition defined for each of the regions of interest, the situation of a surgery corresponding to that condition.

84. The image processing apparatus according to claim 83, wherein the conditions include at least one procedure selected from a group consisting of puncture, arrival of a guiding catheter to the treatment site, balloon guidance, balloon inflation, stent deployment, catheter guidance, insertion of the first coil, insertion of the second and subsequent coils, location of the coil within the aneurysm, deviation of the coil to the parent vessel, catheter removal, coil removal, and end of procedure.

85. The image processing apparatus according to claim 83 or 84, wherein the surgery situation to be recorded includes still images, video, and/or text information explaining the surgery situation.

86. The image processing apparatus according to claim 83 or 84, wherein the video is a video of a certain period of time before and after the point in time at which the condition is met.

87. The image processing apparatus according to claim 83 or 84, wherein the recorded information is modifiable by a user.

88. The image processing apparatus according to claim 83 or 84, further comprising a report creation unit for creating reports based on the recorded information.

89. The image processing apparatus according to claim 83 or 84, wherein the report creation unit creates a summary of a surgery based on recorded still images, video, and/or text information explaining the surgery situation.

90. The image processing apparatus according to claim 83 or 84, further comprising a notification unit for notifying the user or an output unit that performs an output when a specific surgery situation has been recorded.

91. The image processing apparatus according to claim 83 or 84, further comprising a notification unit for notifying the user or an output unit that performs an output in accordance with the time that has elapsed after a specific surgery situation was recorded.

92. The image processing apparatus according to claim 91, wherein the time that has elapsed after a specific surgery situation was recorded is determined based on balloon inflation time, guide wire travel time, speed, and acceleration, or coil travel time.

93. The image processing apparatus according to claim 83 or 84, further comprising a notification unit for notifying the user or an output unit that performs an output in accordance with the number of times that a specific surgery situation has been recorded.

94. The image processing apparatus according to claim 83 or 84, wherein the specific surgery situation is coil insertion.

95. The image processing apparatus according to claim 83 or 84, wherein the images are acquired from video recorded in real time or from previously recorded video.

96. The image processing apparatus according to claim 83 or 84, wherein, if a display device that displays the images has multiple screens, processing is performed for each screen.

97. The image processing apparatus according to claim 96, wherein the multiple screens display two or more images selected from a frontal live image, a lateral live image, a frontal mask image, and a lateral mask image.

98. The image processing apparatus according to claim 96, wherein information of multiple screens is used to improve processing accuracy.

99. The image processing apparatus according to claim 1, further comprising:
an image acquisition unit for acquiring an image including at least a device for examination or treatment in a blood vessel as a subject,
a region of interest acquisition unit for acquiring one or more regions in the image that include at least a portion of the device as a region of interest, and
a notification unit that notifies the user of the image processing apparatus when at least one of the regions of interest satisfies a condition determined for each of the regions of interest,
wherein,
the image processing apparatus further comprises a display unit that causes a display device connected to the image processing apparatus to display the image including the region of interest, and a mark corresponding only to the vertical coordinate of the region of interest next to the image.

100. The image processing apparatus according to claim 1, further comprising:
an image acquisition unit for acquiring an image including at least a device for examination or treatment in a blood vessel as a subject,
a region of interest acquisition unit for acquiring one or more regions in the image that include at least a portion of the device as a region of interest, and
an output unit that, when at least one of the regions of interest satisfies a condition determined for each of the regions of interest, outputs the information to a device connected to the image processing apparatus,
wherein,
the image processing apparatus further comprises a display unit that causes a display device connected to the image processing apparatus to display the image including the region of interest, and a mark corresponding only to the vertical coordinate of the region of interest next to the image.

101. The image processing apparatus according to claim 99 or 100, further comprising a tracking unit for tracking the region of interest in the image.

102. The image processing apparatus according to claim 99 or 100, further comprising a blood vessel recognition unit for recognizing at least some of the blood vessels in the image.

103. The image processing apparatus according to claim 99 or 100, wherein a notification is issued or an output is performed if the region of interest enters a blood vessel having a diameter less than or equal to a threshold value, or a blood vessel that has been specified automatically or manually.

104. The image processing apparatus according to claim 99 or 100, wherein a trajectory of a mark is displayed for a certain period of time.

105. The image processing apparatus according to claim 99 or 100, wherein the display mode of the mark is different for each region of interest.

106. The image processing apparatus according to claim 99 or 100, wherein the display mode is color, shape, pattern, symbol, text, caption, or animation.

107. The image processing apparatus according to claim 99 or 100, wherein a region of interest that should be checked is automatically recognized and highlighted.

108. The image processing apparatus according to claim 92, wherein a region of interest that should be checked is determined in accordance with the situation of a surgery.

109. The image processing apparatus according to claim 99 or 100, wherein only regions of interest specified by the user are displayed.

110. The image processing apparatus according to claim 99 or 100, wherein only automatically selected regions of interest are displayed.

111. The image processing apparatus according to claim 99 or 100, wherein the user is notified if a mark exceeds a user-specified threshold value.

112. The image processing apparatus according to claim 99 or 100, wherein, if a display device that displays the images has multiple screens, processing is performed for each screen.

113. The image processing apparatus according to claim 112, wherein the multiple screens display two or more images selected from a frontal live image, a lateral live image, a frontal mask image, and a lateral mask image.

114. The image processing apparatus according to claim 112, wherein information of multiple screens is used to improve processing accuracy.

115. The image processing apparatus according to claim 1, further comprising:
an image acquisition unit for acquiring an image including at least a device for examination or treatment in a blood vessel as a subject,
a region of interest acquisition unit for acquiring one or more regions in the image that include at least a portion of the device as a region of interest,
a tracking unit that tracks each of the regions of interest in the image, and
a notification unit that notifies the user of the image processing apparatus when at least one of the regions of interest satisfies a condition determined for each of the regions of interest,
wherein,
the image processing apparatus further comprises a display unit for displaying, if a plurality of the regions of interest are tracked, each of the multiple regions of interest using a different display mode.

116. The image processing apparatus according to claim 1, further comprising:
an image acquisition unit for acquiring an image including at least a device for examination or treatment in a blood vessel as a subject,
a region of interest acquisition unit for acquiring one or more regions in the image that include at least a portion of the device as a region of interest,
a tracking unit that tracks each of the regions of interest in the image, and
an output unit that, when at least one of the regions of interest satisfies a condition determined for each of the regions of interest, outputs the information to a device connected to the image processing apparatus,
wherein,
the image processing apparatus further comprises a display unit for displaying, if a plurality of the regions of interest are tracked, each of the multiple regions of interest using a different display mode.

117. The image processing apparatus according to claim 115 or 116, further comprising a blood vessel recognition unit for recognizing at least some of the blood vessels in the image.

118. The image processing apparatus according to claim 117, wherein a notification is issued or an output is performed if the region of interest enters a blood vessel having a diameter less than or equal to a threshold value, or a blood vessel that has been specified automatically or manually.

119. The image processing apparatus according to claim 115 or 116, wherein the display mode is color, shape, pattern, symbol, text, caption, or animation.

120. The image processing apparatus according to claim 115 or 116, wherein a region of interest that should be checked is automatically recognized and highlighted.

121. The image processing apparatus according to claim 120, wherein a region of interest that should be checked is determined in accordance with the situation of a surgery.

122. The image processing apparatus according to claim 115 or 116, wherein only regions of interest specified by the user are displayed.

123. The image processing apparatus according to claim 115 or 116, wherein only automatically selected regions of interest are displayed.

124. The image processing apparatus according to claim 115 or 116, wherein, if a display device that displays the images has multiple screens, processing is performed for each screen.

125. The image processing apparatus according to claim 124, wherein the multiple screens display two or more images selected from a frontal live image, a lateral live image, a frontal mask image, and a lateral mask image.

126. The image processing apparatus according to claim 124, wherein information of multiple screens is used to improve processing accuracy.

127. A method of creating a machine learning model, wherein cerebral blood vessel images acquired from multiple directions are used as input to perform training.

128. The image processing apparatus according to claim 1, further comprising:
an image acquisition unit for acquiring an image including at least a device for examination or treatment in a blood vessel as a subject,
a region of interest acquisition unit for acquiring one or more regions in the image that include at least a portion of the device as a region of interest, and
a notification unit that notifies the user of the image processing apparatus when at least one of the regions of interest satisfies a condition determined for each of the regions of interest,
wherein,
the notification unit performs notification if the region of interest includes a guide wire and the length of the guide wire or the area of segmentation in a specific region including the tip of the guide wire exceeds a certain threshold value.

129. The image processing apparatus according to claim 1, further comprising:
an image acquisition unit for acquiring an image including at least a device for examination or treatment in a blood vessel as a subject,
a region of interest acquisition unit for acquiring one or more regions in the image that include at least a portion of the device as a region of interest, and
an output unit that, when at least one of the regions of interest satisfies a condition determined for each of the regions of interest, outputs the information to a device connected to the image processing apparatus,
wherein,
the output unit performs output if the region of interest includes a guide wire and the length of the guide wire or the area of segmentation in a specific region including the tip of the guide wire exceeds a certain threshold value.
